(19) Europäisches Patentamt
European Patent Office
Office européen des brevets

(11) **EP 2 331 258 B1**

(12) **EUROPÄISCHE PATENTSCHRIFT**

(45) Veröffentlichungstag und Bekanntmachung des Hinweises auf die Patenterteilung:
**02.07.2014 Patentblatt 2014/27**

(21) Anmeldenummer: **09782421.3**

(22) Anmeldetag: **01.09.2009**

(51) Int Cl.:
**B01J 23/888** (2006.01)     **B01J 35/02** (2006.01)
**C07C 45/35** (2006.01)     **C07C 51/25** (2006.01)

(86) Internationale Anmeldenummer:
**PCT/EP2009/061234**

(87) Internationale Veröffentlichungsnummer:
**WO 2010/028977 (18.03.2010 Gazette 2010/11)**

(54) **VERFAHREN ZUR HERSTELLUNG VON GEOMETRISCHEN KATALYSATORFORMKÖRPERN**

METHOD FOR PRODUCING GEOMETRIC CATALYST MOULDED BODIES

PROCÉDÉ DE FABRICATION DE CORPS MOULÉS DE CATALYSEUR GÉOMÉTRIQUES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **12.09.2008 DE 102008042064**
**12.09.2008 US 96537 P**

(43) Veröffentlichungstag der Anmeldung:
**15.06.2011 Patentblatt 2011/24**

(73) Patentinhaber: **BASF SE**
**67056 Ludwigshafen (DE)**

(72) Erfinder:
• **RAICHLE, Andreas**
**01109 Dresden (DE)**
• **HORSTMANN, Catharina**
**67056 Ludwigshafen (DE)**
• **ROSOWSKI, Frank**
**68239 Mannheim (DE)**
• **MÜLLER-ENGEL, Klaus ,Joachim**
**76297 Stutensee (DE)**
• **BORCHERT, Holger**
**67591 Offstein (DE)**
• **COX, Gerhard**
**67098 Bad Dürkheim (DE)**
• **CREMER, Ulrich**
**68161 Mannheim (DE)**

(56) Entgegenhaltungen:
EP-A1- 0 575 897      DE-A1-102007 005 606
DE-A1-102008 040 094      DE-A1-102008 042 060

**Beschreibung**

Vorliegende Erfindung betrifft ein Verfahren zur Herstellung von geometrischen Katalysatorformkörpern K*, die als Aktivmasse ein Multielementoxid I der allgemeinen Stöchiometrie I,

$$[Bi_1W_bO_x]_a[Mo_{12}Z^1_cZ^2_dFe_eZ^3_fZ^4_gZ^5_hO_y]_1 \qquad (I),$$

mit

Z$^1$ =    ein Element oder mehr als ein Element aus der Gruppe bestehend aus Nickel und Kobalt,

Z$^2$ =    ein Element oder mehr als ein Element aus der Gruppe bestehend aus den Alkalimetallen, den Erdalkalimetallen und Thallium,

Z$^3$ =    ein Element oder mehr als ein Element aus der Gruppe bestehend aus Zink, Phosphor, Arsen, Bor, Antimon, Zinn, Cer, Vanadium, Chrom und Wismut,

Z$^4$ =    ein Element oder mehr als ein Element aus der Gruppe bestehend aus Silizium, Aluminium, Titan, Wolfram und Zirkonium,

Z$^5$ =    ein Element oder mehr als ein Element aus der Gruppe bestehend aus Kupfer, Silber, Gold, Yttrium, Lanthan und den Lanthaniden,

a =        0,1 bis 3,
b =        0,1 bis 10,
c =        1 bis 10,
d =        0,01 bis 2,
e =        0,01 bis 5,
f =        0 bis 5,
g =        0 bis 10,
h =        0 bis 1, und
x, y =     Zahlen, die durch die Wertigkeit und Häufigkeit der von Sauerstoff verschiedenen Elemente in I bestimmt werden,

enthalten, bei dem man

- ein feinteiliges Mischoxid $Bi_1W_bO_x$ mit einem in der Längeneinheit µm angegebenen Partikeldurchmesser $d_{50}^{A1}$ als Ausgangsmasse A1 mit der Maßgabe vorbildet, dass $1\ \mu m \leq d_{50}^{A1} \leq 10\ \mu m$ erfüllt ist;

- unter Verwendung von Quellen der von Sauerstoff verschiedenen Elemente des Anteils T = $[Mo_{12}Z^1_cZ^2_dFe_eZ^3_fZ^4_gZ^5_hO_y]_1$ des Multielementoxids I in wässrigem Medium mit der Maßgabe eine innige wässrige Mischung M erzeugt, dass

  - jede der verwendeten Quellen im Verlauf der Herstellung der wässrigen Mischung M einen Zerteilungsgrad Q durchläuft, der dadurch gekennzeichnet ist, dass sein Durchmesser $d_{90}^{Q} \leq 5\ \mu m$ beträgt, und

  - die wässrige Mischung M die Elemente Mo, Z$^1$, Z$^2$, Fe, Z$^3$, Z$^4$ und Z$^5$ in der Stöchiometrie I*,

    $$Mo_{12}Z^1_cZ^2_dFe_eZ^3_fZ^4_gZ^5_h \qquad (I^*),$$

    enthält;

- aus der wässrigen Mischung M durch Trocknen und Einstellen des Zerteilungsgrades $d_{90}^{A2}$ eine feinteilige Ausgangsmasse A2 mit einem in der Längeneinheit µm angegebenen Partikeldurchmesser $d_{90}^{A2}$ unter der Maßgabe erzeugt, dass $200\ \mu m \geq d_{90}^{A2} \geq 20\ \mu m$ erfüllt ist;

- Ausgangsmasse A1 und Ausgangsmasse A2, oder Ausgangsmasse A1, Ausgangsmasse A2 und feinteilige Formgebungshilfsmittel zu einer feinteiligen Ausgangsmasse A3 mit der Maßgabe miteinander vermischt, dass die Ausgangsmasse A3 die über die Ausgangsmassen A1 und A2 in die Ausgangsmasse A3 eingebrachten, von Sauerstoff verschiedenen, Elemente des Multielementoxids I in der Stöchiometrie I**,

$$[Bi_1W_b]_a \ [Mo_{12}Z^1_c Z^2_d Fe_e Z^3_f Z^4_g Z^5_h]_1 \qquad (I^{**}),$$

enthält,

- mit feinteiliger Ausgangsmasse A3 geometrische Formkörper V formt, und

- die Formkörper V bei erhöhter Temperatur unter Erhalt der geometrischen Katalysatorformkörper K* thermisch behandelt.

Außerdem betrifft vorliegende Erfindung die Verwendung von Katalysatorformkörpern K*.

Geometrische Katalysatorformkörper K*, die als Aktivmasse ein Multielementoxid I enthalten, sowie Verfahren zur Herstellung solcher Katalysatorformkörper sind bekannt (vgl. z. B. die Deutsche Anmeldung mit dem Aktenzeichen 102007003778.5, EP-A 575 897, WO 2007/017431, WO 02/24620, WO 2005/42459, WO 2005/47224, WO 2005/49200, WO 2005/113127, die Deutsche Anmeldung mit dem Aktenzeichen 102008040093.9, die Deutsche Anmeldung mit dem Aktenzeichen 102008040094.7 und die DE-A 102007005606).

Es ist ferner bekannt, dass Katalysatoren K* (geometrische Katalysatorformkörper K*) zur Durchführung von heterogen katalysierten Partialoxidationen von 3 bis 6 C-Atome aufweisenden Alkanen, Alkanolen, Alkenen und/oder Alkenalen in der Gasphase geeignet sind.

Unter einer vollständigen Oxidation einer organischen Verbindung mit molekularem Sauerstoff wird in dieser Schrift verstanden, dass die organische Verbindung unter reaktiver Einwirkung von molekularem Sauerstoff so umgesetzt wird, dass der in der organischen Verbindung insgesamt enthaltene Kohlenstoff in Oxide des Kohlenstoffs und der in der organischen Verbindung insgesamt enthaltene Wasserstoff in Oxide des Wasserstoffs umgewandelt wird. Alle davon verschiedenen Umsetzungen einer organischen Verbindung unter reaktiver Einwirkung von molekularem Sauerstoff werden in dieser Schrift als Partialoxidationen einer organischen Verbindung zusammengefasst.

Im Besonderen sollen in dieser Schrift unter Partialoxidationen solche Umsetzungen organischer Verbindungen unter reaktiver Einwirkung von molekularem Sauerstoff verstanden werden, bei denen die partiell zu oxidierende organische Verbindung nach beendeter Umsetzung wenigstens ein Sauerstoffatom mehr chemisch gebunden enthält als vor Durchführung der Partialoxidation.

Der Begriff der partiellen Oxidation soll in dieser Schrift aber auch die oxidative Dehydrierung und die partielle Ammoxidation, d. h., eine partielle Oxidation im Beisein von Ammoniak, umfassen.

Besonders geeignet sind Katalysatoren K* (geometrische Katalysatorformkörper K*) zur Durchführung der heterogen katalysierten partiellen Gasphasenoxidation von Propen zu Acrolein und von iso-Buten zu Methacrolein, sowie zur Durchführung der heterogen katalysierten partiellen Gasphasenammoxidation von Propen zu Acrylnitril und von iso-Buten zu Methacrylnitril.

In der Regel bildet die heterogen katalysierte partielle Gasphasenoxidation von Propen (iso-Buten) zu Acrolein (Methacrolein) die erste Stufe einer zweistufigen heterogen katalysierten partiellen Gasphasenoxidation von Propen (iso-Buten) zu Acrylsäure (Methacrylsäure), wie sie beispielhaft in der WO 2005/42459 beschrieben ist.

Eine mit einer heterogen katalysierten partiellen Gasphasenoxidation von Propen (iso-Buten) zu Acrolein (Methacrolein) einhergehende Nebenproduktbildung an Acrylsäure (Methacrylsäure) ist daher in der Regel nicht unerwünscht und subsumiert normalerweise unter der erwünschten Wertproduktbildung.

Es ist weiterhin bekannt, dass die Performance von geometrischen Katalysatorformkörpern K* im Verlauf des kontinuierlichen Betriebs einer heterogen katalysierten partiellen Gasphasenoxidation von 3 bis 6 C-Atome aufweisenden Alkanen, Alkanolen, Alkenen und/oder Alkenalen (z. B. zu den entsprechenden olefinisch ungesättigten Aldehyden und/oder Carbonsäuren) mit zunehmender Betriebsdauer abnimmt (dies trifft vor allem auf den Fall einer durch geometrische Katalysatorformkörper K* heterogen katalysierten partiellen Gasphasenoxidation von Propen zu Acrolein und/oder Acrylsäure sowie von iso-Buten zu Methacrolein und/oder Methacrylsäure zu; es gilt aber auch für den Fall einer heterogen katalysierten partiellen Gasphasenammoxidation von Propen zu Acrylnitril sowie von iso-Buten zu Methacrylnitril). Diese Feststellung ist auch dann zutreffend, wenn die geometrischen Katalysatorformkörper K* im Verlauf des kontinuierlichen Betriebs der heterogen katalysierten partiellen Gasphasenoxidation einem periodisch wiederholten Regenierverfahren unterworfen werden, wie es z. B. die Schriften WO 2005/42459 sowie WO 2005/49200 empfehlen.

In erster Linie ist es die Aktivität der geometrischen Katalysatorformkörper K*, die im Verlauf des vielfach mehrjährigen Betriebs einer heterogen katalysierten partiellen Gasphasenoxidation einer organischen Verbindung gemindert wird.

Ein Maß für die Aktivität der geometrischen Katalysatorformkörper K* beziehungsweise eines diese enthaltenden Katalysatorbetts ist diejenige Temperatur, die erforderlich ist, um beim Durchgang des die partiell zu oxidierende organische Verbindung enthaltenden Reaktionsgasgemischs durch das Katalysatorbett einen bestimmten Umsatz der organischen Verbindung (z. B. des Propens oder des iso-Butens) zu erzielen.

Nimmt die Aktivität der geometrischen Katalysatorformkörper K* eines diese enthaltenden Katalysatorbetts mit zunehmender Betriebsdauer der Partialoxidation zunehmend ab, bedarf es, unter ansonsten unveränderten Reaktionsbedingungen, einer zunehmend erhöhten Temperatur, um denselben Umsatz der organischen Verbindung beim Einmaldurchgang des Reaktionsgasgemischs durch das Katalysatorbett zu erzielen (befindet sich das Katalysatorbett z. B. in den von einem Salzbad umströmten Rohren eines Rohrbündelreaktors, wird man mit zunehmender Desaktivierung des Katalysatorbetts normalerweise, unter ansonsten unveränderten Betriebsbedingungen, die Eintrittstemperatur des Salzbades in den Rohrbündelreaktor sukzessive erhöhen, um den auf einen Einmaldurchgang des Reaktionsgasgemischs durch das Katalysatorbett bezogenen Partialoxidationsumsatz beizubehalten (vgl. z. B. EP-A 1 734 030, WO 2007/82827, WO 2005/47224 und WO 2005/42459)).

Nachteilig an der vorstehend beschriebenen Verfahrensweise ist jedoch, dass die Katalysatordesaktivierung mit zunehmender Reaktionstemperatur zunehmend rascher voranschreitet, bis das verbrauchte Katalysatorbett wenigstens teilweise oder vollständig durch ein Katalysatorbett mit frischen geometrischen Katalysatorformkörpern K* ersetzt werden muss (vgl. z. B. WO 2004/9525, DE-A 10 2006 00 0996 und WO 2007/77145).

Ein wenigstens teilweiser oder ein vollständiger Katalysatorbettwechsel ist aber insofern von Nachteil, als mit ihm in notwendiger Weise eine Unterbrechung der Zielproduktherstellung einhergeht.

Darüber hinaus ist mit der Herstellung von frischem Katalysator für eine großtechnische Zielproduktherstellung ein erhebliches Investment verbunden, da sowohl die Aufwendungen für die diesbezüglich erforderlichen Rohstoffe als auch der Fertigungsaufwand nicht unerheblich sind.

Insgesamt besteht somit ein generelles Interesse an geometrischen Katalysatorformkörpern K*, die im kontinuierlichen Betrieb der heterogen katalysierten partiellen Gasphasenoxidation eine möglichst geringe Desaktivierungsrate aufweisen.

Die Kausalzusammenhänge, deren Einhaltung bei einer Herstellung von geometrischen Katalysatorformkörpern K* diese möglichst ausgedehnte Langzeitstabilität derselben bedingen, sind im Wesentlichen unbekannt.

Forschungsvorhaben, die sich dem Problem widmen, sind enorm zeitaufwendig, da sie sich mit einem Sachverhalt auseinandersetzen, der sich auf den ersten Blick nur über sehr lange Beobachtungszeiträume abbildet. Darüber hinaus können auch Fehler beim Betrieb der heterogen katalysierten partiellen Gasphasenoxidation bei Verwendung von ein und demselben Katalysatorbett eine erhöhte Desaktivierungsrate bedingen.

Wesentliche Grundlage der vorliegenden Erfindung bildet die Beobachtung, dass geometrische Katalysatorformkörper K* selbst bei identischer chemischer Zusammensetzung ihrer Aktivmasse ein unterschiedliches Desaktivierungsverhalten aufweisen können.

Eine weitere Grundlage der vorliegenden Erfindung bildet die Beobachtung, dass die Desaktivierung eines Katalysatorfestbetts unter ansonsten vorgegebenen Bedingungen rascher voranschreitet, wenn man die Belastung des Katalysatorfestbetts mit Reaktionsgasgemisch erhöht.

Unter der Belastung eines einen Reaktionsschritt katalysierenden Katalysatorfestbetts mit Reaktionsgasgemisch wird in dieser Schrift die Menge an Reaktionsgasgemisch in Normlitern (= Nl; das Volumen in Litern, das die entsprechende Reaktionsgasgemischmenge bei Normalbedingungen, d.h., bei 0 °C und 1 atm, einnehmen würde) verstanden, die dem Katalysatorfestbett, bezogen auf das Volumen seiner Schüttung, d. h., auf sein Schüttvolumen (reine Inertmaterialabschnitte werden dabei nicht miteinbezogen), pro Stunde zugeführt wird ($\rightarrow$ Einheit = Nl/l•h).

Die Belastung kann auch nur auf einen Bestandteil des Reaktionsgasgemischs bezogen sein (z. B. nur auf die partiell zu oxidierende organische Ausgangsverbindung). Dann ist es die Volumenmenge dieses Bestandteils (z. B. der organischen Ausgangsverbindung der Partialoxidation), die dem Katalysatorfestbett, bezogen auf das Volumen seiner Schüttung, pro Stunde zugeführt wird.

Eine zusätzliche wesentliche Grundlage der vorliegenden Erfindung bildet der an entsprechenden Rückstellproben von für die großtechnische Produktion eingesetzten geometrischen Katalysatorformkörpern K* gemachte experimentelle Befund, dass sich die im großtechnischen Langzeitbetrieb innerhalb der verschiedenen Katalysatorbeschickungen der einzelnen Reaktoren unter vergleichbaren Betriebsbedingungen bezüglich ihrer Desaktivierungsrate beobachtete Ordnungsrelation in eine entsprechende Rangordnung abgebildet werden konnte, wenn die Rückstellproben einem einen vergleichsweise wesentlich geringeren Zeitaufwand erfordernden Stresstest unterzogen wurden, der in erster Linie dadurch gekennzeichnet ist, dass die selbe heterogen katalysierte Gasphasenpartialoxidation sowohl bei einer höheren Temperatur als auch bei einer höheren Belastung des Katalysatorfestbetts mit demselben Reaktionsgasgemisch durchgeführt wird. Der Unterschied, zwischen der vorab der Durchführung des Stresstestes unter den eigentlich ins Auge gefassten Betriebsbedingungen zur Einstellung des gewünschten Partialoxidationsumsatzes erforderlichen Temperatur und der nach der Durchführung des Stresstestes unter den, abgesehen von der Temperatur, selben Betriebsbedingungen zur Einstellung des selben Partialoxidationsumsatzes erforderlichen Temperatur, hat sich als treffsicherer Rangordnungsparameter hinsichtlich der Langzeitstabilität des Katalysatorbetts erwiesen.

Aus der EP-A 575897 ist bekannt, dass im Rahmen einer Herstellung von geometrischen Katalysatorformkörpern K* die Partikelgröße des vorgebildeten feinteiligen Mischoxide $Bi_1W_bO_x$ die Anfangsaktivität solcher Katalysatorformkörper bei deren Verwendung für die heterogen katalysierte partielle Gasphasenoxidation von Propylen zu Acrolein beeinflusst.

Angesichts dieses Standes der Technik bestand die Aufgabe der vorliegenden Erfindung darin, geometrische Formkörper K* und ein Verfahren zu deren Herstellung zur Verfügung zu stellen, die, insbesondere im Rahmen einer Verwendung für eine heterogen katalysierte partielle Gasphasenoxidation von Propen zu Acrolein, bei im wesentlichen gleicher Anfangsaktivität (nach beendeter Formierung) im kontinuierlichen Partialoxidationsbetrieb eine verringerte Desaktivierungsrate aufweisen.

Als Lösung der Aufgabe wird ein Verfahren zur Herstellung von geometrischen Katalysatorformkörpern K, die als Aktivmasse ein Multielementoxid I der allgemeinen Stöchiometrie I,

$$[Bi_1W_bO_x]_a \ [Mo_{12}Z^1_cZ^2_dFe_eZ^3_fZ^4_gZ^5_hO_y]_1 \qquad (I),$$

mit

$Z^1$ = ein Element oder mehr als ein Element aus der Gruppe bestehend aus Nickel und Kobalt,

$Z^2$ = ein Element oder mehr als ein Element aus der Gruppe bestehend aus den Alkalimetallen, den Erdalkalimetallen und Thallium,

$Z^3$ = ein Element oder mehr als ein Element aus der Gruppe bestehend aus Zink, Phosphor, Arsen, Bor, Antimon, Zinn, Cer, Vanadium, Chrom und Wismut,

$Z^4$ = ein Element oder mehr als ein Element aus der Gruppe bestehend aus Silizium, Aluminium, Titan, Wolfram und Zirkonium,

$Z^5$ = eine Element oder mehr als ein Element aus der Gruppe bestehend aus Kupfer, Silber, Gold, Yttrium, Lanthan und den Lanthaniden (den seltenen Erdmetallen),

a = 0,1 bis 3,
b = 0,1 bis 10,
c = 1 bis 10,
d = 0,01 bis 2,
e = 0,01 bis 5,
f = 0 bis 5,
g = 0 bis 10,
h = 0 bis 1, und
x, y = Zahlen, die durch die Wertigkeit und Häufigkeit der von Sauerstoff verschiedenen Elemente in I bestimmt werden,

enthalten, und bei dem man

- ein feinteiliges Mischoxid $Bi_1W_bO_x$ mit einem in der Längeneinheit μm angegebenen Partikeldurchmesser $d_{50}^{A1}$ als Ausgangsmasse A1 mit der Maßgabe vorbildet, dass $1 \ \mu m \leq d_{50}^{A1} \leq 10 \ \mu m$ erfüllt ist;

- unter Verwendung von Quellen der von Sauerstoff verschiedenen Elemente des Anteils T = $[Mo_{12}Z^1_cZ^2_dFe_eZ^3_fZ^4_gZ^5_hO_y]_1$ des Multielementoxids I in wässrigem Medium mit der Maßgabe eine innige wässrige Mischung M erzeugt, dass

 - jede der verwendeten Quellen im Verlauf der Herstellung der wässrige Mischung M einen Zerteilungsgrad Q durchläuft, der dadurch gekennzeichnet ist, dass sein Durchmesser $d_{90}^Q \leq 5 \ \mu m$ beträgt, und

 - die wässrige Mischung M die Elemente Mo, $Z^1$, $Z^2$, Fe, $Z^3$, $Z^4$ und $Z^5$ in der Stöchiometrie I*,

$$Mo_{12}Z^1{}_cZ^2{}_dFe_eZ^3{}_fZ^4{}_gZ^5{}_h \qquad (I^*),$$

enthält;

- aus der wässrigen Mischung M durch Trocknen und Einstellen des Zerteilungsgrades $d_{90}^{A2}$ eine feinteilige Ausgangsmasse A2 mit einem in der Längeneinheit μm angegebenen Partikeldurchmesser $d_{90}^{A2}$ unter der Maßgabe erzeugt, dass $200\ \mu m \geq d_{90}^{A2} \geq 20\ \mu m$ erfüllt ist;

- Ausgangsmasse A1 und Ausgangsmasse A2, oder Ausgangsmasse1, Ausgangsmasse A2 und feinteilige Formgebungshilfsmittel zu einer feinteiligen Ausgangsmasse A3 mit der Maßgabe miteinander vermischt, dass die Ausgangsmasse A3 die über die Ausgangsmassen A1 und A2 in die Ausgangsmasse A3 eingebrachten, von Sauerstoff verschiedenen, Elemente des Multielementoxids I in der Stöchiometrie I**,

$$[Bi_1W_b]_a\ [Mo_{12}Z^1{}_cZ^2{}_dFe_eZ^3{}_fZ^4{}_gZ^5{}_h] \qquad (I^{**})$$

enthält,

- mit feinteiliger Ausgangsmasse A3 geometrische Formkörper V formt, und

- die Formkörper V bei erhöhter Temperatur unter Erhalt der geometrischen Katalysatorformkörper K thermisch behandelt,

zur Verfügung gestellt, das dadurch gekennzeichnet ist, dass der Betrag F (der Stabilitätswert F des geometrischen Katalysatorformkörpers K) des Produkts

$$(\,d_{50}^{A1}\,)^{0,7} \qquad \bullet \qquad (\,d_{90}^{A2}\,)^{1,5} \qquad \bullet \qquad (a^{-1})$$

≥ 820 beträgt.

Erfindungsgemäß bevorzugt beträgt F ≥ 830, vorteilhaft ≥ 840 und noch besser ≥ 850.

Besonders vorteilhaft beträgt F ≥ 870 oder ≥ 900, und besonders vorteilhaft beträgt F ≥ 950 oder ≥ 1000.

Ganz besonders vorteilhaft beträgt F ≥ 1050, oder ≥ 1100 bzw. ≥ 1150.

Unter Einbezug des Gesichtspunktes einer befriedigenden Anfangsselektivität der Zielproduktbildung bereits bei Inbetriebnahme des Katalysatorbetts beträgt F mit Vorzug ≤ 2500, häufig ≤ 2400, oder ≤ 2200.

Günstige Werte für F sind auch solche, die ≤ 2000, oder ≤ 1800, oder ≤ 1600 bzw. ≤ 1500 betragen.

D. h., erfindungsgemäß vorteilhafte Werte für F betragen 2500 ≥ F ≥ 850, oder 2450 ≥ F ≥ 900, oder 2400 ≥ F ≥ 950.

Erfindungsgemäß besonders vorteilhafte Werte für F betragen 1900 ≥ F ≥ 1000, oder 1800 ≥ F ≥ 1050.

Erfindungsgemäß ganz besonders vorteilhafte Werte für F betragen 1700 ≥ F ≥ 1100, oder 1500 ≥ F ≥ 1150.

Der stöchiometrische Koeffizient a beträgt erfindungsgemäß vorteilhaft 0,2 bis 2, besonders vorteilhaft 0,4 bis 1,5 und ganz besonders vorteilhaft 0,5 bis 1.

Der Partikeldurchmesser $d_{50}^{A1}$ beträgt erfindungsgemäß vorteilhaft $1,2\ \mu m \leq d_{50}^{A1} \leq 8\ \mu m$, besonders vorteilhaft $1,5\ \mu m \leq d_{50}^{A1} \leq 6\ \mu m$, sowie ganz besonders vorteilhaft $1,5\ \mu m \leq d_{50}^{A1} \leq 4\ \mu m$, bzw. $2\ \mu m \leq d_{50}^{A1} \leq 3\ \mu m$.

Der Partikeldurchmesser $d_{90}^{A2}$ beträgt erfindungsgemäß vorteilhaft $170\ \mu m \geq d_{90}^{A1} \geq 30\ \mu m$, , besonders vorteilhaft $150\ \mu m \geq d_{90}^{A2} \geq 40\ \mu m$, und ganz besonders vorteilhaft $130\ \mu m \geq d_{90}^{A2} \geq 50\ \mu m$.

Zur Bestimmung von Partikeldurchmesserverteilungen in Trockenpulvern sowie den aus diesen entnommenen Partikeldurchmessern wie z. B. $d_{10}$, $d_{50}$ und $d_{90}$, wurde (soweit nicht ausdrücklich etwas anders explizit erwähnt wird) das jeweilige feinteilige Pulver über eine Dispergierrinne in den Trockendispergierer Sympatec RODOS (Sympatec GmbH, System-Partikel-Technik, Am Pulverhaus 1, D-38678 Clausthal-Zellerfeld) geführt, dort mit Druckluft trocken dispergiert und im Freistrahl in die Messzelle geblasen. In dieser wird dann nach ISO 13320 mit dem Laserbeugungsspektrometer Malvern Mastersizer S (Malvern Instruments, Worcestshire WR 14 1AT, United Kingdom) die volumenbezogene Partikeldurchmesserverteilung bestimmt. Die als Messergebnis angegebenen Partikeldurchmesser $d_x$ sind dabei so definiert, dass X % des Gesamtpartikelvolumens aus Partikeln mit diesem oder einem kleinerem Durchmesser bestehen. Das heißt, (100-X) % des Gesamtpartikelvolumens bestehen aus Partikeln mit einem Durchmesser > $d_x$. Wird in dieser Schrift nicht ausdrücklich etwas anderes erwähnt, beziehen sich Partikeldurchmesserbestimmungen und daraus entnommene $d_x$ wie z. B $d_{90}^Q$, $d_{50}^{A1}$ und $d_{90}^{A2}$ auf einen bei der Bestimmung angewandten (die Stärke der Dispergierung des Trockenpulvers während der Messung bestimmenden) Dispergierdruck von 2 bar absolut.

Alle in dieser Schrift auf ein Röntgendiffraktogramm bezogenen Angaben beziehen sich auf ein unter Anwendung von Cu-K$\alpha$-Strahlung als Röntgenstrahlung erzeugtes Röntgendiffraktogramm (Diffraktometer Theta-Theta Bruker D8 Advance, Röhrenspannung: 40 kV, Röhrenstrom: 40 mA, Aperturblende V20 (variabel), Streustrahlblende V20 (variabel), Detektorblende (0,1 mm), Meßintervall (20 = 2 Theta): 0,02°, Meßzeit je Schritt: 2,4 s, Detektor: Si-Halbleiterdetektor).

Die Definition der Intensität eines Beugungsreflexes im Röntgendiffraktogramm bezieht sich in dieser Schrift auf die in der DE-A 198 35 247, sowie die in der DE-A 100 51 419 und DE-A 100 46 672 niedergelegte Definition.

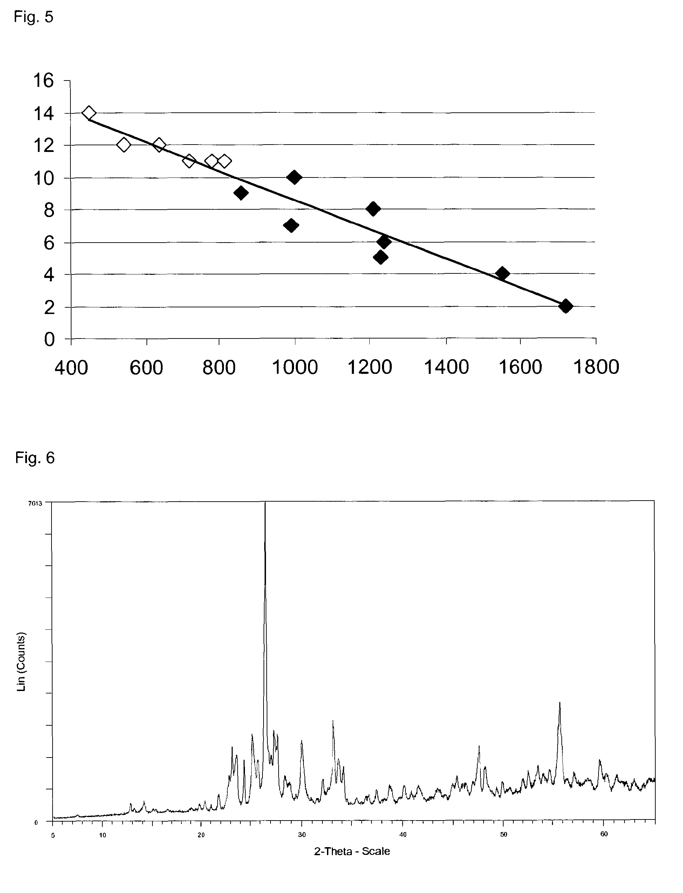

D.h., bezeichnet $A^1$ den Scheitelpunkt eines Reflexes 1 und bezeichnet $B^1$ in der Linie des Röntgendiffraktogramms bei Betrachtung entlang der zur 20-Achse senkrecht stehenden Intensitätsachse das nächstliegende ausgeprägte Minimum (Reflexschultern ausweisende Minima bleiben unberücksichtigt) links vom Scheitelpunkt $A^1$ und $B^2$ in entsprechender Weise das nächstliegende ausgeprägte Minimum rechts vom Scheitelpunkt $A^1$ und bezeichnet $C^1$ den Punkt, an dem eine vom Scheitelpunkt $A^1$ senkrecht zur 20-Achse gezogene Gerade eine die Punkte $B^1$ und $B^2$ verbindende Gerade schneidet, dann ist die Intensität des Reflexes 1 die Länge des Geradenabschnitts $A^1C^1$, der sich vom Scheitelpunkt $A^1$ zum Punkt $C^1$ erstreckt. Der Ausdruck Minimum bedeutet dabei einen Punkt, an dem der Steigungsgradient einer an die Kurve in einem Basisbereich des Reflexes 1 angelegten Tangente von einem negativen Wert auf einen positiven Wert übergeht, oder einen Punkt, an dem der Steigungsgradient gegen Null geht, wobei für die Festlegung des Steigungsgradienten die Koordinaten der 20-Achse und der Intensitätsachse herangezogen werden. Eine beispielhafte Durchführung einer Intensitätsbestimmung zeigt die Fig. 6 in der DE-A 100 46 672. Eingehende Ausführungen zur Intensitätsbestimmung von Röntgenbeugungsreflexen finden sich auch in der DE-A 101 22 027. Aussagen zu Halbwertsbreiten von Beugungslinien beziehen sich in dieser Schrift in entsprechender Weise auf die Länge des Geradenabschnitts, der sich zwischen den beiden Schnittpunkten H1 und H2 ergibt, wenn man in der Mitte des Geradenabschnitts $A^1C^1$ eine Parallele zur 20-Achse zieht, wobei $H^1$, $H^2$ den jeweils ersten Schnittpunkt dieser Parallelen mit der wie vorstehend definierten Linie des Röntgendiffraktogramms links und rechts von $A^1$ meinen. In der Regel betragen die Halbwertsbreiten von Röntgenbeugungsreflexen der Multielementoxid-I-Aktivmassen ≤ 1°, sowie meist ≤ 0,5°.

Alle Angaben in dieser Schrift zu spezifischen Oberflächen von Feststoffen beziehen sich auf Bestimmungen nach DIN 66131 (Bestimmung der spezifischen Oberfläche von Feststoffen durch Gasadsorption ($N_2$) nach Brunauer-Emmet-Teller (BET)), soweit nicht ausdrücklich etwas anderes erwähnt wird.

Das erfindungsgemäße Erfordernis, dass jede Quelle der von Sauerstoff verschiedenen Elemente des Anteils T = $[Mo_{12}Z^1_cZ^2_dFe_eZ^3_fZ^4gZ^5_hO_y]_1$ des Multielementoxids I im Verlauf der Herstellung der wässrigen Mischung M einen Zerteilungsgrad Q durchlaufen muss, der dadurch gekennzeichnet ist, dass sein Durchmesser $d_{90}^Q \leq 5$ μm beträgt, bringt zum Ausdruck, dass durchaus von einer grobkörnigeren Quelle (von einem grobkörnigeren Ausgangsmaterial) ausgegangen werden kann. Auf dem Weg der Einarbeitung einer solchen Quelle in die wässrige Mischung M, muss diese Quelle jedoch wenigstens einmal das Erfordernis $d_{90}^Q \leq 5$ μm erfüllen (selbstredend ist $d_{90}^Q$ stets >0 μm).

Das Erfordernis $d_{90}^Q \leq 5$ μm ist grundsätzlich dann erfüllt, wenn man eine Quelle in einem Lösungsmittel auflöst (z. B. in wässrigem Medium; der Begriff "Lösen" ist dabei im Sinn einer molekularen bzw. ionischen Lösung gemeint) und die dabei resultierende Lösung zur Herstellung der wässrigen Mischung M verwendet.

Dies ist dadurch bedingt, dass beim Lösen einer Quelle (Ausgangsverbindung, Ausgangssubstanz) in einem Lösungsmittel die Quelle im Lösungsmittel molekular bzw. ionisch zerteilt wird.

Das heißt, die in der Lösung befindliche größte geometrische Einheit der gelösten Ausgangssubstanz (Quelle) weist unabdingbar "molekulare" Ausmaße auf, die damit in notwendiger Weise wesentlich kleiner als 5 μm sind. Selbstverständlich kann in ein- und derselben Lösung aber auch mehr als eine Quelle (wobei eine Quelle aber auch mehr als ein Element des Anteils T enthalten und damit gleichzeitig Quelle für mehr als ein Element sein kann) eines Elements des Anteils T gelöst und die dabei resultierende Lösung zur Herstellung der wässrigen Mischung M verwendet werden.

Das Erfordernis $d_{90}^Q \leq 5$ μm ist aber auch dann erfüllt, wenn sich eine Quelle eines Elements des Anteils T in einem Lösungsmittel in kolloidaler Lösung befindet. Kolloidale Lösungen stellen eine Verbindung zwischen echten (molekularen oder ionischen) Lösungen und Suspensionen dar. In diesen kolloiddispersen Systemen befinden sich kleinere Anhäufungen von Molekülen oder Atomen, welche jedoch weder mit dem bloßen Auge, noch mit dem Mikroskop erkennbar sind.

Die kolloidale Lösung erscheint optisch völlig klar (wenn auch oft gefärbt), da die in ihr enthaltenen Teilchen nur einen Durchmesser von 1 bis 250 nm (vorzugsweise bis 150 nm und besonders bevorzugt bis 100 nm) haben, weshalb der zugehörige $d_{90}^Q$ in notwendiger Weise $\leq 5$ μm beträgt. Aufgrund der geringen Größe ist eine Abtrennung der kolloidal gelösten Partikel durch konventionelle Filtration nicht möglich. Sie können jedoch durch Ultrafiltration mit Membranen pflanzlichen, tierischen oder künstlichen Ursprungs (z. B. Pergament, Schweinsblase oder Cellophan) von ihrem "Lösungsmittel" abgetrennt werden. Im Gegensatz zu den "optisch leeren" echten (molekularen oder ionischen) Lösungen, kann ein Lichtstrahl nicht ohne Ablenkung durch eine kolloidale Lösung hindurchtreten. Der Lichtstrahl wird von den kolloidal gelösten Partikeln gestreut und abgelenkt. Um kolloidale Lösungen stabil zu halten und weitergehende Partikelagglomerationen zu verhindern, enthalten sie häufig Netz- und Dispergierhilfsmittel sowie andere Additive zugesetzt.

Beispielsweise kann das Element Silizium beim erfindungsgemäßen Verfahren in Form eines Kieselsols zur Herstellung der wässrigen Mischung M eingebracht werden. Kieselsole sind kolloidale Lösungen von amorphem Siliciumdioxid in Wasser. Sie sind wasserflüssig und enthalten keine sedimentierbaren Bestandteile. Ihr $SiO_2$-Gehalt kann bei oft jahrelanger Haltbarkeit (ohne Sedimentation) bis zu 50 Gew.-% und mehr betragen.

Das Erfordernis $d_{90}^Q \leq 5$ μm ist aber auch dann erfüllt, wenn eine Quelle z. B. trocken auf diese Partikelgröße zerkleinert (z. B. durch Mahlen) wird.

Grundsätzlich kann ein solches Pulver unmittelbar als solches zur Herstellung der innigen wässrigen Mischung M verwendet werden. Selbstverständlich kann es aber auch in einem flüssigen Medium suspendiert und dann in Form dieser Suspension zur Herstellung der wässrigen Mischung M verwendet werden.

Erfindungsgemäß bevorzugt beträgt $d_{90}^Q$ für alle zur Herstellung der wässrigen Mischung M verwendeten Quellen (Ausgangsverbindungen, Ausgangssubstanzen) $\leq 4$ μm oder $\leq 3$ μm, besonders bevorzugt $\leq 2$ μm oder $\leq 1$ μm und ganz besonders bevorzugt $\leq 0,8$ μm oder $\leq 0,5$ μm. Noch besser beträgt $d_{90}^Q$ für alle zur Herstellung der wässrigen Mischung M verwendeten Quellen (Ausgangsverbindungen, Ausgangssubstanzen) $\leq 0,3$ μm oder $\leq 0,2$ μm.

Besonders bevorzugt sind solche erfindungsgemäße Verfahren, bei denen im Verlauf der Herstellung der wässrigen Mischung M alle mitverwendeten Quellen der Elemente des Anteils T den Zustand einer kolloidalen oder einer echten (molekularen bzw. ionischen) Lösung durchlaufen (die dabei resultierenden wässrigen Mischungen M sollen in dieser Schrift als wässrige Mischungen M$^L$ bezeichnet werden).

Ganz besonders bevorzugt sind solche erfindungsgemäße Verfahren, bei denen im Verlauf der Herstellung der wässrigen Mischung M alle mitverwendeten Quellen der von Silizium verschiedenen Elemente des Anteils T den Zustand einer echten (molekularen bzw. ionischen) Lösung durchlaufen (die dabei resultierenden wässrigen Mischungen M sollen in dieser Schrift als wässrige Mischung M$^{L*}$ bezeichnet werden). Enthält die wässrige Mischung M darüber hinaus eine Quelle des Elementes Silizium, handelt es sich dabei mit Vorteil um eine kolloidale Lösung derselben (besonders bevorzugt um ein Kieselsol). Solche wässrigen Mischungen M sollen in dieser Schrift als wässrige Mischungen M$^{L**}$ bezeichnet werden.

Unter einer innigen wässrigen Mischung M sollen in dieser Schrift solche Mischungen M verstanden werden, deren beim Übergang von der wässrigen Mischung M in die feinteilige Ausgangsmasse A2 gasförmig entweichender Stoffanteil zu wenigstens 50 % seines Gewichtes, vorteilhaft zu wenigstens 60 % seines Gewichtes, besonders vorteilhaft zu wenigstens 70 % seines Gewichtes, ganz besonders vorteilhaft zu wenigstens 80 % seines Gewichtes und noch besser zu wenigstens 90 % seines Gewichtes aus Wasserdampf besteht. Neben Wasser kann der vorgenannte gasförmig entweichende Stoffanteil noch Verbindungen wie HCl, $HNO_3$, Kohlendioxid, Ammoniak, Alkohole (z. B. Methanol, Ethanol, Glykol und Glyzerin), Ketone wie z. B. Aceton oder andere in Wasser bei Normalbedingungen (1 atm, 25 °C) lösliche organische Verbindungen enthalten.

Als Quellen für die Elemente des Anteils T der gewünschten erfindungsgemäßen Multielementoxidaktivmasse I kommen prinzipiell solche Verbindungen in Betracht, bei denen es sich bereits um Oxide handelt und/oder um solche Verbindungen, die durch Erhitzen, wenigstens in Anwesenheit von molekularem Sauerstoff, in Oxide überführbar sind.

Neben den Oxiden kommen als solche Ausgangsverbindungen (Quellen) vor allem Halogenide, Nitrate, Formiate, Oxalate, Citrate, Acetate, Carbonate, Aminkomplexe, Ammoniumsalze und/oder Hydroxide (sowie die Hydrate der vorgenannten Salze) in Betracht.

Eine günstige Mo-Quelle ist Ammoniumheptamolybdattetrahydrat. Grundsätzlich ist aber auch z. B. Molybdäntrioxid einsetzbar. Erfindungsgemäß günstige Z$^1$-Quellen sind die Nitrate bzw. Nitrathydrate der Z$^1$-Elemente. Erfindungsgemäß vorteilhafte Z$^2$-Quellen sind die Hydroxide und Nitrate der Z$^2$-Elemente bzw. deren Hydrate. Für das Element Eisen wird beim erfindungsgemäßen Verfahren mit Vorteil ein Eisennitrathydrat verwendet.

Kieselsol bildet die erfindungsgemäß bevorzugte Si-Quelle. Erfindungsgemäß bevorzugte Lanthaniden sind Er, Tb, Ho, Eu, Tm, Nd, Lu, Dy, Gd, Ce und Sm. Als ihre Quelle werden vorzugsweise ebenso wie im Fall von La und Y die entsprechenden Nitrathydrate verwendet.

Neben den relevanten Quellen der Elemente des Anteils T des Multielementoxids I können in die jeweilige wässrige Mischung M auch noch Substanzen eingearbeitet werden, die wenigstens unter den Bedingungen der thermischen Behandlung der geometrischen Formkörper V unter Ausbildung der geometrischen Katalysatorformkörper K zu gasförmig entweichenden Verbindungen zerfallen und/oder zersetzt (chemisch umgesetzt) werden. Derartige Substanzen können beispielsweise als Porenbildner fungieren und zum Zweck der Einstellung der aktiven inneren Oberfläche einbezogen werden. Als solche (Hilfs)Substanzen kommen beispielsweise $NH_4OH$, $(NH_4)_2CO_3$, $NH_4HCO_3$, $NH_4NO_3$, Harnstoff, $NH_4CHO_2$, $H_2CO_3$, $HNO_3$, $H_2SO_4$, $NH_4CH_3CO_2$, $NH_4Cl$, $HCl$, $NH_4HSO_4$, $(NH_4)_2SO_4$, Ammoniumoxalat, Hydrate der vorgenannten Verbindungen sowie organische Substanzen wie z. B. Stearinsäure, Malonsäure, Ammoniumsalze der vorgenannten Säuren, Stärken (z. B. Kartoffelstärke und Maisstärke), Cellulose, gemahlene Nussschale, feinteiliges Kunststoffmehl (z. B. aus Polyethylen, Polypropylen), etc. in Betracht.

Erfindungsgemäß bevorzugt erfolgt die Erzeugung der feinteiligen Ausgangsmasse A2 aus der wässrigen Mischung M (insbesondere im Fall einer wässrigen Mischung $M^L$, oder $M^{L*}$, oder $M^{L**}$) durch Sprühtrocknung derselben. Das heißt, die wässrige Mischung M wird in diesem Fall zunächst in feinteilige Tröpfchen zerteilt und selbige daran anschließend getrocknet. Erfindungsgemäß bevorzugt erfolgt die Trocknung im Heißluftstrom. Grundsätzlich können zur vorgenannten Sprühtrocknung aber auch andere heiße Gase verwendet werden (z. B. Stickstoff, oder mit Stickstoff verdünnte Luft sowie sonstige inerte Gase).

Die Sprühtrocknung kann dabei grundsätzlich sowohl im Gleichstrom als auch im Gegenstrom der Tröpfchen zum heißen Gas erfolgen. Vorzugsweise erfolgt sie im Gegenstrom der Tröpfchen zum heißen Gas. Besonders bevorzugt im Heißluftgegenstrom. Typisch Gaseintrittstemperaturen liegen dabei im Bereich von 250 bis 450, vorzugsweise 270 bis 370 °C. Typische Gasaustrittstemperaturen liegen dabei im Bereich von 100 bis 160 °C.

Erfindungsgemäß bevorzugt wird die Sprühtrocknung dabei so durchgeführt, dass sich der für die feinteilige Ausgangsmasse A2 erwünschte Partikeldurchmesser $d_{90}^{A2}$ als Ergebnis der Sprühtrocknung unmittelbar einstellt (das heißt, der entsprechende $d_{90}$ des resultierenden Sprühpulvers ist), so dass das resultierende Sprühpulver unmittelbar die erfindungsgemäß einzusetzende Ausgangsmasse A2 bilden kann.

Ist der Zerteilungsgrad des resultierenden Sprühpulvers im Vergleich zum erwünschten $d_{90}^{A2}$ zu klein, so kann selbiges z. B. durch nachfolgendes Kompaktieren auf den für die Ausgangsmasse A2 erwünschten Zerteilungsgrad zielgerichtet vergröbert werden. Umgekehrt kann das bei der Sprühtrocknung unmittelbar resultierende Sprühpulver durch Mahlen bei Bedarf auch auf den für die Ausgangsmasse A2 erwünschten Zerteilungsgrad verfeinert werden.

Selbstverständlich kann die innige wässrige Mischung M aber auch zunächst durch konventionelles Eindampfen (vorzugsweise bei vermindertem Druck; die Trocknungstemperatur sollte im Regelfall 150 °C nicht überschreiten) getrocknet und die dabei resultierende Trockenmasse durch nachfolgendes Zerkleinern auf den erfindungsgemäß erforderlichen Zerteilungsgrad $d_{90}^{A2}$ eingestellt werden. Grundsätzlich kann die Trocknung der wässrigen Mischung M beim erfindungsgemäßen Verfahren aber auch durch Gefriertrocknung erfolgen.

$Z^1$ ist beim erfindungsgemäßen Verfahren bevorzugt ausschließlich Co.

$Z^2$ ist beim erfindungsgemäßen Verfahren vorzugsweise K, Cs und/oder Sr, besonders bevorzugt K.

$Z^4$ ist beim erfindungsgemäßen Verfahren vorzugsweise Si.

Der stöchiometrische Koeffizient b beträgt vorteilhaft 0,5 bis 4 oder bis 3, besonders vorteilhaft 1 bis 2,5 und ganz besonders vorteilhaft 1,5 bis 2,5.

Der stöchiometrische Koeffizient c beträgt vorzugsweise 3 bis 8, besonders vorteilhaft 4 bis 7 und ganz besonders vorteilhaft 5 bis 6.

Der stöchiometrische Koeffizient d beträgt vorteilhaft 0,02 bis 2 und besonders vorteilhaft 0,03 bis 1 bzw. 0,05 bis 0,5.

Der stöchiometrische Koeffizient e beträgt vorteilhaft 0,1 bis 4,5, vorzugsweise 0,5 bis 4 und besonders bevorzugt 1 bis 4 oder 2 bis 4.

Der stöchiometrische Koeffizient g beträgt vorzugsweise > 0 bis 10, besonders bevorzugt 0,1 bis 8 oder 0,2 bis 7, ganz besonders bevorzugt 0,3 bis 6 oder 0,4 bis 5, und am vorteilhaftesten 0,5 bis 3 oder 1 bis 3.

Die stöchiometrischen Koeffizienten h und f können beide gleichzeitig 0 betragen, aber auch unabhängig voneinander von 0 verschiedene Werte annehmen. Mit Vorteil enthält der Anteil T kein Bi.

Das heißt, die Ausführungsbeispiele B1 bis B8 und die Vergleichsbeispiele V1 bis V6 (einschließlich der nachfolgenden Verwendung als Katalysatoren für die Propenpartialoxidation) können unter ansonsten unveränderten Bedingungen auch unter Verwendung feinteiliger Ausgangsmassen A2 durchgeführt werden, deren zugehörige Stöchio-

metrie I* $Mo_{12}Co_{6,0}Fe_{3,0}Si_{1,6}K_{0,08}$, oder $Mo_{12}Co_{6,5}Fe_{3,0}Si_{1,6}K_{0,08}$, oder $Mo_{12}Co_{7,0}Fe_{3,0}Si_{1,6}K_{0,08}$, oder $M_{12}Co_{5,0}Fe_{3,0}Si_{1,6}K_{0,08}$, oder $Mo_{12}Co_{4,5}Fe_{3,0}Si_{1,6}K_{0,08}$, oder $Mo_{12}Co_{5,5}Fe_{2,5}Si_{1,6}K_{0,08}$, oder $Mo_{12}Co_{5,5}Fe_{3,5}Si_{1,6}K_{0,08}$, oder $Mo_{12}Co_{5,5}Fe_{4,0}Si_{1,6}K_{0,08}$, oder $Mo_{12}Co_{7,0}Fe_{4,0}Si_{1,6}K_{0,08}$, oder $Mo_{12}Co_{6,0}Fe_{3,5}Si_{1,6}K_{0,08}$, oder $Mo_{12}Co_{7,0}Fe_{2,0}Si_{1,6}K_{0,08}$, oder $Mo_{12}Co_{6,0}Fe_{2,5}Si_{1,6}K_{0,08}$, oder $Mo_{12}Co_{5,5}Fe_{3,0}Si_{0,5}K_{0,08}$, oder $Mo_{12}Co_{5,5}Fe_{3,0}Si_{3}K_{0,08}$, oder $Mo_{12}Co_{5,5}Fe_{3,0}Si_{1,6}K_{0,04}$, oder $Mo_{12}Co_{5,5}Fe_{3,0}Si_{1,6}K_{0,2}$, oder $Mo_{12}Ni_{3,0}Co_{2,5}Fe_{3,0}Si_{1,6}K_{0,08}$, oder $Mo_{12}Ni_{3,0}Co_{4}Fe_{3,0}Si_{1,6}K_{0,08}$, oder $Mo_{12}Sb_{0,2}Co_{4,2}Fe_{1,4}Zn_{0,2}W_{0,1}K_{0,06}$, oder $Mo_{12}Sb_{0,2}Co_{4,2}Fe_{1,4}Zn_{0,2}Bi_{0,9}W_{0,1}K_{0,06}$, oder $Mo_{12}Ni_{2,8}Co_{5,2}Fe_{1,8}K_{0,1}$, oder $Mo_{12}Ni_{2,8}Co_{5,2}Fe_{1,8}Bi_{1,7}K_{0,1}$, oder $Mo_{12}CO_{5}Fe_{1}Ni_{3}W_{0,5}K_{0,1}$, oder $Mo_{12}Co_{5}Fe_{1}Ni_{3}W_{0,5}Bi_{1}K_{0,1}$, oder $Mo_{12}Co_{5,5}Fe_{3,0}Bi_{0,02}Si_{1,6}K_{0,08}$, oder $Mo_{12}Co_{5,5}Fe_{3,0}Bi_{0,05}Si_{1,6}K_{0,08}$, oder $Mo_{12}Co_{5,5}Fe_{3,0}Bi_{0,1}Si_{1,6}K_{0,08}$, oder $Mo_{12}Co_{5,5}Fe_{3,0}Bi_{0,2}Si_{1,6}K_{0,08}$, oder $M0_{12}Co_{5,5}Fe_{3,0}Bi_{0,5}Si_{1,6}K_{0,08}$, oder $Mo_{12}Co_{7}Fe_{3,0}Bi_{0,06}Si_{1,6}K_{0,08}$, oder $Mo_{12}Co_{5,5}Fe_{3,0}Gd_{0,05}Si_{1,6}K_{0,08}$, oder $Mo_{12}Co_{5,5}Fe_{3,0}Y_{0,05}Si_{1,6}K_{0,08}$, oder $Mo_{12}Co_{5,5}Fe_{3,0}Er_{0,05}Si_{1,6}K_{0,08}$, oder $Mo_{12}Co_{5,5}Fe_{3,0}Er_{0,25}Si_{1,6}K_{0,08}$, oder $Mo_{12}Co_{5,5}Fe_{3,0}Sm_{0,05}Si_{1,6}K_{0,08}$, oder $Mo_{12}Co_{5,5}Fe_{3,0}Eu_{0,05}Si_{1,6}K_{0,08}$, oder $Mo_{12}Co_{5,5}Fe_{3,0}Dy_{0,05}Si_{1,6}K_{0,08}$, oder $Mo_{12}Co_{5,5}Fe_{3,0}Yb_{0,05}Si_{1,6}K_{0,08}$, oder $Mo_{12}Co_{5,5}Fe_{3,0}Tb_{0,05}Si_{1,6}K_{0,08}$, oder $Mo_{12}Co_{5,5}Fe_{3,0}Ho_{0,05}Si_{1,6}K_{0,08}$, oder $Mo_{12}Co_{5,5}Fe_{3,0}Ce_{0,05}Si_{1,6}K_{0,08}$, oder $Mo_{12}Co_{5,5}Fe_{3,0}La_{0,05}Si_{1,6}K_{0,08}$ ist. In der feinteiligen Ausgangsmasse A2-4 von Beispiel B 4 nicht enthaltene, von den vorstehenden Stöchiometrien aber umfasste Elemente werden dabei unter Verwendung ihrer Nitrathydrate als Quelle in der Lösung B aufgelöst. Abweichend hiervon wird W als Ammoniumparawolframat zur Lösung A zugesetzt. Der Stöchiometrische Koeffizient a kann dabei in allen Fällen auch 0,5, oder 0,7, oder 0,8 sein. Gleichzeitig kann in allen vorgenannten Fallgestaltungen $d_{50}^{A1}$ = 2,4 μm und $d_{90}^{A2}$ = 68 μm sein.

Die Vorbildung des feinteiligen Mischoxids $Bi_1W_bO_x$ kann in an sich bekannter Weise erfolgen (vgl. z. B. EP-A 575 897, DE-A 3338380, EP-A 835, WO 02/24620, WO 2007/017431, die Deutsche Anmeldung mit dem Aktenzeichen 102007003778.5, WO 2005/030393 und die Deutsche Anmeldung mit dem Aktenzeichen 102008040093.9).

In der Regel wird man dabei wenigstens eine Quelle des Elementes Bi und wenigstens eine Quelle des Elements W (d. h., wenigstens eine das Element Bi enthaltende Ausgangsverbindung und wenigstens eine das Element W enthaltende Ausgangsverbindung) in wässrigem Medium miteinander innig vermischen, das wässrige Gemisch trocknen und die dabei resultierende Trockenmasse bei Temperaturen im Bereich von 400 bis 900 °C (vorzugsweise 600 bis 900 °C und besonders bevorzugt 700 bis 900 °C) kalzinieren (thermisch behandeln) sowie durch Zerteilen des dabei resultierenden Kalzinats unter Erhalt der feinteiligen Ausgangsmasse A1 den erfindungsgemäß erforderlichen Partikeldurchmesser $d_{50}^{A1}$ einstellen. Als Quellen des Bi und W kommen grundsätzlich solche Verbindungen in Betracht, bei denen es sich bereits um Oxide dieser Elemente handelt, oder solche Verbindungen, die durch Erhitzen, wenigstens in Anwesenheit von molekularem Sauerstoff, in Oxide überführbar sind.

Vorzugsweise wird man wasserlösliche Salze des Wismuts wie Nitrate, Carbonate, Hydroxide und/oder Acetate mit Wolframsäure (die in Wasser im wesentlichen unlösliche Wolframsäure wird dabei vorzugsweise als feinteiliges Pulver eingesetzt, dessen $d_{90}$ anwendungstechnisch zweckmäßig ≤ 5 μm oder ≤ 2 μm, vorzugsweise 0,1 bis 1 μm beträgt) und/oder deren Ammoniumsalze in Wasser mischen, die wässrige Mischung trocknen (vorzugsweise sprühtrocknen) und die getrocknete Masse anschließend wie beschrieben thermisch behandeln.

Erfolgte die Trocknung durch Sprühtrocknung, wird man das resultierende Sprühpulver vorab der Kalzination vorteilhaft vergröbern (z. B. anwendungstechnisch vorteilhaft unter Zusatz von bis zu 20 Gew.-% Wasser anteigen und z. B. mittels eines Extruders zu für Kalzinationszwecke einfacher handhabbaren Stränglingen extrudieren; diese werden nachfolgend getrocknet und danach kalziniert). Üblicherweise erfolgt die thermische Behandlung im Luftstrom (z. B. im Fall der vorgenannten Stränglinge in einem Drehrohrofen, wie er in der DE-A 103 25 487 beschrieben ist). Das Zerteilen des resultierenden kalzinierten Mischoxids auf den erfindungsgemäß wesentlichen Partikeldurchmesser $d_{50}^{A1}$ wird man normalerweise durch Mahlen in Mühlen bewirken. Bei Bedarf wird das Mahlgut nachfolgend auf den gewünschten Zerteilungsgrad klassiert.

Bevorzugte im Rahmen des erfindungsgemäßen Verfahrens vorab gebildete Mischoxide $Bi_1W_bO_x$ sind die Mischoxide $Bi_1W_{2,5}O_9(1/2B_2W_2O_9•\ 1,5\ WO_3)$, $Bi_1W_3O_{10,5}$ $(1/2Bi_2W_2O_9•\ 2WO_3)$, $Bi_1W_4O_{13,5}$ $(1/2Bi_2W_2O_9•\ 3WO_3)$, $Bi_1W_{0,5}O_3$, $Bi_1W_1O_{4,5}$ $(1/2Bi_2W_2O_9)$, $Bi_1W_2O_{7,5}$ $(1/2Bi_2W_2O_9•\ 1WO_3)$ und $Bi_1W_{1,5}O_6$ $(1/2Bi_2W_2O_9•\ 1/2WO_3)$, unter denen das $Bi_1W_2O_{7,5}$ erfindungsgemäß ganz besonders bevorzugt wird (die Ausführungsbeispiele B1 bis B8 sowie die Vergleichsbeispiele V1 bis V6 (einschließlich ihrer Verwendung für die Propenpartialoxidation) können daher auch unter Verwendung von $Bi_1W_{1,5}O_6$, oder $Bi_1W_{2,5}O_9$, oder $Bi_1W_3O_{10,5}$, oder $Bi_1W_4O_{13,5}$, oder $Bi_1W_{0,5}O_3$, oder $Bi_1W_1O_{4,5}$ als feinteilige Ausgangsmasse A1 entsprechender Körnung wie das verwendete feinteilige $Bi_1W_2O_{7,5}$ ausgeführt werden).

Ebenso wie in die wässrige Mischung M können auch in das wässrige Gemisch der wenigstens einen Bi- sowie der wenigstens einen W-Quelle im Rahmen der Herstellung des Mischoxids $Bi_1W_bO_x$ zusätzlich Substanzen eingearbeitet werden, die unter den Bedingungen der zur Ausbildung des Mischoxids $Bi_1W_bO_x$ angewandten thermischen

Behandlung zu gasförmig entweichenden Verbindungen zerfallen und/oder zersetzt (chemisch umgesetzt) werden. Derartige Substanzen können beispielsweise als Porenbildner fungieren und zum Zweck der Beeinflussung der aktiven inneren Oberfläche des Mischoxids $Bi_1W_bO_x$ einbezogen werden.

Als solche (Hilfs)Substanzen kommen beispielsweise $NH_4OH$, $(NH_4)_2CO_3$, $NH_4HCO_3$, $NH_4NO_3$, Harnstoff, $NH_4CHO_2$, $H_2CO_3$, $HNO_3$, $H_2SO_4$, $NH_4CH_3CO_2$, $NH_4HSO_4$, $NH_4Cl$, $HCl$, $(NH_4)_2SO_4$, Ammoniumoxalat, Hydrate der vorgenannten Verbindungen sowie organische Substanzen wie z. B. Stearinsäure, Malonsäure, Ammoniumsalze der vorgenannten Säuren, Stärken (z. B. Kartoffelstärke und Maisstärke), Cellulose, gemahlene Nussschale, feinteiliges Kunststoffmehl (z. B. Polyethylen, Polypropylen), etc. in Betracht.

Bei der Herstellung der feinteiligen Ausgangsmasse A3 aus den feinteiligen Ausgangsmassen A1 und A2 werden anwendungstechnisch zweckmäßig, aber nicht in notwendiger Weise, feinteilige Formgebungshilfsmittel mitverwendet.

Diese können bereits vorab einer Vermischung der feinteiligen Ausgangsmassen A1 und A2 in beide dieser feinteiligen Ausgangsmassen oder in nur eine der beiden feinteiligen Ausgangsmassen A1, A2 eingemischt werden.

Selbstverständlich können die feinteiligen Formgebungshilfsmittel aber auch oder nur (erst) in das feinteilige Gemisch aus feinteiliger Ausgangsmasse A1 und feinteiliger Ausgangsmasse A2 eingemischt werden.

Zur Gruppe der feinteiligen Formgebungshilfsmittel (insbesondere dann, wenn diese Elemente $Z^4$ enthalten, werden die Formgebungshilfsmittel mit einem $d_{90} > 5$ $\mu m$ eingesetzt; andere von Sauerstoff verschiedene Elemente des Multielementoxids I enthalten sie üblicherweise nicht) gehören zunächst die sogenannten Antibackmittel.

Hierbei handelt es sich um feinteilige Materialien, die anwendungstechnisch vorteilhaft mitverwendet werden, um im Rahmen des Vermischens z. B. eine Reagglomeration (ein "Zusammenbacken") von Partikeln innerhalb der Ausgangsmasse A1 und/oder innerhalb der Ausgangsmasse A2 weitestgehend zu unterdrücken, da eine solche Reagglomeration den wirksamen Partikeldurchmesser beeinflussen könnte. Eine erfindungsgemäß bevorzugte Gruppe an feinteiligen Antibackmitteln bilden feinteilige hydrophobisierte Kieselsäuren, insbesondere feinteilige hydrophobisierte synthetische Kieselsäuren (Siliziumdioxide). Synthetische Kieselsäuren können einerseits unmittelbar pyrogen aus Sand und andererseits durch Fällungsreaktionen aus Wasserglas erzeugt werden. Insbesondere synthetische Kieselsäuren sind auf Grund ihrer oberflächenständigen OH-Gruppen hydrophil, d. h., sie werden durch Wasser benetzt. Zum Beispiel durch Reaktion dieser oberflächenständigen OH-Gruppen mit Chlorsilanen lassen sich sowohl aus den pyrogenen als auch aus den Fällungskieselsäuren hydrophobisierte Produkte herstellen. Beispielsweise kann die Hydrophobisierung durch Umsetzung mit Dimethyldichlorsilan im Beisein von Wasserdampf bei ca. 400 °C in einem Fließbettreaktor erfolgen (wird vorzugsweise bei pyrogenen Kieselsäuren angewendet).

Insbesondere bei Fällungskieselsäuren wird das Chlorsilan der Fällsuspension bei einer Temperatur von 50 bis 90°C unter gründlichem Rühren zugegeben. Anschließend folgen Filtration, Neutralwaschen mit Wasser, Trocknen der Filterkuchen und Tempern bei 300 bis 400°C. In H. Brunner, D. Schutte, Chem. Ing. Techn. 89, 437 (1965) sowie in DT 2435860 und DT 1117245 wird die Herstellung hydrophobisierter feinteiliger Kieselsäuren näher beschrieben. Handelsprodukte von hydrophobisierten Fällungskieselsäuren bilden z.B. die SIPERNAT®-Marken.

Erfindungsgemäß bevorzugt wird als feinteiliges Antibackmittel Sipernat® D17 der Firma Degussa bzw. der Fa. EVONIK Industries mitverwendet. Sipernat® D17 enthält auf sein Gewicht bezogen etwa 2 Gew.-% an chemisch gebundenem Kohlenstoff und wird von Wasser nicht benetzt. Sein Rüttelgewicht (gemäß ISO 787-11) beträgt 150 g/l. Sein $d_{50}$-Wert beträgt 10 $\mu m$ (Laserbeugung nach ISO 13320-1) und die spezifische Oberfläche (Stickstoffadsorption nach ISO 5794-1, Annex D) beträgt 100 $m^2/g$.

Mit Vorteil wird feinteiliges Antibackmittel wie z.B. Sipernat®D17 in die feinteilige Ausgangsmasse A1 eingemischt, bevor diese mit der feinteiligen Ausgangsmasse A2 zur feinteiligen Ausgangsmasse A3 vermischt wird. In der Regel liegt die Zusatzmenge an feinteiligem Antibackmittel dabei bei 0,1 bis 3 Gew.-%, bezogen auf das Gewicht der feinteiligen Ausgangsmasse A1.

Der Zusatz von Antibackmittel mindert auch den für eine homogene Vermischung der beiden Ausgangsmassen A1 und A2 erforderlichen Energieeintrag, was sich insbesondere auf den Erhalt der Partikelgröße der feinteiligen Ausgangsmasse A2 beim Vermischen vorteilhaft auswirkt.

Erfolgt die erfindungsgemäße Formung der feinteiligen Ausgangsmasse A3 zu den geometrischen Formkörpern V erfindungsgemäß vorteilhaft durch Verdichten (Komprimieren oder Kompaktieren), ist es anwendungstechnisch zweckmäßig, der feinteiligen Ausgangsmasse A3 als weitere feinteilige Formgebungshilfsmittel Gleitmittel wie z.B. Graphit, Ruß, Polyethylenglykol, Polyacrylsäure, Stearinsäure, Stärke, Mineralöl, Pflanzenöl, Wasser, Bortrifluorid und/oder Bornitrid zuzugeben. Eine Mitverwendung von Gleitmitteln im Rahmen einer entsprechenden Formgebung findet sich z.B. in den Schriften DE-A 102007004961, WO 2005/030393, US-A 2005/0131253, WO 2007/017431, DE-A 102007005606 und in der deutschen Anmeldung Nr. 102008040093.9 beschrieben. Erfindungsgemäß bevorzugt wird ausschließlich feinteiliger Graphit als Gleitmittel mitverwendet. Bevorzugt zugesetzte Graphite sind Asbury 3160 und Asbury 4012 der Firma Asbury Graphite Mills, Inc. New Jersey 08802, USA und Timrex® T44 der Firma Timcal Ltd., 6743 Bodio, Schweiz.

Mit Vorteil wird der feinteilige Graphit (typische $d_{90}$-Werte erfindungsgemäß geeigneter Graphite betragen 30 bis 300 $\mu m$) erst dem Gemisch aus feinteiliger Ausgangsmasse A1 und feinteiliger Ausgangsmasse A2 zugesetzt. Er kann jedoch auch vorab der Vermischung der beiden feinteiligen Ausgangsmassen A1, A2 in jede derselben (oder in nur eine der beiden) eingemischt werden. Bezogen auf das Gewicht der feinteiligen Ausgangsmasse A3 kann diese z. B. bis zu 15 Gew.-% an feinteiligem Gleitmittel enthalten. Meist liegt der Gleitmittelgehalt in der feinteiligen Ausgangsmasse A3 jedoch bei ≤ 9 Gew.-%, vielfach bei ≤ 5 Gew.-%, oft bei ≤ 4 Gew.-%; dies insbesondere dann, wenn das feinteilige Gleitmittel Graphit ist. In der Regel beträgt die vorgenannte Zusatzmenge ≥ 0,5 Gew.-%, meist ≥ 2,5 Gew.-%.

Bei Bedarf können der feinteiligen Ausgangsmasse A3 als weitere Formgebungshilfsmittel noch feinteilige Verstärkungsmittel wie Mikrofasern aus Glas, Asbest, Siliciumcarbid oder Kaliumtitanat zugesetzt werden, die sich nach Beendigung der Formgebung durch Verdichten förderlich auf den Zusammenhalt des erhaltenen Komprimats (des resultierenden Formkörpers V) auswirken.

Im Rahmen der erfindungsgemäßen thermischen Behandlung der Formkörper V, bei der die Katalysatorformkörper K erwachsen, können mitverwendete Formgebungshilfsmittel sowohl im resultierenden Katalysatorformkörper K erhalten bleiben, als auch durch thermische und/oder chemische Zersetzung zu gasförmigen Verbindungen (z.B. CO, $CO_2$) wenigstens teilweise gasförmig aus diesen entweichen. Im Katalysatorformkörper K verbleibende Formgebungshilfsmittel wirken im Rahmen einer katalytischen Verwendung desselben in selbigem im Wesentlichen ausschließlich als die Multielementoxid-I-Aktivmasse verdünnend.

Im Regelfall erfolgt die Verdichtung der feinteiligen Ausgangsmasse A3 zur gewünschten Geometrie des Formkörpers V (des geometrischen Katalysatorvorläuferformkörpers) durch Einwirkung äußerer Kräfte (Druck) auf das feinteilige Vorläufergemisch. Der dabei anzuwendende Formgebungsapparat bzw. die dabei anzuwendende Formgebungsmethode unterliegt keiner Beschränkung.

Beispielsweise kann die verdichtende Formgebung durch Strangpressen, Tablettieren oder Extrudieren erfolgen. Dabei wird die feinteilige Ausgangsmasse A3 vorzugsweise anfasstrocken eingesetzt. Sie kann jedoch z. B. bis zu 10 % ihres Gesamtgewichts Substanzen zugesetzt enthalten, die bei Normalbedingungen (25°C, 1 atm) flüssig sind. Auch kann die feinteilige Ausgangsmasse A3 feste Solvate (z.B. Hydrate) enthalten, die solche flüssigen Substanzen in chemisch und/oder physikalisch gebundener Form aufweisen. Selbstverständlich kann die feinteilige Ausgangsmasse A3 aber auch völlig frei von solchen Substanzen sein.

Erfindungsgemäß bevorzugtes Formgebungsverfahren durch Verdichten der feinteiligen Ausgangsmasse A3 ist die Tablettierung. Die Grundzüge des Tablettierens sind z.B. in "Die Tablette", Handbuch der Entwicklung, Herstellung und Qualitätssicherung, W.A. Ritschel und A. Bauer-Brandl, 2. Auflage, Edition Verlag Aulendorf, 2002 beschrieben und in völlig entsprechender Weise auf ein erfindungsgemäßes Tablettierverfahren übertragbar.

Mit Vorteil wird eine erfindungsgemäße Tablettierung wie in den Schriften WO 2005/030393, Deutsche Anmeldung Nr. 102008040093.9, Deutsche Anmeldung Nr. 102008040094.7 und WO 2007/017431 beschrieben durchgeführt.

Anstatt die feinteilige Ausgangsmasse A3 als solche unmittelbar zur gewünschten Geometrie des Formkörpers V zu verdichten (in einem einzigen Verdichtungsschritt), ist es erfindungsgemäß häufig zweckmäßig, als einen ersten Formgebungsschritt zunächst eine Zwischenkompaktierung durchzuführen, um die feinteilige Ausgangsmasse A3 zu vergröbern (in der Regel auf Partikeldurchmesser von 100 bis 2000 μm, bevorzugt 150 bis 1500 μm, besonders bevorzugt 400 bis 1250 μm, oder 400 bis 1000 μm, oder 400 bis 800 μm).

Dabei kann bereits vor der Zwischenkompaktierung z.B. feinteiliges Gleitmittel (z.B. Graphit) zugesetzt werden. Anschließend erfolgt auf der Grundlage des vergröberten Pulvers die endgültige Formgebung, wobei bei Bedarf zuvor nochmals z.B. feinteiliges Gleitmittel (z.B. Graphit) sowie gegebenenfalls weiteres Formgebungs- und/oder Verstärkungshilfsmittel zugegeben werden kann.

Ebenso wie der zur Verdichtung der feinteiligen Ausgangsmasse A3 zu verwendende Formgebungsapparat bzw. die dabei anzuwendende Formgebungsmethode, unterliegt auch die angestrebte Geometrie der resultierenden Formkörper V beim erfindungsgemäßen Verfahren keiner Beschränkung. D.h., die Katalysatorvorläuferformkörper (die Formkörper V) können sowohl regelmäßig als auch unregelmäßig geformt sein, wobei regelmäßig geformte Formkörper V in der Regel erfindungsgemäß bevorzugt sind.

Beispielsweise kann der Formkörper V beim erfindungsgemäßen Verfahren Kugelgeometrie aufweisen. Dabei kann der Kugeldurchmesser z.B. 2 bis 10 mm, oder 4 bis 8 mm betragen. Die Geometrie des Katalysatorvorläuferformkörpers (des Formkörpers V) kann aber auch vollzylindrisch oder hohlzylindrisch (ringförmig) sein. In beiden Fällen können Außendurchmesser (A) und Höhe (H) z.B. 2 bis 10 mm, oder 2 bzw. 3 bis 8 mm betragen. Im Fall von Vollzylindern kann der Außendurchmesser auch 1 bis 10 mm betragen. Im Fall von Hohlzylindern (Ringen) ist in der Regel eine Wandstärke von 1 bis 3 mm zweckmäßig. Selbstverständlich kommen als Katalysatorvorläufergeometrie aber auch alle diejenigen Geometrien in Betracht, die in der WO 02/062737 offenbart und empfohlen werden.

Die im Rahmen einer Verdichtung von feinteiliger Ausgangsmasse A3 angewandten Formgebungsdrucke werden beim erfindungsgemäßen Verfahren im Allgemeinen 50 kg/cm² bis 5000 kg/cm² betragen. Vorzugsweise betragen die Formgebungsdrucke 200 bis 3500 kg/cm², besonders bevorzugt 600 bis 25000 kg/cm².

Insbesondere im Fall von ringförmigen Formkörpern V soll die formgebende Verdichtung beim erfindungsgemäßen Verfahren der Lehre der Schriften Deutsche Anmeldung Nr. 102008040093.9, Deutsche Anmeldung Nr. 102008040094.7 und WO 2005/030393 folgend so durchgeführt werden, dass die Seitendruckfestigkeit SD des resultierenden ringförmigen Formkörpers V 12 N $\leq$ SD $\leq$ 25 N beträgt. Vorzugsweise beträgt SD $\geq$ 13 N und $\leq$ 24 N, bzw. $\geq$ 14 N und $\leq$ 22 N sowie ganz besonders bevorzugt $\geq$ 15 N und $\leq$ 20 N.

Die experimentelle Bestimmung der Seitendruckfestigkeit wird dabei wie in den Schriften WO 2005/030393 sowie WO 2007/017431 beschrieben durchgeführt. Selbstverständlich sind ringähnliche Formkörper V, wie sie die Deutsche Anmeldung Nr. 102008040093.9 empfiehlt, erfindungsgemäß ganz besonders bevorzugt. Die Stirnfläche von ringförmigen oder ringähnlichen Formkörpern V kann beim erfindungsgemäßen Verfahren sowohl gekrümmt als auch nicht gekrümmt sein (vgl. insbesondere DE-A 102007004961, EP-A 184 790 und die deutsche Anmeldung Nr. 102008040093.9). Bei der Ermittlung der Höhe solcher geometrischer Formkörper V wird eine solche Krümmung nicht berücksichtigt.

Erfindungsgemäß erhältliche Katalysatorformkörper K, die durch thermische Behandlung von Formkörpern V hergestellt wurden, welche durch Verdichtung von feinteiliger Ausgangsmasse A3 erhalten worden sind, werden als Vollkatalysatoren (Vollkatalysatorformkörper K) bezeichnet.

Erfindungsgemäß besonders vorteilhafte Ringgeometrien von durch Verdichten von feinteiliger Ausgangsmasse A3 erhältlichen Formkörpern V erfüllen die Bedingung H/A = 0,3 bis 0,7. Besonders bevorzugt ist H/A = 0,4 bis 0,6.

Weiterhin ist es für die vorgenannten ringförmigen Formkörper V erfindungsgemäß günstig, wenn das Verhältnis I/A (wobei I der Innendurchmesser der Ringgeometrie ist) 0,3 bis 0,7, vorzugsweise 0,4 bis 0,7 beträgt.

Besonders vorteilhaft sind vorgenannte Ringgeometrien, wenn sie gleichzeitig eines der vorteilhaften H/A-Verhältnisse und eines der vorteilhaften I/A-Verhältnisse aufweisen. Solche möglichen Kombinationen sind z.B. H/A = 0,3 bis 0,7 und I/A = 0,3 bis 0,8 oder 0,4 bis 0,7. Alternativ kann H/A 0,4 bis 0,6 und I/A gleichzeitig 0,3 bis 0,8 oder 0,4 bis 0,7 betragen. Ferner ist es für die relevanten Ringgeometrien günstig, wenn H 2 bis 6 mm und vorzugsweise 2 bis 4 mm beträgt. Weiterhin ist es vorteilhaft, wenn A bei den Ringen 4 bis 8 mm, vorzugsweise 4 bis 6 mm beträgt. Die Wandstärke erfindungsgemäß bevorzugter Ringgeometrien beträgt 1 bis 1,5 mm.

Mögliche Ringgeometrien für vorgenannte ringförmige Formkörper V sind somit (A x H x I) 5 mm x 2 mm x 2 mm, oder 5 mm x 3 mm x 2 mm, oder 5 mm x 3 mm x 3 mm, oder 5,5 mm x 3 mm x 3,5 mm, oder 6 mm x 3 mm x 4 mm, oder 6,5 mm x 3 mm x 4,5 mm, oder 7 mm x 3 mm x 5 mm, oder 7 mm x 7 mm x 3 mm, oder 7 mm x 7 mm x 4 mm.

Die thermische Behandlung von erfindungsgemäßen Formkörpern V (insbesondere ringförmiger Formkörper V; alles Nachfolgende gilt insbesondere für ihre thermische Behandlung) unter Erhalt der geometrischen Katalysatorformkörper K erfolgt im Rahmen des erfindungsgemäßen Verfahrens in der Regel bei Temperaturen (damit ist in dieser Schrift die Temperatur innerhalb des Kalzinationsgutes gemeint), die 350°C überschreiten. Normalerweise wird im Rahmen der thermischen Behandlung die Temperatur von 650°C jedoch nicht überschritten. Erfindungsgemäß vorteilhaft wird im Rahmen der thermischen Behandlung die Temperatur von 600°C, bevorzugt die Temperatur von 550°C und besonders bevorzugt die Temperatur von 500°C nicht überschritten.

Ferner wird im Rahmen der thermischen Behandlung der Formkörper V vorzugsweise die Temperatur von 380°C, mit Vorteil die Temperatur von 400°C, mit besonderem Vorteil die Temperatur von 420°C und ganz besonders bevorzugt die Temperatur von 440°C überschritten. Dabei kann die thermische Behandlung in ihrem zeitlichen Ablauf auch in mehrere Abschnitte gegliedert sein. Beispielsweise kann zunächst eine thermische Behandlung bei einer Temperatur (Phase 1) von 150 bis 350°C, vorzugsweise 220 bis 290°C, und daran anschließend eine thermische Behandlung bei einer Temperatur (Phase 2) von 400 bis 600°C, vorzugsweise 430 bis 550°C durchgeführt werden.

Normalerweise nimmt die thermische Behandlung der Formkörper V mehrere Stunden (häufig mehr als 5 h) in Anspruch. Vielfach erstreckt sich die Gesamtdauer der thermischen Behandlung auf mehr als 10 h. Meist werden im Rahmen der thermischen Behandlung der Formkörper V Behandlungsdauern von 45 h bzw. 25 h nicht überschritten. Oft liegt die Gesamtbehandlungsdauer unterhalb von 20 h. Grundsätzlich kann die thermische Behandlung bei höheren Temperaturen über eine kürzere Behandlungsdauer oder bei nicht zu hohen Temperaturen während einer längeren Behandlungsdauer durchgeführt werden. In einer erfindungsgemäß vorteilhaften Ausführungsform der thermischen Behandlung der Formkörper V werden 465°C nicht überschritten und die Behandlungsdauer im Temperaturfenster $\geq$ 440°C erstreckt sich auf > 10 bis 20 h. In einer anderen erfindungsgemäß vorteilhaften Ausführungsform (die für die erfindungsgemäßen Zwecke bevorzugt wird) der thermischen Behandlung der Formkörper V werden 465°C (nicht jedoch 500°C) überschritten und die Behandlungsdauer im Temperaturfenster von $\geq$ 465°C erstreckt sich auf 2 bis 10 h.

Das heißt, die Endkalzination in allen Ausführungsbeispielen B1 bis B8 sowie in allen Vergleichsbeispielen V1 bis V6 kann auch unter ansonsten unveränderten Bedingungen (einschließlich der nachfolgenden Verwendung als Katalysatoren für die Propenpartialoxidation) bei einer Endtemperatur von 450, oder 452, oder 454, oder 456, oder 458, oder 460, oder 462, oder 464, oder 466, oder 468, oder 470, oder 472, oder 474, oder 476, oder 478, oder 480, oder 485, oder 490, oder 495, oder 500, oder 505 oder 510 °C durchgeführt werden.

Die Endkalzination in allen Ausführungsbeispielen B1 bis B8 sowie in allen Vergleichsbeispielen V1 bis V6 (einschließlich der nachfolgenden Verwendung als Katalysatoren für die Propenpartialoxidation) kann aber auch unter ansonsten unveränderten Bedingungen während einer auf 9, oder 8, oder 7, oder 6, oder 5, oder 4, oder 3, oder 2, oder 1 h verkürzten Endkalzinationsdauer bei einer jeweils um 2, oder 4, oder 6, oder 8, oder 10, oder 12, oder 14, oder 16, oder 20 °C erhöhten Endkalzinationstemperatur durchgeführt werden.

Die thermische Behandlung (auch die Phase 1 (auch Zersetzungsphase genannt)) der Formkörper V kann sowohl unter Inertgas als auch unter einer oxidativen Atmosphäre wie z.B. Luft (oder ein sonstiges Gemisch aus Inertgas und Sauerstoff) sowie unter reduzierender Atmosphäre (z. B. ein Gemisch aus Inertgas, $NH_3$, CO und/oder $H_2$ oder unter Methan, Acrolein, Methacrolein) erfolgen. Selbstredend kann die thermische Behandlung auch unter Vakuum ausgeführt werden. Auch kann die Kalzinationsatmosphäre über die Kalzinationsdauer variabel gestaltet werden. Erfindungsgemäß bevorzugt erfolgt die thermische Behandlung der Formkörper V in einer oxidierenden Atmosphäre. Anwendungstechnisch zweckmäßig besteht sie überwiegend aus stehender oder bewegter Luft.

Prinzipiell kann die thermische Behandlung der Formkörper V in den unterschiedlichsten Ofentypen wie z.B. beheizbare Umluftkammern (Umluftöfen), Hordenöfen, Drehrohröfen, Bandkalzinierern oder Schachtöfen durchgeführt werden. Erfindungsgemäß vorteilhaft erfolgt die thermische Behandlung der Formkörper V in einer Bandkalzinierungsvorrichtung, wie sie die DE-A 10046957 und die WO 02/24620 empfehlen. Eine Heißpunktausbildung innerhalb des Kalzinationsgutes wird dabei weitestgehend dadurch vermieden, dass mit Hilfe von Ventilatoren durch ein das Kalzinationsgut tragendes gasdurchlässiges Förderband erhöhte Volumenströme an Kalzinationsatmosphäre durch das Kalzinationsgut gefördert werden.

Die thermische Behandlung der Formkörper V unterhalb von 350°C verfolgt in der Regel das Ziel der thermischen Zersetzung der in den Formkörpern V enthaltenen Quellen der Elemente (der elementaren Konstituenten) der angestrebten Multielementoxid-I-Aktivmasse der Katalysatorformkörper K sowie von gegebenenfalls mitverwendeten Formgebungshilfsmitteln. Häufig erfolgt diese Zersetzungsphase im Rahmen des Aufheizens des Kalzinationsgutes auf Temperaturen $\geq$ 350°C.

Grundsätzlich kann die thermische Behandlung wie in der US 2005/0131253 beschrieben erfolgen.

In typischer Weise betragen die Seitendruckfestigkeiten von wie beschrieben erfindungsgemäß erhältlichen ringförmigen Vollkatalysatorformkörpern K 5 bis 13 N, häufig 8 bis 11 N.

Grundsätzlich kann die Formung (Verdichtung) der feinteiligen Ausgangsmasse A3 zu einem Formkörper V auch dadurch erfolgen, dass man die feinteilige Ausgangsmasse A3 mit Hilfe eines geeigneten flüssigen Bindemittels auf die Oberfläche eines wie vorstehend beschriebenen geometrischen Trägerformkörpers aufbringt. Nach Trocknung können die dabei resultierenden Vorläuferformkörper V in erfindungsgemäßer Weise unter Erhalt von erfindungsgemäßen Schalenkatalysatorformkörpern K thermisch behandelt werden.

Als günstig erweist es sich für das erfindungsgemäße Herstellverfahren, wenn für die feinteilige Ausgangsmasse A2 auch $10\ \mu m \leq d_{50}^{A2} \leq 50\ \mu m$, vorzugsweise $20\ \mu m \leq d_{50}^{A2} \leq 40\ \mu m$ erfüllt ist.

Ferner ist es für das erfindungsgemäße Herstellverfahren vorteilhaft, wenn, zusätzlich zur erfindungsgemäßen Bedingung für den Betrag F, für den Betrag F* des Produktes (die Partikeldurchmesser $d_{50}^{A1}$ (der feinteiligen Ausgangsmasse A1), $d_{50}^{A2}$ (der feinteiligen Ausgangsmasse A2) sind dabei wieder in der Längeneinheit $\mu m$ angegeben)

$$(d_{50}^{A1})^{0,7} \quad \bullet \quad (d_{50}^{A2})^{0,7} \quad \bullet \quad (a^{-1})$$

die Bedingung F* $\geq$ 15 (vorzugsweises 18), besonders bevorzugt 25 $\geq$ F* $\geq$ 18, erfüllt ist.

Als erfindungsgemäß günstig erweist sich ferner ein Verhältnis des Partikeldurchmessers $d_{90}^{A2}$ der feinteiligen Ausgangsmasse A2 zum Partikeldurchmesser $d_{10}^{A2}$ der feinteiligen Ausgangsmasse A2, das heißt, $d_{90}^{A2} : d_{10}^{A2}$ (angegeben in derselben Längeneinheit) im Bereich von 5 bis 20, vorzugsweise im Bereich von 10 bis 15.

Die erfindungsgemäß erhältlichen Katalysatorformkörper K eignen sich als Katalysatoren für alle heterogen katalysierten partiellen Gasphasenoxidationen, für die in dieser Schrift bereits die geometrischen Katalysatorformkörper K* als geeignet erwähnt worden sind. Besonders geeignet sind erfindungsgemäß erhältliche geometrische Katalysatorformkörper K jedoch als Katalysatoren für die Partialoxidationen von Propen zu Acrolein und von iso-Buten und/oder tert. Butanol zu Methacrolein. Dies gilt insbesondere für erfindungsgemäße ringförmige Vollkatalysatorformkörper K. Die Partialoxidation kann dabei z.B. wie in den Schriften DE-A 102007004961, WO 02/49757,

WO 02/24620, Deutsche Anmeldung Nr. 102008040093.9, WO 2005/030393, EP-A 575 897, WO 2007/082827, WO 2005/113127, WO 2005/047224, WO 2005/042459 und WO 2007/017431 beschrieben durchgeführt werden.

Dabei erweisen sich die in dieser Schrift individualisiert hervorgehobenen Ringgeometrien der wie beschrieben erhältlichen ringförmigen Vollkatalysatoren insbesondere dann als vorteilhaft, wenn die Belastung der Katalysatorbeschickung mit im Reaktionsgasausgangsgemisch enthaltenem Propen, iso-Buten und/oder tert. Butanol (bzw. dessen Methylether) $\geq$ 130 NI/l Katalysatorbeschickung · h beträgt (Vor- und/oder Nachschüttungen aus reinem Inertmaterial werden bei Belastungsbetrachtungen in dieser Schrift nicht als zur Katalysatorbeschickung gehörig betrachtet).

Diese Vorteilhaftigkeit ("geringe Deaktivierungsrate") von wie beschrieben erhältlichen ringförmigen Vollkatalysatorformkörpern K liegt aber auch dann vor, wenn die vorgenannte Belastung der Katalysatorbeschickung $\geq$ 140 Nl/l·h, oder $\geq$ 150 Nl/l·h, oder $\geq$ 160 Nl/l·h beträgt. Im Normalfall wird die vorgenannte Belastung der Katalysatorbeschickung $\leq$ 600 Nl/l·h, häufig $\leq$ 500 Nl/l·h, vielfach $\leq$ 400 Nl/l·h oder $\leq$ 350 Nl/l·h betragen. Belastungen im Bereich von 160 Nl/l·h bis 300 bzw. 250 oder 200 Nl/l·h sind besonders zweckmäßig.

Selbstverständlich können die wie beschrieben erhältlichen ringförmigen Vollkatalysatorformkörper K als Katalysatoren für die Partialoxidation von Propen zu Acrolein bzw. von iso-Buten und/oder tert. Butanol (bzw. dessen Methylether) zu Methacrolein auch bei Belastungen der Katalysatorbeschickung mit der partiell zu oxidierenden Ausgangsverbindung von $\leq$ 130 Nl/l·h, oder $\leq$ 120 Nl/l·h, oder $\leq$ 110 Nl/l·h, in erfindungsgemäß vorteilhafter Weise betrieben werden. In der Regel wird diese Belastung jedoch bei Werten $\geq$ 60 Nl/l·h, oder $\geq$ 70 Nl/l·h, oder $\geq$ 80 Nl/l·h liegen.

Prinzipiell kann die Belastung der Katalysatorbeschickung mit der partiell zu oxidierenden Ausgangsverbindung (Propen, iso-Buten und/oder tert. Butanol (bzw. dessen Methylether)) über zwei Stellschrauben eingestellt werden:

a) die Belastung der Katalysatorbeschickung mit Reaktionsgasausgangsgemisch (das Reaktionsgasgemisch, das dem Katalysatorfestbett zugeführt wird),
und/oder
b) den Gehalt des Reaktionsgasausgangsgemischs mit der partiell zu oxidierenden Ausgangsverbindung.

Die erfindungsgemäß erhältlichen z. B. ringförmigen Vollkatalysatorformkörper K eignen sich insbesondere auch dann, wenn bei oberhalb von 130 Nl/l·h liegenden Belastungen der Katalysatorbeschickung mit der partiell zu oxidierenden organischen Verbindung die Belastungseinstellung vor allem über die vorgenannte Stellschraube a) erfolgt.

Der Propenanteil (iso-Butenanteil bzw. tert. Butanolanteil (bzw. der Methyletheranteil)) im Reaktionsgasausgangsgemisch wird im Regelfall (d.h. im wesentlichen unabhängig von der Belastung) 4 bis 20 Vol.-%, häufig 5 bis 15 Vol.-%, oder 5 bis 12 Vol.-%, oder 5 bis 8 Vol.-% betragen (jeweils bezogen auf das Gesamtvolumen des Reaktionsgasausgangsgemischs).

Häufig wird man das Gasphasenpartialoxidationsverfahren der mit den wie beschrieben erhältlichen z. B. ringförmigen Vollkatalysatorformkörper K (oder sonstigen geometrischen Katalysatorformkörpern K) katalysierten Partialoxidation (im wesentlichen unabhängig von der Belastung) bei einem partiell zu oxidierende (organische) Verbindung (z.B. Propen): Sauerstoff : indifferente Gase (einschließlich Wasserdampf) Volumenverhältnis im Reaktionsgasausgangsgemisch von 1:(1,0 bis 3,0):(5 bis 25), vorzugsweise 1:(1,5 bis 2,3):(10 bis 20), durchführen.

Unter indifferenten Gasen (oder auch Inertgasen) werden dabei solche Gase verstanden, die im Verlauf der Partialoxidation zu wenigstens 95 mol-%, vorzugsweise zu wenigstens 98 mol-% chemisch unverändert erhalten bleiben.

Bei den vorstehend beschriebenen Reaktionsgasausgangsgemischen kann das indifferente Gas zu $\geq$ 20 Vol.-%, oder zu $\geq$ 30 Vol.-%, oder zu $\geq$ 40 Vol.-%, oder zu $\geq$ 50 Vol.-%, oder zu $\geq$ 60 Vol.-%, oder zu $\geq$ 70 Vol.-%, oder zu $\geq$ 80 Vol.-%, oder zu $\geq$ 90 Vol.-%, oder zu $\geq$ 95 Vol.-% aus molekularem Stickstoff bestehen.

Bei Belastungen der Katalysatorbeschickung mit der partiell zu oxidierenden organischen Verbindung von $\geq$ 150 Nl/l·h ist jedoch die Mitverwendung von inerten Verdünnungsgasen wie Propan, Ethan, Methan, Pentan, Butan, $CO_2$, CO, Wasserdampf und/oder Edelgasen für das Rektionsgasausgangsgemisch empfehlenswert. Generell können diese inerten Gase und ihre Gemische aber auch bereits bei geringeren Belastungen der Katalysatorbeschickung mit der partiell zu oxidierenden organischen Verbindung eingesetzt werden. Auch kann Kreisgas als Verdünnungsgas mitverwendet werden. Unter Kreisgas wird das Restgas verstanden, das verbleibt, wenn man aus dem Produktgasgemisch der Partialoxidation die Zielverbindung im Wesentlichen selektiv abtrennt. Dabei ist zu berücksichtigen, dass die Partialoxidationen zu Acrolein oder Methacrolein mit den erfindungsgemäß erhältlichen z. B. ringförmigen Katalysatorformkörpern K nur die erste Stufe einer zweistufigen Partialoxidation zu Acrylsäure oder Methacrylsäure als den eigentlichen Zielverbindungen sein können, so dass die Kreisgasbildung dann meist erst nach der zweiten Stufe erfolgt. Im Rahmen einer solchen zweistufigen Partialoxidation wird in der Regel das Produktgasgemisch der ersten Stufe als solches, gegebenenfalls nach Abkühlung und/oder Sekundärsauerstoffzugabe, der zweiten Partialoxidationsstufe zugeführt.

Bei der Partialoxidation von Propen zu Acrolein, unter Anwendung der wie beschrieben erhältlichen z. B. ringförmigen Katalysatorformkörper K, kann eine typische Zusammensetzung des Reaktionsgasausgangsgemischs gemessen am Reaktoreingang (unabhängig von der gewählten Belastung) z.B. die nachfolgenden Komponenten enthalten:

| | |
|---|---|
| 6 bis 6,5 Vol.-% | Propen, |
| 1 bis 3,5 Vol.-% | $H_2O$, |
| 0,2 bis 0,5 Vol.-% | CO, |
| 0,6 bis 1,2 Vol.-% | $CO_2$, |

(fortgesetzt)

| | |
|---|---|
| 0,015 bis 0,04 Vol.-% | Acrolein, |
| 10,4 bis 11,3 Vol.-% | $O_2$, und |
| als Restmenge ad 100 Vol.-% molekularer Stickstoff; | |

oder:

| | |
|---|---|
| 5,6 Vol.-% | Propen, |
| 10,2 Vol.-% | Sauerstoff, |
| 1,2 Vol.-% | $CO_X$, |
| 81,3 Vol.-% | $N_2$, und |
| 1,4 Vol.-% | $H_2O$. |

Erstere Zusammensetzungen eignen sich insbesondere bei Propenbelastungen von $\geq$ 130 Nl/l·h und letztere Zusammensetzung insbesondere bei Propenbelastungen < 130 Nl/l·h, insbesondere $\leq$ 100 Nl/l·h des Katalysatorfestbetts.

Als alternative Zusammensetzungen des Reaktionsgasausgangsgemischs kommen (unabhängig von der gewählten Belastung) solche in Betracht, die nachfolgende Komponentengehalte aufweisen:

| | |
|---|---|
| 4 bis 25 Vol.-% | Propen, |
| 6 bis 70 Vol.-% | Propan, |
| 5 bis 60 Vol.-% | $H_2O$, |
| 8 bis 65 Vol.-% | $O_2$, und |
| 0,3 bis 20 Vol.-% | $H_2$; |

oder

| | |
|---|---|
| 4 bis 25 Vol.-% | Propen, |
| 6 bis 70 Vol.-% | Propan, |
| 0 bis 60 Vol.-% | $H_2O$, |
| 8 bis 16 Vol.-% | $O_2$, |
| 0 bis 20 Vol.-% | $H_2$, |
| 0 bis 0,5 Vol.-% | CO, |
| 0 bis 1,2 Vol.-% | $CO_2$, |
| 0 bis 0,04 Vol.-% | Acrolein, |
| und als Restmenge ad 100 Vol.-% im wesentlichen $N_2$; | |

oder

| | |
|---|---|
| 50 bis 80 Vol.-% | Propan, |
| 0,1 bis 20 Vol.-% | Propen, |
| 0 bis 10 Vol.-% | $H_2$, |
| 0 bis 20 Vol.-% | $N_2$, |
| 5 bis 15 Vol.-% | $H_2O$, und |
| soviel molekularer Sauerstoff, dass das molare Verhältnis von Sauerstoffgehalt zu Propengehalt 1,5 bis 2,5 beträgt. | |

oder

| | |
|---|---|
| 6 bis 9 Vol.-% | Propen, |
| 8 bis 18 Vol.-% | molekularer Sauerstoff, |
| 6 bis 30 Vol.-% | Propan, und |
| 32 bis 72 Vol.-% | molekularer Stickstoff. |

Das Reaktionsgasausgangsgemisch kann aber auch wie folgt zusammengesetzt sein:

| | |
|---|---|
| 4 bis 15 Vol.-% | Propen, |
| 1,5 bis 30 Vol.-% | (häufig 6 bis 15 Vol.-%) Wasser, |
| $\geq 0$ bis 10 Vol.-% | (vorzugsweise $\geq 0$ bis 5 Vol.-%) von Propen, Wasser, Sauerstoff und Stickstoff verschiedene Bestandteile, soviel molekularer Sauerstoff, dass das molare Verhältnis von enthaltenem molekularem Sauerstoff zu enthaltenem molekularem Propen 1,5 bis 2,5 beträgt, und als Restmenge bis zur 100 Vol.-% Gesamtmenge molekularer Stickstoff. |

Eine andere mögliche Reaktionsgasausgangsgemischzusammensetzung kann enthalten:

| | |
|---|---|
| 6,0 Vol.-% | Propen, |
| 60 Vol.-% | Luft, und |
| 34 Vol.-% | $H_2O$. |

Alternativ können auch Reaktionsgasausgangsgemische der Zusammensetzung gemäß Example 1 der EP-A 990 636, oder gemäß Example 2 der EP-A 990 636, oder gemäß Example 3 der EP-A 1 106 598, oder gemäß Example 26 der EP-A 1 106 598, oder gemäß Example 53 der EP-A 1 106 598 verwendet werden.

Auch eignen sich die wie beschrieben erhältlichen z. B. ringförmigen Katalysatorformkörper K für die Verfahren der DE-A 10246119 bzw. DE-A 10245585.

Weitere erfindungsgemäß geeignete Reaktionsgasausgangsgemische können im nachfolgenden Zusammensetzungsraster liegen:

| | |
|---|---|
| 7 bis 11 Vol.-% | Propen, |
| 6 bis 12 Vol.-% | Wasser, |
| $\geq 0$ bis 5 Vol.-% | von Propen, Wasser, Sauerstoff und Stickstoff verschiedener Bestandteile, |

soviel molekularer Sauerstoff, dass das molare Verhältnis von enthaltenem molekularem Sauerstoff zu enthaltenem Propen 1,6 bis 2,2 beträgt, und

als Restmenge bis zur 100 Vol.-% Gesamtmenge molekularer Stickstoff.

Im Fall von Methacrolein als Zielverbindung kann das Reaktionsgasausgangsgemisch insbesondere auch wie in der DE-A 44 07 020 beschrieben zusammengesetzt sein.

Die Reaktionstemperatur für die Propenpartialoxidation liegt bei Verwendung der wie beschrieben erhältlichen z. B. ringförmigen Katalysatorformkörper K häufig bei 300 bis 380°C. Das gleiche trifft im Fall von Methacrolein als Zielverbindung zu.

Der Reaktionsdruck liegt für die vorgenannten Partialoxidationen in der Regel bei 0,5 bzw. 1,5 bis 3 bzw. 4 bar (gemeint sind in dieser Schrift, soweit nicht ausdrücklich anders erwähnt ist, stets Absolutdrucke).

Die Gesamtbelastung der Katalysatorbeschickung mit Reaktionsgasausgangsgemisch beläuft sich bei den vorgenannten Partialoxidationen typisch auf 1000 bis 10000 Nl/l·h, meist auf 1500 bis 5000 Nl/l·h und oft auf 2000 bis 4000 Nl/l·h.

Als im Reaktionsgasausgangsgemisch zu verwendendes Propen kommen vor allem polymer grade Propen und chemical grade Propen in Betracht, wie es z. B. die DE-A 102 32 748 beschreibt.

Als Sauerstoffquelle wird normalerweise Luft eingesetzt.

Die Partialoxidation in Anwendung der wie beschrieben erhältlichen z. B. ringförmigen Katalysatorformkörper K kann im einfachsten Fall z. B. in einem Einzonen-Vielkontaktrohr-Festbettreaktor durchgeführt werden, wie ihn die DE-A 44 31 957, die EP-A 700 714 und die EP-A 700 893 beschreiben.

Üblicherweise sind in den vorgenannten Rohrbündelreaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Eine typische Kontaktrohrlänge beläuft sich z. B. auf 3,20 m. Anwendungstechnisch zweckmäßig beläuft sich die im Rohrbündelbehälter untergebrachte Anzahl an Kontaktrohren auf wenigstens 1000, vorzugsweise auf wenigstens 5000. Häufig beträgt die Anzahl der im Reaktionsbehälter untergebrachten Kontaktrohre 15000 bis 35000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. EP-B 468 290).

Die Partialoxidation kann aber auch in einem Mehrzonen (z. B. "Zwei-Zonen")-Vielkontaktrohr-Festbettreaktor durchgeführt werden, wie es die DE-A 199 10 506, die DE-A 103 13 213, die DE-A 103 13 208 und die EP-A 1 106 598 insbesondere bei erhöhten Belastungen der Katalysatorbeschickung mit der partiell zu oxidierenden organischen Verbindung empfehlen. Eine typische Kontaktrohrlänge im Fall eines Zweizonen-Vielkontaktrohr-Festbettreaktors beträgt 3,50 m. Alles andere gilt im Wesentlichen wie beim Einzonen-Vielkontaktrohr-Festbettreaktor beschrieben. Um die Kontaktrohre, innerhalb derer sich die Katalysatorbeschickung befindet, wird in jeder Temperierzone ein Wärmeaustauschmittel geführt. Als solche eignen sich z. B. Schmelzen von Salzen wie Kaliumnitrat, Kaliumnitrit, Natriumnitrit und/oder Natriumnitrat, oder von niedriger schmelzenden Metallen wie Natrium, Quecksilber sowie Legierungen verschiedener Metalle. Die Fließgeschwindigkeit des Wärmeaustauschmittels innerhalb der jeweiligen Temperierzone wird in der Regel so gewählt, dass die Temperatur des Wärmeaustauschmittels von der Eintrittsstelle in die Temperaturzone bis zur Austrittstelle aus der Temperaturzone um 0 bis 15 °C, häufig 1 bis 10 °C, oder 2 bis 8 °C, oder 3 bis 6 °C ansteigt.

Die Eingangstemperatur des Wärmeaustauschmittels, das, über die jeweilige Temperierzone betrachtet, im Gleichstrom oder im Gegenstrom zum Reaktionsgasgemisch geführt werden kann, wird vorzugsweise wie in den Schriften EP-A 1 106 598, DE-A 199 48 523, DE-A 199 48 248, DE-A 103 13 209, EP-A 700 714, DE-A 103 13 208, DE-A 103 13 213, WO 00/53557, WO 00/53558, WO 01/36364, WO 00/53557 sowie den anderen in dieser Schrift als Stand der Technik zitierten Schriften empfohlen gewählt. Innerhalb der Temperierzone wird das Wärmeaustauschmittel bevorzugt mäanderförmig geführt. In der Regel verfügt der Vielkontaktrohr-Festbettreaktor zusätzlich über Thermorohre zur Bestimmung der Gastemperatur im Katalysatorbett. In zweckmäßiger Weise wird der Innendurchmesser der Thermorohre und der Durchmesser der innenliegenden Aufnahmehülse für das Thermoelement so gewählt, dass das Verhältnis von Reaktionswärme entwickelndem Volumen zu Wärme abführender Oberfläche bei Thermorohren und Arbeitsrohren gleich oder nur geringfügig unterschiedlich ist.

Der Druckverlust sollte bei Arbeitsrohren und Thermorohren, bezogen auf gleiche GHSV, gleich sein. Ein Druckverlustausgleich beim Thermorohr kann durch Zusatz von versplittetem Katalysator zu den Katalysatorformkörpern erfolgen. Dieser Ausgleich erfolgt zweckmäßig über die gesamte Thermorohrlänge homogen.

Zur Bereitung der Katalysatorbeschickung in den Kontaktrohren können, wie bereits erwähnt, nur wie beschrieben erhältliche z. B. ringförmige Katalysatorformkörper K oder z. B. auch weitgehend homogene Gemische aus wie beschrieben erhältlichen z. B. ringförmigen Katalysatorformkörpern K und keine Aktivmasse aufweisenden, sich bezüglich der heterogen katalysierten partiellen Gasphasenoxidation im wesentlichen inert verhaltenden Formkörpern verwendet werden. Als Materialien für solche inerten Formkörper kommen z. B. poröse oder unporöse Aluminiumoxide, Siliciumdioxid, Zirkondioxid, Siliciumcarbid, Silikate wie Magnesium- oder Aluminiumsilikat und/oder Steatit (z.B. vom Typ C220 der Fa. CeramTec, DE) in Betracht.

Die Geometrie solcher inerter Verdünnungsformkörper kann im Prinzip beliebig sein. D.h., es können beispielsweise Kugeln, Polygone, Vollzylinder oder auch, wie z. B. im Fall ringförmiger Katalysatorformkörper K, Ringe sein. Häufig wird man als inerte Verdünnungsformkörper solche wählen, deren Geometrie derjenigen der mit ihnen zu verdünnenden Katalysatorformkörper K entspricht. Längs der Katalysatorbeschickung können aber auch die Geometrie des Katalysatorformkörpers K gewechselt oder Katalysatorformkörper K unterschiedlicher Geometrie in weitgehend homogener Abmischung eingesetzt werden. In einer weniger bevorzugten Vorgehensweise kann auch die Aktivmasse des Katalysatorformkörpers K längs der Katalysatorbeschickung verändert werden.

Ganz generell wird, wie bereits erwähnt, die Katalysatorbeschickung mit Vorteil so gestaltet, dass die volumenspezifische (d. h., die auf die Einheit des Volumens normierte) Aktivität in Strömungsrichtung des Reaktionsgasgemischs entweder konstant bleibt oder zunimmt (kontinuierlich, sprunghaft oder stufenförmig).

Eine Verringerung der volumenspezifischen Aktivität kann in einfacher Weise z. B. dadurch erzielt werden, dass man eine Grundmenge von erfindungsgemäß einheitlich hergestellten z. B. ringförmigen Katalysatorformkörpern K mit inerten Verdünnungsformkörpern homogen verdünnt. Je höher der Anteil der Verdünnungsformkörper gewählt wird, desto geringer ist die in einem bestimmten Volumen der Beschickung enthaltene Aktivmasse bzw. Katalysatoraktivität. Eine Verringerung kann aber auch dadurch erzielt werden, dass man die Geometrie der erfindungsgemäß erhältlichen Katalysatorformkörper K so verändert, dass die in der Einheit des Reaktionsrohrinnenvolumens enthaltene Aktivmassenmenge kleiner wird.

Für die heterogen katalysierten Gasphasenpartialoxidationen mit wie beschrieben erhältlichen ringförmigen Vollkatalysatorformkörpern K wird die Katalysatorbeschickung vorzugsweise entweder auf der gesamten Länge einheitlich mit nur einem Typ Vollkatalysatorringformkörper K gestaltet oder wie folgt strukturiert. Am Reaktoreingang wird auf einer Länge von 10 bis 60 %, bevorzugt 10 bis 50 %, besonders bevorzugt 20 bis 40 % und ganz besonders bevorzugt 25 bis 35 % (d. h., z. B. auf einer Länge von 0,70 bis 1,50 m, bevorzugt 0,90 bis 1,20 m), jeweils der Gesamtlänge der Katalysatorbeschickung, ein im Wesentlichen homogenes Gemisch aus erfindungsgemäß erhältlichem ringförmigem Vollkatalysatorformkörper K und inertem Verdünnungsformkörper (wobei beide vorzugsweise im Wesentlichen die gleiche Geometrie aufweisen) platziert, wobei der Gewichtsanteil der Verdünnungsformkörper (die Massendichten von Katalysatorformkörpern K und von Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diesen ersten Beschickungsabschnitt befindet sich dann vorteilhaft bis zum Ende der Länge der Katalysatorbeschickung (d. h., z. B. auf einer Länge von 1,00 bis 3,00 m oder von 1,00 bis 2,70 m, bevorzugt 1,40 bis 3,00 m, oder 2,00 bis 3,00 m) entweder eine nur in geringerem Umfang (als im ersten Abschnitt) verdünnte Schüttung des wie beschrieben erhältlichen ringförmigen Vollkatalysatorformkörpers K, oder, ganz besonders bevorzugt, eine alleinige (unverdünnte) Schüttung desselben ringförmigen Vollkatalysatorformkörper K, der auch im ersten Abschnitt verwendet worden ist. Natürlich kann über die gesamte Beschickung auch eine konstante Verdünnung gewählt werden. Auch kann im ersten Abschnitt nur mit einem erfindungsgemäß erhältlichen ringförmigen Vollkatalysatorformkörper K von geringer, auf seinen Raumbedarf bezogener, Aktivmassendichte und im zweiten Abschnitt mit einem erfindungsgemäß erhältlichen ringförmigen Vollkatalysatorformkörper K mit hoher, auf seinen Raumbedarf bezogener, Aktivmassendichte beschickt werden (z.B. 6,5 mm x 3 mm x 4,5 mm [A x H x I] im ersten Abschnitt, und 5 x 2 x 2 mm im zweiten Abschnitt).

Insgesamt werden bei einer mit den wie beschrieben erhältlichen z. B. ringförmigen Katalysatorformkörpern K als Katalysatoren durchgeführten Partialoxidation zur Herstellung von Acrolein oder Methacrolein die Katalysatorbeschickung, das Reaktionsgasausgangsgemisch, die Belastung und die Reaktionstemperatur in der Regel so gewählt, dass beim einmaligen Durchgang des Reaktionsgasgemischs durch die Katalysatorbeschickung ein Umsatz der partiell zu oxidierenden organischen Verbindung (Propen, iso-Buten, tert.-Butanol bzw. dessen Methylether) von wenigstens 90 mol-%, oder wenigstens 92 mol-%, vorzugsweise von wenigstens 94 mol-% resultiert. Die Selektivität der Acrolein- bzw. Methacroleinbildung wird dabei regelmäßig ≥ 80 mol-%, bzw. ≥ 85 mol-% betragen. In natürlicher Weise werden dabei möglichst geringe Heißpunkttemperaturen angestrebt.

Abschließend sei festgehalten, dass wie beschrieben erhältliche ringförmige Vollkatalysatorformkörper K auch ein vorteilhaftes Bruchverhalten bei der Reaktorbefüllung aufweisen.

Die Inbetriebnahme einer(s) frischen, erfindungsgemäß erhältliche geometrische Katalysatorformkörper K enthaltenden, Katalysatorbeschickung (Katalysatorfestbetts) kann z. B. wie in der DE-A 103 37 788 beschrieben erfolgen.

In der Regel nehmen Aktivität und Selektivität der Zielproduktbildung anfänglich mit der Betriebsdauer der Katalysatorbeschickung zu, bevor deren alterungsbedingte Minderung eintritt. Diese Formierung kann dadurch beschleunigt werden, dass man sie bei im Wesentlichen gleich bleibendem Umsatz unter erhöhter Belastung der Katalysatorbeschickung mit Reaktionsgasausgangsgemisch durchführt und nach weitgehend vollzogener Formierung die Belastung auf ihren Zielwert zurücknimmt.

Im Übrigen sind erfindungsgemäß erhältliche geometrische Katalysatorformkörper K ganz generell als Katalysatoren mit geringer Deaktivierungsrate für gasphasenkatalytische Partialoxidationen 3 bis 6 (d. h., 3, 4, 5 oder 6) C-Atome enthaltender Alkane (insbesondere Propan), Alkanole, Alkanale, Alkene und Alkenale zu z. B. olefinisch ungesättigten Aldehyden und/oder Carbonsäuren, sowie den entsprechenden Nitrilen (Ammoxidation, vor allem von Propen zu Acrylnitril und von 2-Methylpropen bzw. tert.-Butanol (bzw. dessen Methylether) zu Methacrylnitril) sowie für gasphasenkatalytische oxidative Dehydrierungen der vorgenannten 3, 4, 5 oder 6 C-Atome enthaltenden organischen Verbindungen geeignet.

Wird eine ausgeprägt exotherme heterogen katalysierte partielle Gasphasenoxidation an einem in einem Reaktionsrohr befindlichen Katalysatorfestbett durchgeführt, das von außen mit Hilfe eines fluiden Wärmeträgers (z. B. eine Salzschmelze) gekühlt wird, so läuft die Temperatur des Reaktionsgasgemischs (und damit auch des Katalysatorfestbetts) in Strömungsrichtung des Reaktionsgases, im wesentlichen unabhängig von der konkreten Strömungsführung des Wärmeträgers relativ zur Flußrichtung des Reaktionsgasgemischs, häufig durch ein Temperaturmaximum, das als sogenannte Heißpunkttemperatur bezeichnet wird (vgl. z. B. Ullmann's Encyclopedia of Industrial Chemistry, VCH, 5. Ed., Volume B4, 1992, Seite 221).

Um die Ausprägung der Heißpunkttemperatur zu minimieren, wird in vielen Fällen die Aktivitätsstrukturierung des im Reaktionsrohr befindlichen Katalysatorfestbetts so gestaltet, dass sich in Strömungsrichtung des Reaktionsgasgemischs und auf die Gesamtlänge L des Katalysatorfestbetts bezogen (reine Inertmaterialabschnitte werden bei dieser Betrachtung als nicht zum Katalysatorfestbett zugehörig betrachtet) der Heißpunktbereich etwa im ersten Viertel der Gesamtlänge L befindet, weshalb dieser Längenbereich (Längenabschnitt) des Katalysatorfestbetts in dieser Schrift als "Heißpunktzone" bezeichnet werden soll, während der gesamte restliche Längenbereich des Katalysatorfestbetts in Strömungsrichtung des Reaktionsgasgemischs (3/4 • L) als "Hauptzone" bezeichnet wird. In der Regel schreitet die Desaktivierung von in der Heißpunktzone befindlichen geometrischen Katalysatorformkörpern K rascher voran als diejenige von geometrischen Katalysatorformkörpern K der gleichen Art, die sich in der Hauptzone befinden. Anstelle eines Rohrbündelreaktors kann zur Aufnahme des Katalysatorfestbetts aber auch ein Thermoplattenreaktor verwendet werden (vgl. z. B. DE-A 103 61 456).

Im Übrigen lässt die Verknüpfung von bereits veröffentlichten Forschungsergebnissen (z. B. im bereits zitierten Stand der Technik sowie in den folgenden wissenschaftlichen Veröffentlichungen: O. V. Udalova, D. P. Shashkin, M. D. Shibanova, O. V. Krylov, Kinetics and Catalysis 46 (2005), S. 535-544; D. P. Shashkin, O. V. Udalova, M. D. Shibanova, O. V. Krylov, Kinetics and Catalysis 46 (2005), S. 545-549; Y. Moro-Oka, W. Ueda, Adv. Catal. 40 (1994), S. 233 - 273; M. W. J. Wolfs, P. A. Batist, J. Catal. 32 (1974) S. 25 - 36; D.-H. He, W. Ueda, Y. Moro-Oka, Catal. Lett. 12 (1992), S. 35 - 44; Y. Haykawa, T. Tsunoda, H. Orita, T. Kameyama, H. Takahashi, K. Fukuda, K. Takehira, J. Chem. Soc. Chem. Commun (1987), S. 780 - 782; M. T. Le, W. J. M. v. Well, P. Stoltze, I. v. Driessche, S. Haste, Appl. Catal. A. Gen. 282 (2005), S. 189 - 194; W. J. M. v. Well, M. T. Le, N.C. Schiedt, S. Hoste, P. Stolzte, J. Mol. Catal. A. Chemical 256 (2006), S. 1 - 8 und J. M. M. Millet, G. Coudurier, J. M. Herrmann, J. C. Védrine, J. Catal. 142 (1993), S. 381 - 391) mit den Ergebnissen der in dieser Schrift ausgeführten Beispiele und Vergleichsbeispiele sowie Ergebnissen von über die in dieser Anmeldung ausgeführten Beispiele und Vergleichsbeispiele hinausgehenden experimentellen Untersuchungen der Anmelderin nachfolgende Feststellungen zu:

1. Untersuchungen von geometrischen Katalysatorformkörpern K* mittels Rasterelektronenmikroskopie (SEM) in Kombination mit energiedispersiver Röntgenanalyse (EDX) nach Beendigung der zu ihrem Erhalt angewandten thermischen Behandlung von Formkörpern V weisen aus, dass die Partikel der jeweils vorgebildeten feinteiligen Ausgangsmasse A1 in den resultierenden Katalysatorformkörpern K* scheinbar unverändert vorliegen.

2. Untersuchungen des Röntgendiffraktogramms von geometrischen Katalysatorformkörpern K* sowie von in identischer Weise, jedoch ohne Mitverwendung von feinteiliger Ausgangsmasse A1, hergestellten Vergleichsformkörpern K*$^V$ weisen aus, dass sowohl der Anteil T in den Aktivmassen der Katalysatorformkörper K* als auch die in den Vergleichsformkörpern K*$^V$ ausgebildeten Multielementoxidmassen im wesentlichen aus kristallinen Phasen bestehen, wobei sowohl hinsichtlich der Art dieser kristallinen Phasen als auch hinsichtlich ihrer Mengenverteilung (Gew.-%, bezogen auf das jeweilige Gesamtgewicht) kaum ein Unterschied besteht. Die Körnung der Ausgangsmassen A1, A2 beeinflusst vorstehende Kristallphasenausbildung allenfalls unwesentlich.

3. Bei Verwendung von erfindungsgemäß hergestellten geometrischen Katalysatorformkörpern K und von nicht erfindungsgemäß hergestellten geometrischen Katalysatorformkörpern K*, deren Herstellung sich bei im wesentlichen identischer Stöchiometrie des jeweiligen Anteils T sowie im wesentlichen identischer Stöchiometrie der jeweiligen Ausgangsmasse A1 und nur geringfügig voneinander abweichenden stöchiometrischen Koeffizienten a vor allem durch voneinander verschiedene Werte der $d_{50}^{A1}$ Partikeldurchmesser und $d_{90}^{A2}$ unterschied, als Katalysatoren für eine heterogen katalysierte partielle Gasphasenoxidation von Propen zu Acrolein, zeigen im Langzeitbetrieb beide, abgesehen von einem jeweils wahrnehmbaren Verlust an Mo, eine im wesentlichen unveränderte Bruttostöchiometrie der jeweiligen Multielementoxidaktivmasse I, bei vergleichbarer Anfangsaktivität jedoch voneinander signifikant verschiedene Desaktivierungsraten. Eingehendere Untersuchungen weisen aus, dass die bis zum gleichen Desaktivierungsgrad erlittenen jeweiligen Mo-Verluste sich deutlich voneinander unterscheiden, jedoch weitgehend mit der bis dahin jeweils erforderlichen Betriebsdauer der Partialoxidation korrelieren. Das auch in der Fachliteratur vielfach berichtete Ausbluten an Mo kann somit nicht den dominanten Desaktivierungsmechanismus bilden.

4. Bei isolierter Herstellung der in den Multielementoxid-I-Aktivmassen von ungebrauchten geometrischen Katalysatorformkörpern K und K* identifizierten kristallinen Phasen und jeweiliger isolierter Verwendung derselben als Aktivmasse für die heterogen katalysierte partielle Gasphasenoxidation von Propen zu Acrolein liegen die resultierenden Aktivitäten sowie Selektivitäten der Acroleinbildung weit unterhalb von jenen der geometrischen Katalysatorformkörper K bzw. K*. Das qualitativ gleiche Ergebnis stellt sich bei einer Verwendung von Vergleichsformkörpern K*$^V$ (vgl. "2") als Katalysatoren für die heterogen katalysierte partielle Gasphasenoxidation von Propen zu Acrolein ein.

5. Im Langzeitbetrieb einer mit geometrischen Katalysatorformkörpern K* bzw. K katalysierten partiellen Gasphasenoxidation von Propen zu Acrolein bildet sich in den Multielementoxid-I-Aktivmassen der Katalysatorformkörper über die Zeit eine neue kristalline Phase aus, die in frisch hergestellten, unbenutzten Katalysatorformkörpern K* bzw. K nicht nachweisbar ist und die die Stöchiometrie $Bi_2Mo_3O_{12}$ aufweist.

6. Die Desaktivierungsrate eines gemäß "5." im Langzeitbetrieb befindlichen Katalysatorfestbetts korreliert mit der Bildungsrate an kristallinem $Bi_2Mo_3O_{12}$.

7. Bei isolierter Herstellung von kristallinem $Bi_2Mo_3O_{12}$ und Verwendung desselben als Aktivmasse für die heterogen katalysierte partielle Gasphasenoxidation von Propen zu Acrolein, liegen die resultierenden Aktivitäten weit unterhalb von jenen der geometrischen Katalysatorformkörper K bzw. K*. Die resultierenden Selektivitäten der Acroleinbildung sind jedoch ähnlich zu jenen, die sich bei Verwendung von geometrischen Katalysatorformkörpern K bzw. K* einstellen.

Zusammenfassend lassen vorstehende Feststellungen die folgende Auslegung zu. Die eigentliche katalytisch aktive, die partiell zu oxidierende organische Substanz (z. B. das Propen) aktivierende Masse bildet eine sich im Rahmen der Herstellung von geometrischen Katalysatorformkörpern K* bzw. K durch Migrationsprozesse auf der inneren Oberfläche des Anteils T ausbildende dünne Schicht eines amorphen Wismutmolybdats. Die Sauerstoffversorgung dieser Schicht erfolgt aus dem Anteil T heraus, der den in der Gasphase enthaltenen molekularen Sauerstoff zu aktivieren vermag. Der Anteil $Bi_1W_bO_x$ fungiert als Bi-Depot und der Anteil T als Mo-Depot. Auf der Grundlage der im Verlauf der Partialoxidation anhaltenden Migrationsprozesse wird fortwährend katalytisch aktives amorphes Wismutmolybdat nachgebildet, das sich seinerseits aber gleichzeitig kontinuierlich in im Wesentlichen inaktives kristallines $Bi_2Mo_3O_{12}$ wandelt. Mit zunehmender bereits gebildeter Gesamtmenge an kristallinem $Bi_2Mo_3O_{12}$ erschöpft die Nachbildung des katalytisch aktiven amorphen Wismutmolybdats und/oder die Versorgung desselben mit aktivem Sauerstoff zusehends. Dieser Zusammenhang dominiert die Desaktivierung von geometrischen Katalysatorformkörpern K* bzw. K. Die dem Erscheinungsbild der Desaktivierung unterliegenden Migrations- und Phasenumwandlungsvorgänge werden sowohl durch den stöchiometrischen Koeffizienten a (durch das molare Verhältnis Bi : Mo) als auch durch die Partikeldurchmesser $d_{50}^{A1}$ und $d_{90}^{A2}$ gelenkt. Gröbere Partikel in der Ausgangsmasse A2 dämpfen den Erschöpfungsprozess. Kleine Partikel in der Ausgangsmasse A2 begünstigen die Anfangsselektivität der Zielproduktbildung. Hohe Werte für a fördern die Bildung von kristallinem Wismutmolybdat.

Die großtechnische Herstellung von erfindungsgemäßen Katalysatorformkörpern K (sowie aller Ausführungsbeispiele B1 bis B8 und Vergleichsbeispiele V1 bis V6) erfolgt anwendungstechnisch zweckmäßig wie in den Deutschen Anmeldungen Nr. 102008040093.9 und 102008040094.7 beschrieben (besonders vorteilhaft gemäß Beispiel 1.3. der Anmeldung Nr. 102008040094.7).

Beispiele und Vergleichsbeispiele

l) Herstellung von erfindungsgemäßen ringförmigen Vollkatalysatorformkörpern B1 bis B8 sowie von ringförmigen Vergleichsvollkatalysatorformkörpern V1 bis V6 mit der nachfolgenden Stöchiometrie der Aktivmasse:

$[Bi_1W_2O_{7,5}]_a[Mo_{12}Co_{5,5}Fe_{3,0}Si_{1,6}K_{0,08}O_x]_1$.

a) Herstellung von feinteiligen Ausgangsmassen A1-1 bis A1-5 ($Bi_1W_2O_{7,5}$ = 1/2 $Bi_2W_2O_9$ • $1WO_3$)

In einem 1,75-m³-Doppelmantelbehälter (der Zwischenraum wurde von Temperierwasser durchströmt) aus Edelstahl mit Balkenrührer wurden in 780 kg einer 25 °C aufweisenden wässrigen salpetersauren Wismutnitratlösung (11,2 Gew.-% Bi; freie Salpetersäure: 3 bis 5 Gew.-%; hergestellt mit Salpetersäure aus Wismutmetall der Firma Sidech S.A., 1495 Tilly, Belgien, Reinheit: > 99,997 Gew.-% Bi, < 7 mg/kg Pb, je < 5 mg/kg Ni, Ag, Fe, je < 3 mg/kg Cu, Sb und je < 1 mg/kg Cd, Zn) bei 25 °C innerhalb von 20 min portionsweise 214,7 kg 25 °C aufweisende Wolframsäure (74,1 Gew.-% W, mittlere Korngröße (laut Hersteller bestimmt gemäß ASTM B330) 0,4 bis 0,8 $\mu$m, Glühverlust (2 h bei 750 °C an Luft) 6 - 8 Gew.-%, Schüttgewicht 5 - 8 g/inch³, H.C. Starck, D-38615 Goslar) eingerührt (70 U/min). Das resultierende wässrige Gemisch wurde anschließend noch 3 h bei 25 °C gerührt und dann sprühgetrocknet. Die Sprühtrocknung erfolgte in einem Drehscheibensprühturm vom Typ FS 15 der Firma Niro im Heißluftgleichstrom bei einer Gaseintrittstemperatur von 300 ± 10 °C, einer Gasaustrittstemperatur von 100 ± 10°C, einer Scheibendrehzahl von 18000 U/min, einem Durchsatz von 200 1/h und einer Luftmenge von 1800 Nm³/h. Das resultierende Sprühpulver wies einen Glühverlust von 12,8 Gew.-% (3 h bei 600 °C im Porzellantiegel (der bei

900 °C bis zur Gewichtskonstanz geglüht worden war) unter Luft glühen) und (bei einem Dispergierdruck von 1,1 bar absolut) einen $d_{50}$ von 28,0 $\mu$m ($d_{10}$ = 9,1 $\mu$m, $d_{90}$ = 55,2 $\mu$m) auf. Die nachfolgende Tabelle 1 gibt einen Überblick über repräsentative $d_x$-Werte des Sprühpulvers in Abhängigkeit vom angewandten absoluten Dispergierdruck:

Tabelle 1

|  | 2 bar | 1,5 bar | 1,2 bar | 1,1 bar |
|---|---|---|---|---|
| $d_{10}$ ($\mu$m) | 0,91 | 1,17 | 3,4 | 9,1 |
| $d_{50}$ ($\mu$m) | 5,8 | 8,5 | 19,7 | 28,0 |
| $d_{90}$ ($\mu$m) | 27,5 | 34,3 | 47,2 | 55,2 |

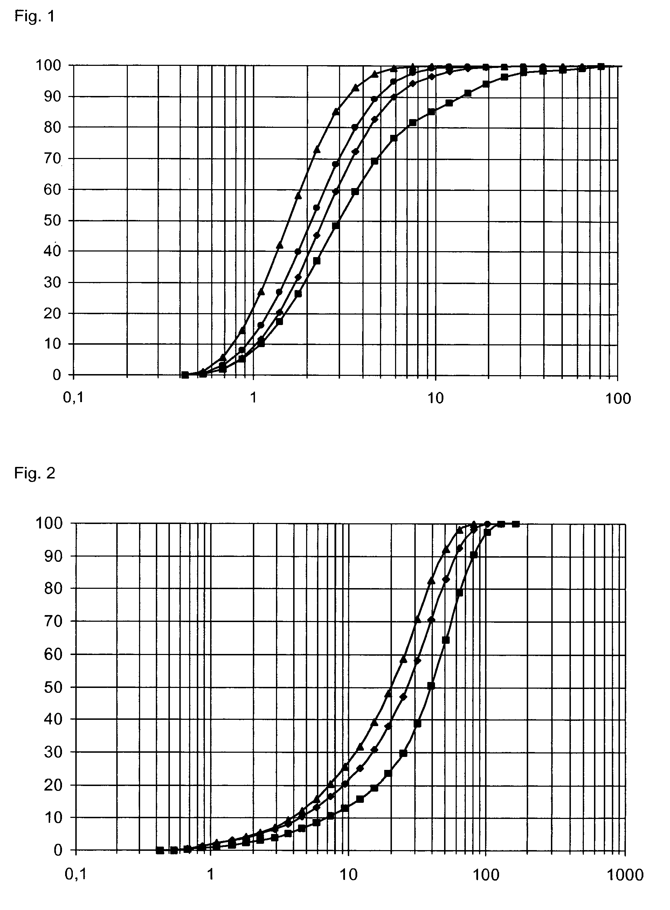

Das erhaltene Sprühpulver wurde anschließend mit 16,7 Gew.-% (bezogen auf das Gewicht des Sprühpulvers) an 25 °C aufweisendem Wasser in einem Auspresskneter für 30 min angeteigt und mittels eines Extruders zu Strängen des Durchmessers 6 mm extrudiert. Diese wurden in Abschnitte von 6 cm geschnitten, auf einem 3-zonigen Bandtrockner bei einer Verweilzeit von 120 min je Zone bei Temperaturen von 90-95°C (Zone 1), 115 °C (Zone 2) und 125 °C (Zone 3) an Luft getrocknet und dann bei einer Temperatur im Bereich um 830 °C thermisch behandelt (kalziniert; im luftdurchströmten Drehrohrofen (0,3 mbar Unterdruck, 200 Nm$^3$/h Luft, 50 kg/h Extrudat, Drehzahl: 1 U/min)). Wesentlich bei der genauen Einstellung der Kalzinationstemperatur ist, dass sie an der angestrebten Phasenzusammensetzung des Kalzinationsprodukts orientiert zu erfolgen hat, das kalzinierte Material aber andererseits eine BET-Oberfläche $\geq 0{,}2$ m$^2$/g aufweist. Gewünscht sind die Phasen WO$_3$ (monoklin) und Bi$_2$W$_2$O$_9$ (orthorhombisch), unerwünscht ist hier das Vorhandensein von $\gamma$-Bi$_2$WO$_6$ (Russellit). Sollte daher nach der Kalzination der Gehalt der Verbindung $\gamma$-Bi$_2$WO$_6$ mehr als 5 Intensitäts-% betragen (berechnet als Verhältnis (Int.-V) der Intensität des Beugungsreflexes von $\gamma$-Bi$_2$WO$_6$ im Röntgenpulverdiffraktogramm bei $2\Theta = 28{,}4$ ° (CuK$\alpha$-Strahlung) zur Intensität des Reflexes von Bi$_2$W$_2$O$_9$ bei $2\Theta = 30{,}0$°), so ist die Präparation zu wiederholen und die Kalzinationstemperatur oder die Verweilzeit bei gleichbleibender Kalzinationstemperatur zu erhöhen, bis der vorgenannte Grenzwert erreicht oder unterschritten wird. Das so erhaltene vorgebildete kalzinierte Mischoxid wurde mit einer Biplex-Querstromsichtmühle Typ 500 BQ der Firma Hosokawa Alpine AG, Augsburg mit 2500 U/min mit unterschiedlichem Durchsatz gemahlen, so dass sich für die Ausgangsmassen A1-1 bis A1-5 als Kenngrößen der Partikelgrößenverteilung (bei einem Dispergierdruck von 2,0 bar absolut gemessen), als BET-Oberfläche (BET) und als $\gamma$-Bi$_2$WO$_6$-Gehalt (Int.-V.) die in Tabelle 2 genannten Werte ergaben. Fig. 1 gibt die bei einem Dispergierdruck von 2,0 bar absolut gemessenen Partikelgrößenverteilungen von A1-1 bis A1-4 zusätzlich im Detail wieder (die Abszisse zeigt die Partikeldurchmesser $d_X^{A1}$ ($\mu$m) im logarithmischen Maßstab, die Ordinate zeigt den Volumenanteil X der jeweiligen feinteiligen Ausgangsmasse A1 (A1-1($\blacktriangle$); A1-2 ($\bullet$); A1-3 ($\blacklozenge$) und A1-4 ($\blacksquare$)), der den jeweiligen Durchmesser oder einen kleineren Durchmesser aufweist (Vol.-.%)). Tabelle 3 zeigt für die Ausgangsmasse A1-2 exemplarisch den Einfluss des Dispergierdrucks auf die Partikelgrößenverteilung einer Ausgangsmasse A1. Bei der Ausgangsmasse A1-1 wurde im Anschluss an die Mahlung in der Querstromsichtmühle noch in einer Spiralstrahlmühle nachgemahlen, bei der Ausgangsmasse A1-5 erfolgte ausschließlich eine milde Mahlung in einer Pralltellermühle. Vor der unter c) beschriebenen Weiterverarbeitung wurden die verschiedenen feinteiligen Ausgangsmassen A1-1 bis A1-5 in Portionen von jeweils 20 kg in einem Schräglagen-Mischer mit Misch- und Schneidflügel (Drehzahl Mischflügel: 60 U/min, Drehzahl Schneidflügel: 3000 U/min) innerhalb von 5 min homogen mit 0,5 Gew.-% (bezogen auf die jeweilige feinteilige Ausgangsmasse A1) feinteiligem hydrophobisiertem SiO$_2$ der Fa. Degussa vom Typ Sipernat® D17 (Rüttelgewicht 150 g/l; d$_{50}$-Wert der SiO$_2$-Partikel (Laserbeugung nach ISO 13320-1) = 10 $\mu$m, die spezifische Oberfläche (Stickstoffadsorption nach ISO 5794-1, Annex D) = 100 m$^2$/g) vermischt.

Tabelle 2

| Ausg.- masse 1 | Mahlung | $d_{10}^{A1}$ / μm | $d_{50}^{A1}$ / μm | $d_{90}^{A1}$ / μm | BET / m$^2$·g$^{-1}$ | Int.-V. / Int.-% |
|---|---|---|---|---|---|---|
| A1-1 | zusätzlich Spiralstrahlmühle | 0,78 | 1,60 | 3,3 | 1,9 | 4 |
| A1-2 | Sichtmühle, geringer Durchsatz | 0,92 | 2,10 | 4,7 | 1,2 | 4 |
| A1-3 | Sichtmühle, mittlerer Durchsatz | 1,05 | 2,45 | 5,9 | 0,8 | 3 |
| A1-4 | Sichtmühle, hoher Durchsatz | 1,10 | 3,0 | 13,9 | 0,5 | 4 |
| A1-5 | Pralltellermühle | 1,45 | 5,0 | 21 | 0,1 | 0 |

Tabelle 3

| Dispergierdruck / bar absolut | $d_{10}^{A1-2}$ / μm | $d_{50}^{A1-2}$ / μm | $d_{90}^{A1-2}$ / μm |
|---|---|---|---|
| 1,1 | 0,70 | 1,25 | 3,0 |
| 2,0 | 0,92 | 2,10 | 4,7 |
| 4,5 | 1,20 | 4,5 | 62 |

b) Herstellung von feinteiligen Ausgangsmassen A2-1 bis A2-7 (Mo$_{12}$Co$_{5,5}$Fe$_{3,0}$Si$_{1,6}$K$_{0,08}$)

Eine Lösung A wurde hergestellt, indem man in einem wassertemperierten 1,75-m$^3$-Doppelmantelbehälter aus Edelstahl mit einem Balkenrührer bei 60 °C unter Rühren (70 U/min) zu 660 l eine Temperatur von 60 °C aufweisendem Wasser innerhalb einer Minute 1,075 kg einer eine Temperatur von 60 °C aufweisenden wässrigen Kaliumhydroxidlösung (47,5 Gew.-% KOH) und anschließend über eine Differenzialdosierwaage mit einer Dosiergeschwindigkeit von 600 kg/h 237,1 kg Ammoniumheptamolybdattetrahydrat der Temperatur 25 °C (weiße Kristalle mit einer Körnung d < 1 mm, 81,5 Gew.-% MoOs, 7,0-8,5 Gew.-% NH$_3$, max. 150 mg/kg Alkalimetalle, H.C. Starck, D-38642 Goslar) dosierte und die resultierende leicht trübe Lösung bei 60 °C 60 min rührte (70 U/min).

Eine Lösung B wurde hergestellt, indem man in einem wassertemperierten 1,75-m$^3$-Doppelmantelbehälter aus Edelstahl mit einem Balkenrührer bei 60 °C in 282,0 kg einer eine Temperatur von 60 °C aufweisenden wässrigen Kobalt(-II)-nitratlösung (12,5 Gew.-% Co, hergestellt mit Salpetersäure aus Kobaltmetall der Firma MFT Metals & Ferro-Alloys Trading GmbH, D-41747 Viersen, Reinheit, >99,6 Gew.-%, < 0,3 Gew.-% Ni, < 100 mg/kg Fe, < 50 mg/kg Cu) vorlegte und zu dieser unter Rühren (70 U/min) 142,0 kg einer 60 °C warmen Eisen-(III)-nitrat-nonahydrat-Schmelze (13,8 Gew.-% Fe, < 0,4 Gew.-% Alkalimetalle, < 0,01 Gew.-% Chlorid, < 0,02 Gew.-% Sulfat, Dr. Paul Lohmann GmbH, D-81857 Emmerthal) dosierte. Anschließend wurde unter Aufrechterhaltung der 60 °C 30 Minuten nachgerührt. Dann wurde unter Beibehalt der 60 °C die Lösung B in die vorgelegte Lösung A abgelassen und weitere 15 Minuten mit 70 U/min bei 60 °C gerührt. Anschließend wurden dem resultierenden wässrigen Gemisch 19,9 kg eines 25 °C aufweisenden Kieselsols der Fa. Grace vom Typ Ludox TM 50 (50,1 Gew.-% SiO$_2$, Dichte: 1,29 g/ml, pH 8,5 bis 9,5, Alkaligehalt max. 0,5 Gew.-%) zugegeben und danach noch weitere 15 Minuten mit 70 U/min bei 60 °C gerührt. Anschließend wurde in einem Drehscheibensprühturm vom Typ FS-15 der Firma Niro im Heißluftgegenstrom sprühgetrocknet (Gaseintrittstemperatur: 350 ± 10 °C, Gasaustrittstemperatur: 140 ± 5 °C, Durchsatz: 270 kg/h). Durch Einstellung der in Tabelle 4 genannten Scheibendrehzahl und Luftmenge wurden die Sprühpulver (die feinteiligen Ausgangsmassen) A2-1 bis A2-6 mit den dort ebenfalls angegebenen Partikelgrößenverteilungen (bei einem Dispergierdruck von 2,0 bar absolut gemessen) und Glühverlusten GV (3 h bei 600°C unter Luft glühen) erhalten. Fig. 2 gibt die bei einem Dispergierdruck von 2,0 bar absolut gemessenen Partikelgrößenverteilungen ausgewählter Ausgangsmassen A2 zusätzlich im Detail wieder (die Abszisse zeigt die Partikeldurchmesser $d_X^{A2}$ (μm) im logarithmischen Maßstab, die Ordinate zeigt den Volumenanteil der jeweiligen feinteiligen Ausgangsmasse A2 (A2-1 (▲); A2-2 (♦) und A2-4 (■)), der den jeweiligen Durchmesser oder einen kleineren Durchmesser aufweist (Vol.-%). Für die Ausgangsmasse A2-6 gibt Tabelle 5 außerdem exemplarisch den Einfluss des Dispergierdrucks auf die Partikelgrößenverteilung einer Ausgangsmasse A2 wieder. Die Ausgangsmasse A2-7 wurde durch mehrfache Sichtung der Ausgangsmasse A2-2 mit dem Ziel der Erzeugung einer im Wesentlichen einheitlichen Körnung von 30 μm hergestellt.

Tabelle 4

| Ausg.-masse 2 | Zerstäuberscheibendrehzahl / U·min$^{-1}$ | Luftmenge / Nm$^3$·h$^{-1}$ | GV / Gew.-% | $d_{10}^{A2}$ /μm | $d_{50}^{A2}$ /μm | $d_{90}^{A2}$ /μm |
|---|---|---|---|---|---|---|
| A2-1 | 18000 | 2100 | 30,0 | 3,9 | 20,3 | 47,1 |
| A2-2 | 15000 | 2100 | 30,0 | 4,5 | 26,3 | 59,6 |
| A2-3 | 14000 | 2100 | 30,0 | 5,6 | 32,5 | 69,1 |
| A2-4 | 12500 | 2100 | 30,0 | 7,0 | 39,4 | 80,3 |
| A2-5 | 18000 | 2000 | 31,5 | 3,2 | 19,7 | 51,5 |
| A2-6 | 18000 | 2200 | 30,5 | 5,2 | 23,6 | 49,5 |
| A2-7 | mehrfache Sichtung von | A2-2 | 30,0 | 26,1 | 30,0 | 35,0 |

Tabelle 5

| Dispergierdruck / bar absolut | $\overline{d_{10}^{A2-6}}$ /μm | $\overline{d_{50}^{A2-6}}$ /μm | $\overline{d_{90}^{A2-6}}$ /μm |
|---|---|---|---|
| 1,1 | 9,9 | 28,5 | 56,3 |
| 2,0 | 5,2 | 23,6 | 49,5 |

c) Herstellung der ringförmigen Vollkatalysatorformkörper B1 bis B8 und V1 bis V6

Die Sipernat® D17 zugesetzt enthaltenden feinteiligen Ausgangsmassen A1-1 bis A1-5 wurden mit den feinteiligen Ausgangsmassen A2-1 bis A2-7 in der in Tabelle 6 genannten Kombination in für Multimetalloxidaktivmassen der ebenfalls dort genannten Stöchiometrien (Angabe des jeweiligen Koeffizienten a) erforderlichen Mengen (Gesamtmenge: jeweils 3 kg) in einem Eirich-Intensivmischer (Typ R02, Füllvolumen: 3-5 l, Leistung: 1,9 kW, Maschinenfabrik Gustav Eirich GmbH & Co KG, D-74736 Hardheim) mit gegenläufig zum Teller rotierenden Messerköpfen (Drehzahl Teller: 44 U/min, Drehzahl Messerköpfe: 2500 U/min) innerhalb von 5 min homogen vermischt. Bezogen auf die vorgenannte Gesamtmasse wurden zu dieser in einem Rhönradmischer (Raddurchmesser: 650 mm, Trommelvolumen: 5 l) zusätzlich jeweils 1 Gew.-% Graphit TIMREX® T44 der Firma Timcal AG ($d_{50}$ = 20,8 μm) innerhalb von 30 min bei einer Drehzahl von ca. 30 U/min homogen untergemischt. Das resultierende Gemisch wurde dann in einem Laborkalander mit 2 gegenläufigen Stahlwalzen bei einem Pressdruck von 9 bar kompaktiert und durch ein Sieb mit einer Maschenweite von 0,8 mm gedrückt. Das Kompaktat wurde anschließend in obigem Rhönradmischer bei einer Drehzahl von ca. 30 U/min innerhalb von 30 Minuten mit, bezogen auf sein Gewicht, weiteren 2,5 Gew.-% des genannten Graphits vermischt und anschließend wie in der Deutschen Anmeldung mit dem Aktenzeichen 102008040093.9 (DE 102008040093) beschrieben in einem Kilian Rundläufer (9-fach-Tablettiermaschine) vom Typ S100 (Fa. Kilian, D-50735 Köln) unter Stickstoffatmosphäre zu ringförmigen Vollkatalysatorvorläuferformkörpern (Formkörpern V) der Geometrie 5 x 3 x 2 mm (A (Außendurchmesser) x H (Höhe) x I (Innendurchmesser)) mit einer Seitendruckfestigkeit SN von 20 ± 1 N und einer Masse von 125 mg verdichtet (Füllhöhe: 7,5-9 mm, Presskraft: 3,0-3,5 kN). Zur abschließenden thermischen Behandlung wurden jeweils 1000 g der jeweils hergestellten ringförmigen Vollkatalysatorvorläuferformkörper V gleichmäßig aufgeteilt auf nebeneinander angeordnete 4 Gitternetze mit einer quadratischen Grundfläche von jeweils 150 mm x 150 mm (Schütthöhe: ca. 15 mm) in einem mit 650 Nl/h auf anfänglich 140 °C temperierter Luft (anstelle des Luftstroms kann auch ein Gasstrom aus 25 Vol.-% $N_2$ und 75 Vol.-% Luft, oder aus 50 Vol.-% $N_2$ und 50 Vol.-% Luft, oder aus 75 Vol.-% $N_2$ und 25 Vol.-% Luft angewendet werden) durchströmten Umluftofen (Firma Heraeus Instruments GmbH, D-63450 Hanau, Typ: K 750/2) zunächst innerhalb von 120 min von 25°C auf 185 °C aufgeheizt. Diese Temperatur wurde für 1 h aufrechterhalten und dann innerhalb von 50 min auf 225°C erhöht. Die 225°C wurden für 2 h gehalten, bevor die Temperatur innerhalb von 23 min weiter auf 270°C erhöht wurde. Diese Temperatur wurde ebenfalls 1 h aufrechterhalten, bevor sie, mit einer Heizrampe von 1 °C/min auf die in Tabelle 6 genannte Endtemperatur ($T_{Endkalzination}$) erhöht wurde. Diese Endtemperatur wurde für 10 Stunden aufrechterhalten. Danach wurde innerhalb von ca. 12 h auf 25 °C abgekühlt. Die Endtemperatur wurde in allen Fällen im Intervall (463 ± 6) °C liegend gewählt, so dass bei der unter II) beschriebenen Durchführung des Stresstestes in allen Fällen im wesentlichen die gleiche Anfangsaktivität ($T^S_{210h}$, = 328 ± 3 °C) resultierte. Tabelle 6 zeigt zusätzlich die wesentlichen übrigen Herstellungsmerkmale der hergestellten ringförmigen Vollkatalysatorformkörper B1 bis B8 und V1 bis V6.

Tabelle 6: Herstellungsmerkmale der verschiedenen ringförmigen Vollkatalysatorformkörper mit der Stöchiometrie [Bi$_1$W$_2$O$_{7,5}$]$_a$[Mo$_{12}$Co$_{5,5}$Fe$_{3,0}$Si$_{1,6}$K$_{0,08}$O$_x$]$_1$ ihrer Multielementoxidaktivmasse

|  | Pulver A1 | $d_{50}^{A1}$ /μm | Pulver A2 | $d_{90}^{A2}$ /μm | a | F | $T_{Endkalzination}$ / °C |
|---|---|---|---|---|---|---|---|
| V1 | A1-1 | 1,60 | A2-1 | 47,1 | 1,0 | 449 | 457 |
| B1 | A1-1 | 1,60 | A2-4 | 80,3 | 1,0 | 1000 | 457 |
| V2 | A1-2 | 2,10 | A2-1 | 47,1 | 1,0 | 543 | 459 |
| B2 | A1-2 | 2,10 | A2-4 | 80,3 | 1,0 | 1210 | 459 |
| V3 | A1-3 | 2,45 | A2-2 | 59,6 | 1,1 | 783 | 462 |
| B3 | A1-3 | 2,45 | A2-2 | 59,6 | 1,0 | 862 | 462 |
| B4 | A1-3 | 2,45 | A2-2 | 59,6 | 0,7 | 1231 | 465 |
| B5 | A1-3 | 2,45 | A2-2 | 59,6 | 0,5 | 1723 | 468 |
| B6 | A1-4 | 3,0 | A2-2 | 59,6 | 1,0 | 993 | 465 |
| B7 | A1-4 | 3,0 | A2-3 | 69,1 | 1,0 | 1239 | 465 |
| B8 | A1-4 | 3,0 | A2-4 | 80,3 | 1,0 | 1553 | 465 |
| V4 | A1-3 | 2,45 | A2-5 | 51,5 | 0,96 | 721 | 464 |
| V5 | A1-3 | 2,45 | A2-6 | 49,5 | 0,8 | 815 | 460 |
| V6 | A1-5 | 5,0 | A2-7 | 35,0 | 1,0 | 639 | 465 |

II. Ermittlung der Langzeitstabilität der ringförmigen Vollkatalysatorformkörper B1 bis B8 und V1 bis V6 in einer durch sie katalysierten Gasphasenpartialoxidation von Propen zu Acrolein

Zur Ermittlung der Langzeitstabilität der ringförmigen Vollkatalysatorformkörper B1 bis B8 und V1 bis V6 in einer durch sie katalysierten Gasphasenpartialoxidation von Propen zu Acrolein, wurden die ringförmigen Vollkatalysatorformkörper B1 bis B8 und V1 bis V6 dem nachfolgend beschriebenen Stresstest unterworfen.

Ein Reaktionsrohr (V2A Stahl; 21 mm Außendurchmesser, 3 mm Wandstärke, 15 mm Innendurchmesser, 120 cm Länge) wurde von oben nach unten in Strömungsrichtung des Reaktionsgasgemischs jeweils wie folgt beschickt:

Abschnitt 1:   ca. 30 cm Länge

40 g Steatitkugeln (C220 Steatit der Fa. CeramTec) mit einem Durchmesser von 1,5 bis 2,0 mm als Vorschüttung (Aufheizzone).

Abschnitt 2:   ca. 70 cm Länge

Eine homogene Mischung aus 90 g des jeweiligen ringförmigen Vollkatalysatorformkörpers und 10 g Steatitringen (C220 Steatit der Fa.

CeramTec) derselben Ringgeometrie wie der jeweilige Vollkatalysatorformkörper als Katalysatorfestbett.

Die Temperierung des Reaktionsrohres erfolgte jeweils mittels eines mit molekularem Stickstoff durchperlten, die jeweils erforderliche Salzbadtemperatur $T^S$ (°C) aufweisenden Salzbades (53 Gew.-% Kaliumnitrat, 40 Gew.-% Natriumnitrit und 7 Gew.-% Natriumnitrat).

Während der Durchführung des Stresstests wurde das Reaktionsrohr kontinuierlich mit einem Reaktionsgasausgangsgemisch (Beschickungsgasgemisch aus Luft, polymer grade Propen und molekularem Stickstoff) der nachfolgenden Zusammensetzung beschickt:

5 Vol.-%     Propen (polymer grade),

9,5 Vol.-%     molekularer Sauerstoff, und

85,5 Vol.-%     $N_2$.

Zum Zweck der Formierung des frisch in das Reaktionsrohr eingebrachten Katalysatorfestbetts wurde $T^S$ während der ersten 210 h Betriebsdauer bei einem in das Reaktionsrohr geführten Volumenstrom des Reaktionsgasausgangsgemischs (dessen Eintrittstemperatur in das Reaktionsrohr ca. 30 °C betrug) von 90 NI/h so einjustiert, dass der Propenumsatz $U^P$ bei einmaligem Durchgang des Beschickungsgasgemischs durch das Reaktionsrohr kontinuierlich etwa 95 mol-% betrug (die sich nach einer Betriebsdauer von 90 h einstellenden Anfangsselektivitäten der Zielproduktgesamtbildung (Acrolein + Acrylsäure) waren (in mol-% der beim Einmaldurchgang durch des Reaktionsrohr umgesetzten molaren Propenmenge): 96 (V1); 94,5 (B1); 95,8 (V2); 94,5 (B2); 94,5 (V3); 94,6 (B3); 94,2 (B4); 93,8 (B5); 94,4 (B6); 94,5 (B7); 93,6 (B8); 94,9 (V4); 94,3 (V5) und 94,9 (V6)). Der Druck am Eingang in das Reaktionsrohr betrug 1,2 bar absolut. Die am Ende der Formierungsphase zur Zielumsatzerreichung (95 mol-%) erforderliche Salzbadtemperatur $T^S_{210h}$ (°C) ist ein Maß für die jeweilige Anfangsaktivität des jeweiligen Katalysatorfestbetts. Tabelle 7 weist aus, dass die ringförmigen Vollkatalysatorformkörper B1 bis B8 und V1 bis V6 eine im Wesentlichen identische Anfangsaktivität aufwiesen.

Im Anschluss an die Formierung wurde der in das Reaktionsrohr geführte Volumenstrom an Reaktionsgasausgangsgemisch auf 200 NI/h erhöht und für einen Zeitraum von 216 h beibehalten. Die Salzbadtemperatur $T^S$ wurde im vorgenannten Zeitraum unabhängig vom sich jeweils einstellenden Propenumsatz auf den konstanten Wert von 380 °C eingestellt (der Druck am Eingang in das Reaktionsrohr betrug 1,6 bar absolut).

Nach Beendigung der vorgenannten Stressphase wurde der in das Reaktionsrohr geführte Volumenstrom an Reaktionsgasausgangsgemisch wieder auf die für den eigentlichen Normalbetrieb der Propenpartialoxidation vorgesehenen 90 NI/h verringert. Unter Beibehalt dieses Volumenstroms über 94 weitere Betriebsstunden, wurde die Salzbadtemperatur $T^S$ wieder so einjustiert, dass sich kontinuierlich der (auf einen Einmaldurchgang des Reaktionsgasausgangsgemischs durch das Reaktionsrohr bezogene) ins Auge gefasste Zielumsatz an Propen ($U^P$) von etwa 95 mol-% einstellte.

Die am Ende der dann insgesamt 520 Betriebsstunden zur Erlangung des Zielumsatzes (95 mol-%) erforderliche Salzbadtemperatur $T^S_{520h}$ (°C) reflektiert die am Ende des Stresstests jeweils vorliegende Endaktivität des jeweiligen Katalysatorfestbetts.

Die Differenz $T^S_{520h}$ (°C) - $T^S_{210h}$ (°C) = $\Delta T^S$ bestimmt die Ordnungsrelation innerhalb der ringförmigen Vollkatalysatorformkörper B1 bis B8 und V1 bis V6 hinsichtlich deren Langzeitstabilität in der durch sie katalysierten Propenpartialoxidation zu Acrolein (Hauptprodukt) und Acrylsäure (Nebenprodukt). Je kleiner $\Delta T^S$, desto geringer ist die Desaktivierungsrate des Katalysatorfestbetts bzw. der in selbigem enthaltenen ringförmigen Vollkatalysatorformkörper im Partialoxidationsbetrieb. Tabelle 7 weist aus, dass die Desaktivierungsrate des jeweiligen ringförmigen Vollkatalysatorformkörpers bei im wesentlichen einheitlicher Anfangsaktivität ( $T^S_{210h}$ (°C)) und im wesentlichen einheitlicher Endselektivität $S_{520h}$ der Zielproduktgesamtbildung (Acrolein + Acrylsäure, in mol-% der beim Einmaldurchgang durch das Reaktionsrohr umgesetzten molaren Propenmenge) um so geringer ist, je größer der erfindungsgemäße Stabilitätswert F des jeweiligen ringförmigen Vollkatalysatorformkörpers ist.

Tabelle 7

| Katalysatorformkörper | F | $T_{210h}^{S}$ / °C | $\Delta T^{S}$/°C | $S_{520h}$ / mol-% |
|---|---|---|---|---|
| V1 | 449 | 326 | 14 | 96,7 |
| B1 | 1000 | 330 | 10 | 96,4 |
| V2 | 543 | 326 | 12 | 96,7 |
| B2 | 1210 | 328 | 8 | 97,0 |
| V3 | 783 | 327 | 11 | 96,8 |
| B3 | 862 | 326 | 9 | 96,7 |
| B4 | 1231 | 325 | 5 | 96,7 |
| B5 | 1723 | 325 | 2 | 96,8 |
| B6 | 993 | 328 | 7 | 96,9 |
| B7 | 1239 | 329 | 6 | 96,9 |
| B8 | 1553 | 331 | 4 | 96,8 |
| V4 | 721 | 328 | 11 | 96,8 |
| V5 | 815 | 326 | 11 | 96,7 |
| V6 | 639 | 325 | 12 | 96,6 |

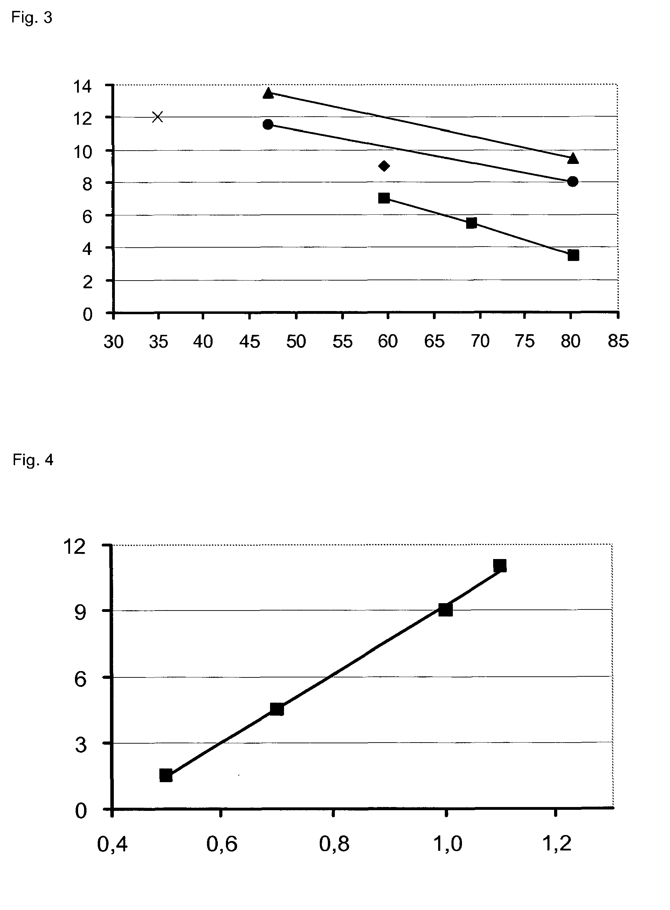

Figur 3 (die Abszisse zeigt $d_{90}^{A2}$ (in μm) und die Ordinate $\Delta T^{S}$ (in °C)) weist als Auswertung über die ringförmigen Vollkatalysatorformkörper V1, B1, V2, B2, B3, B6, B7, B8 und V6 im Blick von jeweils links nach rechts aus, dass sich bei jeweiliger Verwendung derselben feinteiligen Ausgangsmasse A1 (A1-1(▲); A1-2 (●); A1-3 (♦); A1-4 (■) und A1-5 (X)) sowie konstantem Koeffizienten a und gleicher chemischer Beschaffenheit der feinteiligen Ausgangsmasse A2 durch eine Vergröberung der feinteiligen Ausgangsmasse A2 (ein zunehmendes $d_{90}^{A2}$) die Desaktivierungsrate ($\Delta T^{S}$) bei im wesentlichen einheitlicher Anfangsaktivität ($T_{210h}^{S}$) signifikant verringern lässt.

Umgekehrt weist eine Betrachtung der selben Figur 3 im Blick von jeweils oben nach unten aus, dass sich bei jeweiliger Verwendung derselben feinteiligen Ausgangsmasse A2 sowie konstantem Koeffizienten a und gleicher chemischer Beschaffenheit der feinteiligen Ausgangsmasse A1 durch eine Vergröberung der feinteiligen Ausgangsmasse A1 (ein zunehmendes $d_{50}^{A1}$) die Desaktivierungsrate ($\Delta T^{S}$) bei im wesentlichen einheitlicher Anfangsaktivität ($T_{210h}^{S}$) ebenfalls verringern lässt. Dabei ist insbesondere die Abhängigkeit der Desaktivierungsrate von $d_{90}^{A2}$ ausgeprägt. Bemerkenswerter Weise fügt sich auch der ringförmige Vergleichsvollkatalysator V6 nahtlos in diese Systematik ein, zu dessen Herstellung gezielt eine vergleichsweise monodisperse feinteilige Ausgangsmasse A2 verwendet wurde. Die Tatsache, dass sich der Vergleichsvollkatalysator V6 bei einer entsprechenden Auftragung über $d_{50}^{A2}$ nicht in entsprechend nahtloser Weise einfügen würde, lässt den Rückschluss zu, dass für die Dämpfung der Desaktivierungsrate das Vorhandensein eines Anteils vergleichsweise großer Partikel in der feinteiligen Ausgangsmasse A2 wesentlich ist. D. h., bei einer im Wesentlichen einheitlichen Körnung der feinteiligen Ausgangsmasse A2 sollte diese im erfindungsgemäßen Sinn vergleichsweise grobteilig gewählt werden.

Figur 4 weist als Auswertung über die ringförmigen Vollkatalysatorformkörper V3, B3, B4 und B5 aus (die Abszisse zeigt den stöchiometrischen Koeffizienten a und die Ordinate $\Delta T^{S}$ (in °C)), dass bei Verwendung derselben feinteiligen Ausgangsmasse A1 (A1-3) und derselben feinteiligen Ausgangsmasse A2 (A2-2) die Desaktivierungsrate ($\Delta T^{S}$) mit abnehmendem stöchiometrischem Koeffizienten a signifikant abnimmt. Das heißt, bereits durch die alleinige Maßnahme der Verringerung des molaren Verhältnisses $n_{Bi}/n_{Mo}$ (n = molare Menge des jeweiligen indizierten Elements) im die Aktivmasse bildenden Multielementoxid der relevanten Vollkatalysatorformkörper lässt sich $\Delta T^{S}$ bei im Wesentlichen gleicher Anfangsaktivität ($T_{210h}^{S}$ = (326 ± 1) °C) von 11 auf 2 verringern. Ein Beibehalt der bei der Herstellung von V3 angewandten Endkalzinationstemperatur bei der Herstellung von B4 und B5 würde den $\Delta T^{S}$-Unterschied noch verstärken.

Tabelle 8 zeigt den Rahmen auf, innerhalb dessen bei gleicher chemischer Beschaffenheit der feinteiligen Ausgangsmassen A1 und A2, jedoch variabler Körnung derselben sowie variablem stöchiometrischem Koeffizienten a und im Übrigen gleicher Herstellweise wie bei den ringförmigen Vollkatalysatorformkörpern B1 bis B8 sowie V1 bis V6 erfindungsgemäße Stabilitätswerte F eingestellt werden können.

EP 2 331 258 B1

Tabelle 8

| $d_{50}^{A1}$ /µm | $d_{50}^{A2}$ /µm | $d_{90}^{A2}$ /µm | a | F | $d_{50}^{A1}$ /µm | $d_{50}^{A2}$ /µm | $d_{90}^{A2}$ /µm | a | F |
|---|---|---|---|---|---|---|---|---|---|
| 2 | 36,3 | 72,3 | 1 | 999 | 2,6 | 27,2 | 59,2 | 1 | 889 |
| 2 | 38,6 | 78 | 1 | 1119 | 2,6 | 29,1 | 60,2 | 1 | 912 |
| 2,2 | 36,3 | 72,3 | 1 | 1068 | 2,6 | 36,3 | 72,3 | 1 | 1200 |
| 2,2 | 38,6 | 78 | 1 | 1196 | 2,6 | 38,6 | 78 | 1 | 1345 |
| 2,2 | 27,2 | 59,2 | 0,9 | 879 | 2,6 | 27,2 | 59,2 | 0,9 | 988 |
| 2,2 | 29,1 | 60,2 | 0,9 | 901 | 2,6 | 29,1 | 60,2 | 0,9 | 1013 |
| 2,2 | 36,3 | 72,3 | 0,9 | 1186 | 2,6 | 36,3 | 72,3 | 0,9 | 1333 |
| 2,2 | 38,6 | 78 | 0,9 | 1329 | 2,6 | 38,6 | 78 | 0,9 | 1494 |
| 2,2 | 27,2 | 59,2 | 0,8 | 989 | 2,6 | 27,2 | 59,2 | 0,8 | 1111 |
| 2,2 | 29,1 | 60,2 | 0,8 | 1014 | 2,6 | 29,1 | 60,2 | 0,8 | 1140 |
| 2,2 | 36,3 | 72,3 | 0,8 | 1334 | 2,6 | 36,3 | 72,3 | 0,8 | 1500 |
| 2,2 | 38,6 | 78 | 0,8 | 1495 | 2,6 | 38,6 | 78 | 0,8 | 1681 |
| 2,2 | 27,2 | 59,2 | 0,7 | 1130 | 2,6 | 27,2 | 59,2 | 0,7 | 1270 |
| 2,2 | 29,1 | 60,2 | 0,7 | 1159 | 2,6 | 29,1 | 60,2 | 0,7 | 1303 |
| 2,2 | 36,3 | 72,3 | 0,7 | 1525 | 2,6 | 36,3 | 72,3 | 0,7 | 1714 |
| 2,2 | 38,6 | 78 | 0,7 | 1709 | 2,6 | 38,6 | 78 | 0,7 | 1921 |
| 2,2 | 27,2 | 59,2 | 0,6 | 1318 | 2,6 | 27,2 | 59,2 | 0,6 | 1482 |
| 2,2 | 29,1 | 60,2 | 0,6 | 1352 | 2,6 | 29,1 | 60,2 | 0,6 | 1520 |
| 2,2 | 36,3 | 72,3 | 0,6 | 1779 | 2,6 | 36,3 | 72,3 | 0,6 | 2000 |
| 2,2 | 38,6 | 78 | 0,6 | 1994 | 2,6 | 38,6 | 78 | 0,6 | 2241 |
| 2,4 | 27,2 | 59,2 | 1 | 841 | 2,8 | 27,2 | 59,2 | 1 | 936 |
| 2,4 | 29,1 | 60,2 | 1 | 862 | 2,8 | 29,1 | 60,2 | 1 | 960 |
| 2,4 | 36,3 | 72,3 | 1 | 1135 | 2,8 | 36,3 | 72,3 | 1 | 1264 |
| 2,4 | 38,6 | 78 | 1 | 1271 | 2,8 | 38,6 | 78 | 1 | 1416 |
| 2,4 | 27,2 | 59,2 | 0,9 | 934 | 2,8 | 27,2 | 59,2 | 0,9 | 1041 |
| 2,4 | 29,1 | 60,2 | 0,9 | 958 | 2,8 | 29,1 | 60,2 | 0,9 | 1067 |
| 2,4 | 36,3 | 72,3 | 0,9 | 1261 | 2,8 | 36,3 | 72,3 | 0,9 | 1404 |
| 2,4 | 38,6 | 78 | 0,9 | 1413 | 2,8 | 38,6 | 78 | 0,9 | 1574 |
| 2,4 | 27,2 | 59,2 | 0,8 | 1051 | 2,8 | 27,2 | 59,2 | 0,8 | 1171 |
| 2,4 | 29,1 | 60,2 | 0,8 | 1078 | 2,8 | 29,1 | 60,2 | 0,8 | 1200 |
| 2,4 | 36,3 | 72,3 | 0,8 | 1418 | 2,8 | 36,3 | 72,3 | 0,8 | 1580 |
| 2,4 | 38,6 | 78 | 0,8 | 1589 | 2,8 | 38,6 | 78 | 0,8 | 1770 |

EP 2 331 258 B1

(fortgesetzt)

| $\overline{d_{50}^{A1}}$ /μm | $\overline{d_{50}^{A2}}$ /μm | $\overline{d_{90}^{A2}}$ /μm | a | F | $\overline{d_{50}^{A1}}$ /μm | $\overline{d_{50}^{A2}}$ /μm | $\overline{d_{90}^{A2}}$ /μm | a | F |
|---|---|---|---|---|---|---|---|---|---|
| 2,4 | 27,2 | 59,2 | 0,7 | 1201 | 2,8 | 27,2 | 59,2 | 0,7 | 1338 |
| 2,4 | 29,1 | 60,2 | 0,7 | 1232 | 2,8 | 29,1 | 60,2 | 0,7 | 1372 |
| 2,4 | 36,3 | 72,3 | 0,7 | 1621 | 2,8 | 36,3 | 72,3 | 0,7 | 1806 |
| 2,4 | 38,6 | 78 | 0,7 | 1816 | 2,8 | 38,6 | 78 | 0,7 | 2023 |
| 2,4 | 27,2 | 59,2 | 0,6 | 1401 | 2,8 | 27,2 | 59,2 | 0,6 | 1561 |
| 2,4 | 29,1 | 60,2 | 0,6 | 1437 | 2,8 | 29,1 | 60,2 | 0,6 | 1600 |
| 2,4 | 36,3 | 72,3 | 0,6 | 1891 | 2,8 | 36,3 | 72,3 | 0,6 | 2107 |
| 2,4 | 38,6 | 78 | 0,6 | 2119 | 2,8 | 38,6 | 78 | 0,6 | 2360 |

Figur 5 zeigt im Überblick eine Auftragung der in der beschriebenen Stresstestung der ringförmigen Vollkatalysatorformkörper B1 bis B8 (♦) und V1 bis V6 (◇) ermittelten $\Delta T^S$ (in °C als Ordinate) in Abhängigkeit von den zugehörigen Stabilitätswerten F (als Abszisse).

III. Bestimmung der Kristallphasenzusammensetzung eines erfindungsgemäß hergestellten ringförmigen Katalysatorformkörpers K (F = 1091) und eines nicht erfindungsgemäß hergestellten ringförmigen Katalysatorformkörpers K* (F = 803) im Zustand ihrer Desaktivierung und im unbenutzten Zustand

Die Desaktivierung erfolgte in beiden Fällen im Rahmen ihrer Verwendung als Katalysatoren für die heterogen katalysierte partielle Gasphasenoxidation von Propylen zu Acrolein unter vergleichbaren Betriebsbedingungen in salzbadgekühlten Rohrreaktoren wie in den Schriften WO 2005/42459 und WO 2005/49200 beschrieben. Die Herstellung der beiden ringförmigen Katalysatorformkörper erfolgte wie unter I. beschrieben. Im Fall des Katalysatorformkörpers K war $d_{90}^{A1} = 2\ \mu m$, $d_{90}^{A2} = 72\ \mu m$ und a = 0,91. Im Fall des Katalysatorformkörpers K* war $d_{50}^{A1} = 2\ \mu m$, $d_{90}^{A2} = 60\ \mu m$ und a = 0,94.

Die wie in II. beschrieben ermittelte Anfangsaktivität der beiden Katalysatorformkörper lag im Intervall $T_{210h}^{S} = 317 \pm 5\ °C$.

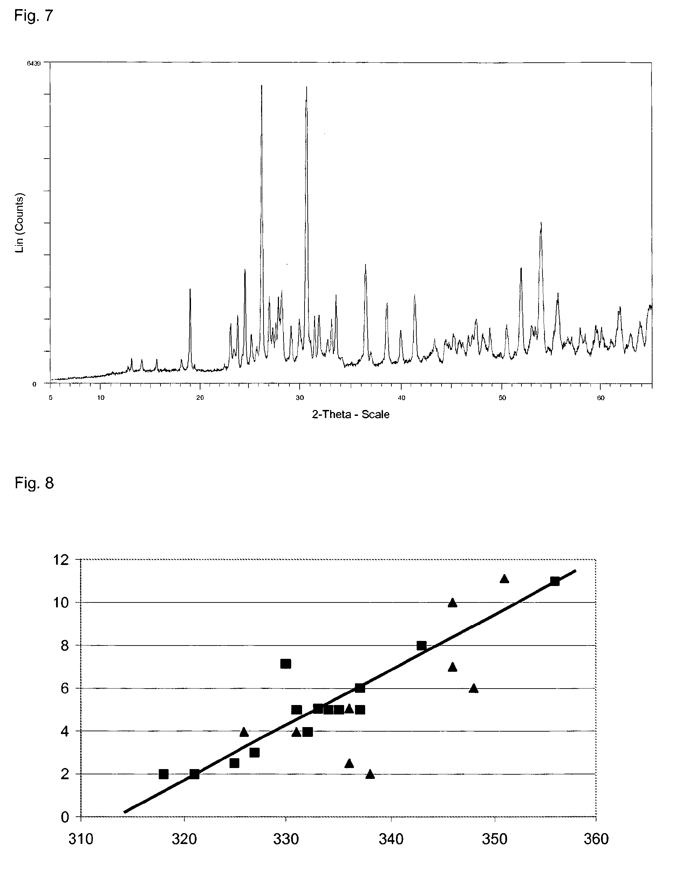

Fig. 6 zeigt beispielhaft das Pulverröntgendiffraktogramm des ringförmigen Katalysatorformkörpers K im unbenutzten Zustand und Fig. 7 zeigt beispielhaft das Pulverröntgendiffraktogramm des ringförmigen Katalysatorformkörpers K im im Rahmen der heterogen katalysierten partiellen Gasphasenoxidation von Propen zu Acrolein desaktivierten Zustand (Ausbauprobe aus der Heißpunktzone). Die Abszisse zeigt dabei den Beugungswinkel in der 20-Skala (2 Theta-Skala) und die Ordinate zeigt die absolute Intensität der gebeugten Röntgenstrahlung.

Durch Analyse des Röntgendiffraktogramms (vgl. z. B. Moderne Röntgenbeugung, Spieß-Schwarzer-Behnken-Teichert, Vieweg + Teubner (2005); R. A. Young, The Rietveld Methode, IUCr, Oxford University Press (1995); und H. Krischner, Röntgenstrukturanalyse und Rietveldmethode, Vieweg Lehrbuch, 5. Auflage, Braunschweig (1994)) lässt sich sowohl die qualitative als auch die quantitative Kristallphasenzusammensetzung ermitteln. Grundlage der Ermittlung der qualitativen Kristallphasenzusammensetzung ist der Sachverhalt, dass das Röntgendiffraktogramm eines Gemisches von Kristallphasen eine gewichtete Summe aus den Röntgendiffraktogrammen der verschiedenen darin enthaltenen einzelnen kristallinen Phasen ist. Da die Elementzusammensetzung der Multielementoxid-I-Aktivmasse bekannt ist, lassen sich diejenigen Kristallphasen eingrenzen, die sich bei einem Vorhandensein der vorgenannten Elemente grundsätzlich ausbilden können.

Die Pulverröntgendiffraktogramme derselben stehen der Öffentlichkeit in entsprechenden Datensammlungen zur Verfügung (z. B. The International Centre of Diffraction Data (ICDD) mit seinem Headquarter in 12 Campus Boulevard, Newton Square, PA, USA, 19073 - 3273). Durch einfachen Abgleich der Lagen der im Röntgendiffraktogramm enthaltenen Beugungsreflexe mit jenen aus der Datensammlung, lässt sich somit die qualitative Kristallphasenzusammensetzung (d.h., die Sorte der enthaltenen einzelnen Phasen) ermitteln. Die zur quantitativen Bestimmung der Kristallphasenzusammensetzung zusätzlich benötigten Kristallstrukturen (der jeweiligen Phasen) können ebenfalls entsprechenden Datenbanken entnommen werden (z. B. der Inorganic Crystal Structure Database (ICSD) des Fachinformationszentrums (FIZ) D-76102 Karlsruhe)

Die klassische Methode der quantitativen Phasenzusammensetzung suchte sich im Röntgendiffraktogramm gut separierte Reflexe der jeweiligen individuellen enthaltenen Kristallphasen und bestimmte aus deren relativen Intensitäten unter Einbezug von Standards die relativen Gewichtsanteile der einzelnen Phasen.

Bei komplizierteren Fällen mit vielen Phasen ist aber die Trennung einzelner Beugungslinien oft nicht mehr möglich. Hier simuliert man daher nach Rietveld die Überlagerung der Pulverdiffraktogramme derjenigen Kristallphasen, die man bei der qualitativen Phasenanalyse gefunden hat. Diese Einhüllende wird per "least squares"- Verfahren, bei dem die Parameter der den Einzelpulverdiagrammen zugrunde liegenden Strukturmodelle variiert werden (Multiphasen-Rietveldmethode), optimal an das gemessene Pulverröntgendiffraktogramm des Gemischs angepasst.

Anstelle der Intensitäten der Einzellinien der jeweiligen Kristallphasen wird so die Intensität aller Linien der Pulverdiffraktogramme der jeweiligen Kristallphasen in Form eines phasenspezifischen "Skalierungsfaktors" berücksichtigt.

Nach einer von Hill und Howard (J. Appl. Cryst. 20 (1987) 467 - 474) aufgestellten Beziehung, ist der jeweilige Skalierungsfaktor über für die Kristallstruktur (den Aufbau ihrer Elementarzelle) der zugehörigen Phase charakteristische Konstanten (die in der ICSD-Datenbank hinterlegt sind) mit dem relativen Gewichtsanteil der jeweiligen Kristallphase am Phasengemisch verknüpft, wodurch sich die relativen Gewichtsanteile aus den Skalierungsfaktoren ermitteln lassen. Der erhebliche Vorteil der Anwendung der Multiphasen-Rietveldmethode zur Analyse der quantitativen Phasenzusammensetzung liegt dabei darin begründet, dass sie nicht der Mitverwendung von stofflichen Standards bei der Aufnahme des Röntgendiffraktogramms bedarf, da der "Standard" bei dieser Methode in der bekannten Kristallstruktur der einzelnen Phase besteht.

Im hier vorliegenden Fall erfolgte die Analyse des Röntgendiffraktogramms mit Hilfe des von der Firma Bruker AXS GmbH, D-76187 Karlsruhe diesbezüglich entwickelten DIFFRAC[plus] TOPAS Softwarepakets.

Für die unbenutzten ringförmigen Katalysatorformkörper K, K* ergab sich folgende Kristallphasenzusammensetzung (in Gew.-% bezogen auf das jeweilige Gesamtgewicht):

| Kristallphase | K | K* |
|---|---|---|
| $\beta$-(Co, Fe$^{II}$)MoO$_4$ | 44 | 38 |
| Bi$_1$W$_1$O$_{4,5}$ | 13 | 11 |
| WO$_3$ | 8 | 7 |
| MoO$_3$ | 9 | 7 |
| Fe$^{III}_2$(MoO$_4$)$_3$ | 20 | 29 |
| $\alpha$-(Co, Fe$^{II}$)MoO$_4$ | 7 | 8 |
| CoWO$_4$ | 0 | 0 |
| Bi$_2$Mo$_3$O$_{12}$ | 0 | 0 |

Nach mehrjährigem (> 2 J. in beiden Fällen) Betrieb der jeweiligen heterogen katalysierten partiellen Propenoxidation ergab eine entsprechende Kristallphasenanalyse bei vergleichbarem Desaktivierungsgrad des jeweiligen Katalysatorfestbetts die folgenden Ergebnisse (die Ziffer "1" zeigt an, dass sich die Analyse auf eine Ausbauprobe aus der Heißpunktzone des Katalysatorfestbetts bezieht; die Ziffer "2" zeigt an, dass sich die Analyse auf eine Ausbauprobe aus der Hauptzone des Katalysatorfestbetts bezieht),
wobei die Zahlenangaben wieder Gew.-% bezogen auf das jeweilige Gesamtgewicht sind:

| Kristallphase | K-1 | K*-1 | K-2 | K*-2 |
|---|---|---|---|---|
| $\beta$-(Co, Fe$^{II}$)MoO$_4$ | 46 | 46 | 47 | 38 |
| WO$_3$ | 2 | 7 | 7 | 8 |
| $\alpha$-(Co, Fe$^{II}$)MoO$_4$ | 8 | 13 | 6 | 11 |
| MoO$_3$ | 0 | 4 | 7 | 8 |
| Bi$_1$W$_1$O$_{4,5}$ | -0,7/J | -2,5/J | -0,6/J | -1,5/J |
| Fe$^{III}$2(MoO$_4$)$_3$ | -2/J | -9/J | -0,7/J | -3,5/J |
| CoWO$_4$ | +2,7/J | +3/J | +0,6/J | $\pm$0/J |
| Bi$_2$Mo$_3$O$_{12}$ | +1,1/J | +3,5/J | +0,7/J | +2,5/J |

Im Fall der letzten 4 Kristallphasen sind die durchschnittlichen Bildungs(+)- bzw. Abbau(-)raten in Gew.-%/Jahr angegeben.

Die wesentlich höhere Bildungsrate an Bi$_2$Mo$_3$O$_{12}$ im Fall des ringförmigen Katalysatorformkörpers K* korreliert mit dessen wesentlich höherer Desaktivierungsrate besonders gut.

Hinsichtlich der quantitativen Co- und Fe$^{II}$-Anteile in den jeweiligen $\alpha$-, $\beta$-MoO$_4$-Phasen und die Veränderung dieser Anteile über die jeweilige Betriebsdauer ist keine präzise Aussage möglich.

Die Bildung von CoWO$_4$ und Bi$_2$Mo$_3$O$_{12}$ unter Abbau von Bi$_1$W$_1$O$_{4,5}$ weist zwischen den Ausgangsmassen der ringförmigen Vollkatalysatoren abgelaufene Migrationsprozesse aus. Neben einer Fülle verschiedener sonstiger Migrations- und Phasenumwandlungsprozesse, deren Einfluss auf die katalytische Aktivität vergleichsweise beschränkt ist, dominiert die Bi$_2$Mo$_3$O$_{12}$ Bildung die Desaktivierung. Hervorzuheben sind unter diesen anderen Prozessen der Verlust an MoO$_3$ über die Gasphase, die Reduktion des im Fe$^{III}_2$ (MoO$_4$)$_3$ enthaltenen 3-wertigen Eisens, verbunden mit einer Anreicherung des Eisens im $\beta$-(Co, Fe$^{II}$)MoO$_4$, sowie die Bildung der Co-reichen Phasen $\alpha$-(Co, Fe$^{II}$)MoO$_4$ und CoWO$_4$ und das aus der Veränderung der Linienbreite ablesbare Kristallwachstum der $\beta$-(Co, Fe$^{II}$)MoO$_4$-Phase (ca. 40 bis 60 nm bei den ungenutzten ringförmigen Katalysatorformkörpern K, K* und ca. 70 bis 110 nm bei den desaktivierten ringförmigen Katalysatorformkörpern K, K*).

In Ergänzung zu den vorstehenden Ausführungen zeigt Fig. 8 für verschiedene, in entsprechender Weise wie die vorstehenden ringförmigen Vollkatalysatorformkörper K und K* hergestellte, ringförmige Vollkatalysatorformkörper (ihre wie in II. beschrieben für ihren unbenutzten Zustand ermittelten Anfangsaktivitäten lagen im Intervall

$$T^S_{210h} = \ 317 \pm 5\ °C;$$ ihre Stabilitätswerte F lagen im Intervall $800 \leq F \leq 1500$) den Gewichtsanteil an Bi$_2$Mo$_3$O$_{12}$ (aufgetragen auf der Ordinate in Gew.-% (bezogen auf das Gesamtgewicht des jeweiligen Vollkatalysatorformkörpers) und wie beschrieben aus dem zugehörigen Röntgendiffraktogramm ermittelt), den sie in verschiedenen während einer durch sie katalysierten partiellen Gasphasenoxidation von Propen zu Acrolein vorliegenden Desaktivierungszuständen aufwiesen (die dazu jeweils untersuchten Vollkatalysatorformkörper wurden sowohl aus der Hauptzone (■) als auch aus der Heißpunktzone (▲) entnommen und anschließend jeweils einer wie in II. für unbenutzte Vollkatalysatorformkörper beschriebenen Bestimmung von $T_{210h}^{S}$ unterworfen; die Abszisse zeigt die den jeweiligen Aktivitätszustand wiedergebenden $T_{210h}^{S}$ in °C).

Die Fig. 8 belegt, dass der Desaktivierungsgrad (das $T_{210h}^{S}$) umso größer ist, je größer der vorliegende Gewichtsanteil an $B_{12}Mo_3O_{12}$ ist.

## Patentansprüche

1. Verfahren zur Herstellung von geometrischen Katalysatorformkörpern K, die als Aktivmasse ein Multielementoxid I der allgemeinen Stöchiometrie I,

$$[Bi_1W_bO_x]_a[Mo_{12}Z^1bZ^2_dFe_eZ^3_fZ^4_gZ^5_hO_y]_1 \qquad (I),$$

mit

$Z^1$ = ein Element oder mehr als ein Element aus der Gruppe bestehend aus Nickel und Kobalt,
$Z^2$ = ein Element oder mehr als ein Element aus der Gruppe bestehend aus den Alkalimetallen, den Erdalkalimetallen und Thallium,
$Z^3$ = ein Element oder mehr als ein Element aus der Gruppe bestehend aus Zink, Phosphor, Arsen, Bor, Antimon, Zinn, Cer, Vanadium, Chrom und Wismut,
$Z^4$ = ein Element oder mehr als ein Element aus der Gruppe bestehend aus Silizium, Aluminium, Titan, Wolfram und Zirkonium,
$Z^5$ = ein Element oder mehr als ein Element aus der Gruppe bestehend aus Kupfer, Silber, Gold, Yttrium, Lanthan und den Lanthaniden,
a = 0,1 bis 3,
b = 0,1 bis 10,
c = 1 bis 10,
d = 0,01 bis 2,
e = 0,01 bis 5,
f = 0 bis 5,
g = 0 bis 10,
h = 0 bis 1, und
x, y = Zahlen, die durch die Wertigkeit und Häufigkeit der von Sauerstoff verschiedenen Elemente in I bestimmt werden,

enthalten, bei dem man

- ein feinteiliges Mischoxid $Bi_1W_bO_x$ mit einem in der Längeneinheit μm angegebenen Partikeldurchmesser $d_{50}^{A1}$ als Ausgangsmasse A1 mit der Maßgabe vorbildet, dass $1\ \mu m \leq d_{50}^{A1} \leq 10\ \mu m$ erfüllt ist;
- unter Verwendung von Quellen der von Sauerstoff verschiedenen Elemente des Anteils T = $[Mo_{12}Z^1_cZ^2_dFe_eZ^3_fZ^4_gZ^5_hO_y]_1$ des Multielementoxids 1 in wässrigem Medium mit der Maßgabe eine innige wässrige Mischung M erzeugt, dass

- jede der verwendeten Quellen im Verlauf der Herstellung der wässrigen Mischung M einen Zerteilungsgrad Q durchläuft, der **dadurch gekennzeichnet ist, dass** sein Durchmesser $d_{90}^{Q} \leq 5\ \mu m$ beträgt, und
- die wässrige Mischung M die Elemente Mo, $Z^1$, $Z^2$, Fe, $Z^3$, $Z^4$ und $Z^5$ in der Stöchiometrie I*,

$$Mo_{12}Z^1_cZ^2_dFe_eZ^3_fZ^4_gZ^5_h \qquad (I^*),$$

enthält;

- aus der wässrigen Mischung M durch Trocknen und Einstellen des Zerteilungsgrades $d_{90}^{A2}$ eine feinteilige Ausgangsmasse A2 mit einem in der Längeneinheit μm angegebenen Partikeldurchmesser $d_{90}^{A2}$ unter der Maßgabe erzeugt, dass $200 \ \mu m \geq d_{90}^{A2} \geq 20 \ \mu m$ erfüllt ist;

- Ausgangsmasse A1 und Ausgangsmasse A2, oder Ausgangsmasse A1, Ausgangsmasse A2 und feinteilige Formgebungshilfsmittel zu einer feinteiligen Ausgangsmasse A3 mit der Maßgabe miteinander vermischt, dass die Ausgangsmasse A3 die über die Ausgangsmassen A1 und A2 in die Ausgangsmasse A3 eingebrachten, von Sauerstoff verschiedenen, Elemente des Multielementoxids I in der Stöchiometrie I\*\*,

$$[Bi_1W_b]_a[Mo_{12}Z^1{}_cZ^2{}_dFe_eZ^3{}_fZ^4{}_gZ^5{}_h]_1 \qquad (I^{**}),$$

enthält,

- mit feinteiliger Ausgangsmasse A3 geometrische Formkörper V formt, und
- die Formkörper V bei erhöhter Temperatur unter Erhalt der geometrischen Katalysatorformkörper K thermisch behandelt,

**dadurch gekennzeichnet, dass** der Betrag F des Produktes

$$(d_{50}^{A1})^{0,7} \quad \bullet \quad (d_{90}^{A2})^{1,5} \quad \bullet \quad (a^{-1})$$

$\geq 820$ beträgt,

wobei die Partikeldurchmesser $d_{50}^{A1}$, $d_{90}^{O}$ und $d_{90}^{A2}$ aus der mittels Laserbeugung nach ISO 13320 bei einem Dispergierdruck von 2 bar absolut bestimmten volumenbezogenen Partikeldurchmesser ermittelt werden.

2. Verfahren nach Anspruch 1, **dadurch gekennzeichnet, dass** F $\geq$ 830 beträgt.

3. Verfahren nach Anspruch 1, **dadurch gekennzeichnet, dass** F $\geq$ 840 beträgt.

4. Verfahren nach Anspruch 1, **dadurch gekennzeichnet, dass** F $\geq$ 850 beträgt.

5. Verfahren nach Anspruch 1, **dadurch gekennzeichnet, dass** F $\geq$ 870 beträgt.

6. Verfahren nach Anspruch 1, **dadurch gekennzeichnet, dass** F $\geq$ 900 beträgt.

7. Verfahren nach Anspruch 1, **dadurch gekennzeichnet, dass** F $\geq$ 950 beträgt.

8. Verfahren nach Anspruch 1, **dadurch gekennzeichnet, dass** F $\geq$ 1000 beträgt.

9. Verfahren nach Anspruch 1, **dadurch gekennzeichnet, dass** F $\geq$ 1050 beträgt.

10. Verfahren nach Anspruch 1, **dadurch gekennzeichnet, dass** F $\geq$ 1100 beträgt.

11. Verfahren nach Anspruch 1, **dadurch gekennzeichnet, dass** F $\geq$ 1150 beträgt.

12. Verfahren nach einem der Ansprüche 1 bis 11, **dadurch gekennzeichnet, dass** F $\leq$ 2500 beträgt.

13. Verfahren nach einem der Ansprüche 1 bis 11, **dadurch gekennzeichnet, dass** F $\leq$ 2400 beträgt.

14. Verfahren nach einem der Ansprüche 1 bis 11, **dadurch gekennzeichnet, dass** F $\leq$ 2200 beträgt.

15. Verfahren nach einem der Ansprüche 1 bis 11, **dadurch gekennzeichnet, dass** F $\leq$ 2000 beträgt.

16. Verfahren nach einem der Ansprüche 1 bis 11, **dadurch gekennzeichnet, dass** F $\leq$ 1900 beträgt.

**17.** Verfahren nach einem der Ansprüche 1 bis 11; **dadurch gekennzeichnet, dass** F ≤ 1800 beträgt.

**18.** Verfahren nach einem der Ansprüche 1 bis 11, **dadurch gekennzeichnet, dass** F ≤ 1700 beträgt.

**19.** Verfahren nach einem der Ansprüche 1 bis 11, **dadurch gekennzeichnet, dass** F ≤ 1500 beträgt.

**20.** Verfahren nach einem der Ansprüche 1 bis 19, **dadurch gekennzeichnet, dass** der stöchiometrische Koeffizient a 0,2 bis 2 beträgt.

**21.** Verfahren nach einem der Ansprüche 1 bis 19, **dadurch gekennzeichnet, dass** der stöchiometrische Koeffizient a 0,4 bis 1,5 beträgt.

**22.** Verfahren nach einem der Ansprüche 1 bis 19, **dadurch gekennzeichnet, dass** der stöchiometrische Koeffizient a 0,5 bis 1 beträgt.

**23.** Verfahren nach einem der Ansprüche 1 bis 22, **dadurch gekennzeichnet, dass** $1,2\ \mu m \le d_{50}^{A1} \le 8\ \mu m$ erfüllt ist.

**24.** Verfahren nach einem der Ansprüche 1 bis 22, **dadurch gekennzeichnet, dass** $1,5\ \mu m \le d_{50}^{A1} \le 6\ \mu m$ erfüllt ist.

**25.** Verfahren nach einem der Ansprüche 1 bis 22, **dadurch gekennzeichnet, dass** $1,5\ \mu m \le d_{50}^{A1} \le 4\ \mu m$ erfüllt ist.

**26.** Verfahren nach einem der Ansprüche 1 bis 22, **dadurch gekennzeichnet, dass** $2\ \mu m \le d_{50}^{A1} \le 3\ \mu m$ erfüllt ist.

**27.** Verfahren nach einem der Ansprüche 1 bis 26, **dadurch gekennzeichnet, dass** $170\ \mu m \ge d_{90}^{A2} \ge 30\ \mu m$ erfüllt ist.

**28.** Verfahren nach einem der Ansprüche 1 bis 26, **dadurch gekennzeichnet, dass** $150\ \mu m \ge d_{90}^{A2} \ge 40\ \mu m$ erfüllt ist.

**29.** Verfahren nach einem der Ansprüche 1 bis 26, **dadurch gekennzeichnet, dass** $130\ \mu m \ge d_{90}^{A2} \ge 50\ \mu m$ erfüllt ist.

**30.** Verfahren nach einem der Ansprüche 1 bis 29, **dadurch gekennzeichnet, dass** $d_{90}^{Q}$ für jede der verwendeten Quellen ≤ 4 $\mu$m ist.

**31.** Verfahren nach einem der Ansprüche 1 bis 29, **dadurch gekennzeichnet, dass** $d_{90}^{Q}$ für jede der verwendeten Quellen ≤ 3 $\mu$m ist.

**32.** Verfahren nach einem der Ansprüche 1 bis 29, **dadurch gekennzeichnet, dass** $d_{90}^{Q}$ für jede der verwendeten Quellen ≤ 2 $\mu$m ist.

**33.** Verfahren nach einem der Ansprüche 1 bis 29, **dadurch gekennzeichnet, dass** $d_{90}^{Q}$ für jede der verwendeten Quellen ≤ 1 $\mu$m ist.

**34.** Verfahren nach einem der Ansprüche 1 bis 29, **dadurch gekennzeichnet, dass** $d_{90}^{Q}$ für jede der verwendeten Quellen ≤ 0,8 $\mu$m ist.

**35.** Verfahren nach einem der Ansprüche 1 bis 29, **dadurch gekennzeichnet, dass** $d_{90}^{Q}$ für jede der verwendeten Quellen ≤ 0,5 $\mu$m ist.

**36.**

Verfahren nach einem der Ansprüche 1 bis 29, **dadurch gekennzeichnet, dass** $d_{90}^{Q}$ für jede der verwendeten Quellen ≤ 0,3 $\mu$m ist.

**37.** Verfahren nach einem der Ansprüche 1 bis 29, **dadurch gekennzeichnet, dass** $d_{90}^{Q}$ für jede der verwendeten

Quellen $\leq 0,2$ μm ist.

38. Verfahren nach einem der Ansprüche 1 bis 29, **dadurch gekennzeichnet, dass** jede der verwendeten Quellen im Verlauf der Herstellung der wässrigen Mischung M den Zustand einer kolloidalen oder einer echten Lösung durchläuft.

39. Verfahren nach einem der Ansprüche 1 bis 29, **dadurch gekennzeichnet, dass** der Anteil T das Element Si enthält und jede der verwendeten Quellen der von Silizium verschiedenen Elemente im Verlauf der Herstellung der wässrigen Mischung M den Zustand einer echten Lösung durchläuft und als Quelle des Elementes Si ein Kieselsol verwendet wird.

40. Verfahren nach einem der Ansprüche 1 bis 39, **dadurch gekennzeichnet, dass** die feinteilige Ausgangsmasse A2 durch Sprühtrocknung der wässrigen Mischung M erzeugt wird.

41. Verfahren nach einem der Ansprüche 1 bis 40, **dadurch gekennzeichnet, dass** die wässrige Mischung M wenigstens eine Hilfssubstanz aus der Gruppe, bestehend aus $NH_4OH$, $(NH_4)_2CO_3$, $NH_4HCO_3$, $NH_4NO_3$, Harnstoff, $NH_4CHO_2$, $H_2CO_3$, $HNO_3$, $H_2SO_4$, $NH_4CH_3CO_2$, $NH_4Cl$, $HCl$, $NH_4HSO_4$, $(NH_a)_2SO_4$, Ammoniumoxalat und den Hydraten der vorgenannten Verbindungen, enthält.

42. Verfahren nach einem der Ansprüche 1 bis 41, **dadurch gekennzeichnet, dass** $Z^1 = Co$ ist.

43. Verfahren nach einem der Ansprüche 1 bis 42, **dadurch gekennzeichnet, dass** $Z^2 = K$, Cs und/oder Sr ist.

44. Verfahren nach einem der Ansprüche 1 bis 43, **dadurch gekennzeichnet, dass** $Z^4 = Si$ ist.

45. Verfahren nach einem der Ansprüche 1 bis 44, **dadurch gekennzeichnet, dass** b 0,5 bis 3 ist.

46. Verfahren nach einem der Ansprüche 1 bis 44, **dadurch gekennzeichnet, dass** b 1 bis 2,5 ist.

47. Verfahren nach einem der Ansprüche 1 bis 46, **dadurch gekennzeichnet, dass** c 3 bis 8 ist.

48. Verfahren nach einem der Ansprüche 1 bis 46, **dadurch gekennzeichnet, dass** c 4 bis 7 ist.

49. Verfahren nach einem der Ansprüche 1 bis 48, **dadurch gekennzeichnet, dass** d 0,02 bis 2 ist.

50. Verfahren nach einem der Ansprüche 1 bis 48, **dadurch gekennzeichnet, dass** d 0,03 bis 1 ist.

51. Verfahren nach einem der Ansprüche 1 bis 48, **dadurch gekennzeichnet, dass** d 0,05 bis 0,5 ist.

52. Verfahren nach einem der Ansprüche 1 bis 51, **dadurch gekennzeichnet, dass** e 0,1 bis 4,5 ist.

53. Verfahren nach einem der Ansprüche 1 bis 51, **dadurch gekennzeichnet, dass** e 1 bis 4 ist.

54. Verfahren nach einem der Ansprüche 1 bis 53, **dadurch gekennzeichnet, dass** g 0,1 bis 8 ist.

55. Verfahren nach einem der Ansprüche 1 bis 53, **dadurch gekennzeichnet, dass** g 0,5 bis 3 ist.

56. Verfahren nach einem der Ansprüche 1 bis 55, **dadurch gekennzeichnet, dass** das feinteilige Mischoxid $Bi_1W_bO_x$ das Mischoxid $Bi_1W_2O_{7,5}$ ist.

57. Verfahren nach einem der Ansprüche 1 bis 56, **dadurch gekennzeichnet, dass** feinteilige Ausgangsmasse A1, feinteilige Ausgangsmasse A2 und feinteilige hydrophobisierte Kieselsäure umfassende Formgebungshilfsmittel zu der feinteiligen Ausgangsmasse A3 miteinander vermischt werden.

58. Verfahren nach einem der Ansprüche 1 bis 57, **dadurch gekennzeichnet, dass** feinteilige Ausgangsmasse A1, feinteilige Ausgangsmasse A2 und feinteiligen Graphit umfassende Formgebungshilfsmittel zu der feinteiligen Ausgangsmasse A3 miteinander vermischt werden.

**59.** Verfahren nach einem der Ansprüche 1 bis 58, **dadurch gekennzeichnet, dass** die Formung von geometrischen Formkörpern V mit feinteiliger Ausgangsmasse A3 durch Verdichten der feinteiligen Ausgangsmasse A3 erfolgt.

**60.** Verfahren nach Anspruch 59, **dadurch gekennzeichnet, dass** das Verdichten durch Strangpressen, Extrudieren oder Tablettieren erfolgt.

**61.** Verfahren nach einem der Ansprüche 1 bis 60, **dadurch gekennzeichnet, dass** der geometrische Formkörper V ein Ring ist.

**62.** Verfahren nach Anspruch 61, **dadurch gekennzeichnet, dass** die Seitendruckfestigkeit SD des ringförmigen Formkörpers V die Bedingung 12 N ≤ SD ≤ 25 N erfüllt.

**63.** Verfahren nach einem der Ansprüche 1 bis 60, **dadurch gekennzeichnet, dass** der geometrische Formkörper V eine Kugel ist.

**64.** Verfahren nach einem der Ansprüche 1 bis 60, **dadurch gekennzeichnet, dass** der geometrische Formkörper V ein Vollzylinder ist.

**65.** Verfahren nach Anspruch 61 oder 62, **dadurch gekennzeichnet, dass** der Außendurchmesser = 2 bis 10 mm, die Höhe = 2 bis 10 mm und die Wandstärke des Rings 1 bis 3 mm betragen.

**66.** Verfahren nach Anspruch 63, **dadurch gekennzeichnet, dass** der Kugeldurchmesser 2 bis 10 mm beträgt.

**67.** Verfahren nach Anspruch 64, **dadurch gekennzeichnet, dass** der Außendurchmesser = 1 bis 10 mm und die Höhe des Vollzylinders 2 bis 10 mm betragen.

**68.** Verfahren nach einem der Ansprüche 1 bis 58, **dadurch gekennzeichnet, dass** die Formung von geometrischen Formkörpern V mit feinteiliger Ausgangsmasse A3 dadurch erfolgt, dass man die feinteilige Ausgangsmasse A3 mit Hilfe eines flüssigen Bindemittels auf die Oberfläche eines geometrischen Trägerformkörpers aufbringt.

**69.** Verfahren nach einem der Ansprüche 1 bis 68, **dadurch gekennzeichnet, dass** im Rahmen der thermischen Behandlung der Formkörper V die Temperatur 350 °C überschritten und die Temperatur 600 °C nicht überschritten wird.

**70.** Verfahren nach einem der Ansprüche 1 bis 68, **dadurch gekennzeichnet, dass** im Rahmen der thermischen Behandlung der Formkörper V die Temperatur 420 °C überschritten und die Temperatur 500 °C nicht überschritten wird.

**71.** Verfahren nach einem der Ansprüche 1 bis 70, **dadurch gekennzeichnet, dass** die thermische Behandlung im Beisein von Luft erfolgt.

**72.** Verfahren nach einem der Ansprüche 1 bis 71, **dadurch gekennzeichnet, dass** der Partikeldurchmesser $d_{50}^{A2}$ der feinteiligen Ausgangsmasse A2 die Bedingung $10\ \mu m \leq d_{50}^{A2} \leq 50\ \mu m$ erfüllt.

**73.** Verfahren nach einem der Ansprüche 1 bis 71, **dadurch gekennzeichnet, dass** der Partikeldurchmesser $d_{50}^{A2}$ der feinteiligen Ausgangsmasse A2 die Bedingung $20\ \mu m \leq d_{50}^{A2} \leq 40\ \mu m$ erfüllt.

**74.** Verfahren nach einem der Ansprüche 1 bis 73, **dadurch gekennzeichnet, dass** der Betrag F* des Produktes

$$(d_{50}^{A1})^{0,7} \bullet (d_{50}^{A2})^{0,7} \bullet (a^{-1})$$

die Bedingung F* ≥ 15 erfüllt, wobei die beiden Partikeldurchmesser $d_{50}$ in der Längeneinheit $\mu m$ angegeben sind.

**75.** Verfahren nach einem der Ansprüche 1 bis 74, **dadurch gekennzeichnet, dass** das Verhältnis des Partikeldurch-

messers $d_{90}^{A2}$ der feinteiligen Ausgangsmasse A2 zum Partikeldurchmesser $d_{10}^{A2}$ der feinteiligen Ausgangsmasse A2 im Bereich von 5 bis 20 liegt.

**76.** Katalysatorformkörper erhältlich nach einem Verfahren gemäß einem der Ansprüche 1 bis 75.

**77.** Verfahren der heterogen katalysierten partiellen Gasphasenoxidation eines 3 bis 6 C-Atome enthaltenden Alkans, Alkanols, Alkanals, Alkens und/oder Alkenals an einem Katalysatorbett, **dadurch gekennzeichnet, dass** das Katalysatorbett einen Katalysatorformkörper gemäß Anspruch 76 umfasst.

**78.** Verfahren nach Anspruch 77, **dadurch gekennzeichnet, dass** es ein Verfahren der heterogen katalysierten partiellen Gasphasenoxidation von Propen zu Acrolein ist.

**79.** Verfahren nach Anspruch 77, **dadurch gekennzeichnet, dass** es ein Verfahren der heterogen katalysierten partiellen Gasphasenoxidation von iso-Buten zu Methacrolein ist.

**80.** Verfahren nach Anspruch 77, **dadurch gekennzeichnet, dass** es ein Verfahren der Ammoxidation von Propen zu Acrylnitril oder ein Verfahren der Ammoxidation von iso-Buten zu Methacrylnitril ist.

**Claims**

**1.** A process for producing geometric shaped catalyst bodies K which comprise, as an active material, a multielement oxide I of the general stoichiometry I

$$[Bi_1W_bO_x]_a[MO_{12}Z^1{}_cZ^2{}_dFe_eZ^3{}_fZ^4{}_gZ^5{}_hO_y]_1 \qquad (I)$$

where

Z$^1$ = one element or more than one element from the group consisting of nickel and cobalt,
Z$^2$ = one element or more than one element from the group consisting of the alkali metals, the alkaline earth metals and thallium,
Z$^3$ = one element or more than one element from the group consisting of zinc, phosphorus, arsenic, boron, antimony, tin, cerium, vanadium, chromium and bismuth,
Z$^4$ = one element or more than one element from the group consisting of silicon, aluminum, titanium, tungsten and zirconium,
Z$^5$ = one element or more than one element from the group consisting of copper, silver, gold, yttrium, lanthanum and the lanthanides,
a = 0.1 to 3,
b = 0.1 to 10,
c = 1 to 10,
d = 0.01 to 2,
e = 0.01 to 5,
f = 0 to 5,
g = 0 to 10,
h = 0 to 1, and
x, y = numbers determined by the valency and frequency of the elements in I other than oxygen,

in which

- a finely divided mixed oxide Bi$_1$W$_b$O$_x$ with a particle diameter $d_{50}^{A1}$ reported in the length unit $\mu$m, as starting material A1, is preformed with the proviso that $1\ \mu m \leq d_{50}^{A1} \leq 10\ \mu m$;
- sources of the elements other than oxygen in the component T = [Mo$_{12}$Z$^1{}_c$Z$^2{}_d$Fe$_e$Z$^3{}_f$Z$^4{}_g$Z$^5{}_h$O$_y$]$_1$ of the multielement oxide I are used in an aqueous medium to obtain an intimate aqueous mixture M, with the proviso that

- each of the sources used, in the course of preparation of the aqueous mixture M, passes through a degree of division Q for which its diameter $d_{90}^{Q}$ is $\leq 5\ \mu$m,

and
- the aqueous mixture M comprises the elements Mo, $Z^1$, $Z^2$, Fe, $Z^3$, $Z^4$ and $Z^5$ in the stoichiometry I*

$$Mo_{12}Z^1_cZ^2_dFe_eZ^3_fZ^4_gZ^5_h \qquad (I^*);$$

- the aqueous mixture M, by means of drying and adjusting the degree of division $d_{90}^{A2}$, is used to obtain a finely divided starting material A2 with a particle diameter reported in the length unit $\mu$m, with the proviso that

$200\ \mu m \geq d_{90}^{A2} \geq 20\ \mu m;$

- starting material A1 and starting material A2, or starting material A1, starting material A2 and finely divided shaping assistant, are mixed with one another to form a finely divided starting material A3, with the proviso that the starting material A3 comprises the elements other than oxygen introduced into the starting material A3 via starting materials A1 and A2 in the multielement oxide I in the stoichiometry I**

$$[Bi_1W_b]_a\,[Mo_{12}Z^1_cZ^2_dFe_eZ^3_fZ^4_gZ^5_h]_1 \qquad (I^{**}),$$

- finely divided starting material A3 is used to form geometric shaped bodies V,
and
- the shaped bodies V are treated thermally at elevated temperature to obtain the geometric shaped catalyst bodies K,

wherein the value F of the product

$$(d_{50}^{A1})^{0.7} \qquad \bullet \qquad (d_{90}^{A2})^{1.5} \qquad \bullet \qquad (a^{-1})$$

is $\geq 820$,

where the particle diameters $d_{50}^{A1}$, $d_{90}^{Q}$ and $d_{90}^{A2}$ are determined from the volume-based particle diameter determined by laser diffraction to ISO 13320 at a dispersing pressure of 2 bar absolute.

2. The process according to claim 1, wherein F is $\geq 830$.

3. The process according to claim 1, wherein F is $\geq 840$.

4. The process according to claim 1, wherein F is $\geq 850$.

5. The process according to claim 1, wherein F is $\geq 870$.

6. The process according to claim 1, wherein F is $\geq 900$.

7. The process according to claim 1, wherein F is $\geq 950$.

8. The process according to claim 1, wherein F is $\geq 1000$.

9. The process according to claim 1, wherein F is $\geq 1050$.

10. The process according to claim 1, wherein F is $\geq 1100$.

11. The process according to claim 1, wherein F is $\geq 1150$.

12. The process according to any one of claims 1 to 11, wherein F is $\leq 2500$.

13. The process according to any one of claims 1 to 11, wherein F is $\leq 2400$.

14. The process according to any one of claims 1 to 11, wherein F is $\leq 2200$.

**15.** The process according to any one of claims 1 to 11, wherein F is $\leq 2000$.

**16.** The process according to any one of claims 1 to 11, wherein F is $\leq 1900$.

**17.** The process according to any one of claims 1 to 11, wherein F is $\leq 1800$.

**18.** The process according to any one of claims 1 to 11, wherein F is $\leq 1700$.

**19.** The process according to any one of claims 1 to 11, wherein F is $\leq 1500$.

**20.** The process according to any one of claims 1 to 19, wherein the stoichiometric coefficient a is from 0.2 to 2.

**21.** The process according to any one of claims 1 to 19, wherein the stoichiometric coefficient a is from 0.4 to 1.5.

**22.** The process according to any one of claims 1 to 19, wherein the stoichiometric coefficient a is from 0.5 to 1.

**23.** The process according to any one of claims 1 to 22, wherein $1.2\ \mu m \leq d_{50}^{A1} \leq 8\ \mu m$.

**24.** The process according to any one of claims 1 to 22, wherein $1.5\ \mu m \leq d_{50}^{A1} \leq 6\ \mu m$.

**25.** The process according to any one of claims 1 to 22, wherein $1.5\ \mu m \leq d_{50}^{A1} \leq 4\ \mu m$.

**26.** The process according to any one of claims 1 to 22, wherein $2\ \mu m \leq d_{50}^{A1} \leq 3\ \mu m$.

**27.** The process according to any one of claims 1 to 26, wherein $170\ \mu m \geq d_{90}^{A2} \geq 30\ \mu m$.

**28.** The process according to any one of claims 1 to 26, wherein $150\ \mu m \geq d_{90}^{A2} \geq 40\ \mu m$.

**29.** The process according to any one of claims 1 to 26, wherein $130\ \mu m \geq d_{90}^{A2} \geq 50\ \mu m$.

**30.** The process according to any one of claims 1 to 29, wherein $d_{90}^{Q}$ for each of the sources used is $\leq 4\ \mu m$.

**31.** The process according to any one of claims 1 to 29, wherein $d_{90}^{Q}$ for each of the sources used is $\leq 3\ \mu m$.

**32.** The process according to any one of claims 1 to 29, wherein $d_{90}^{Q}$ for each of the sources used is $\leq 2\ \mu m$.

**33.** The process according to any one of claims 1 to 29, wherein $d_{90}^{Q}$ for each of the sources used is $\leq 1\ \mu m$.

**34.** The process according to any one of claims 1 to 29, wherein $d_{90}^{Q}$ for each of the sources used is $\leq 0.8\ \mu m$.

**35.** The process according to any one of claims 1 to 29, wherein $d_{90}^{Q}$ for each of the sources used is $\leq 0.5\ \mu m$.

**36.** The process according to any one of claims 1 to 29, wherein $d_{90}^{Q}$ for each of the sources used is $\leq 0.3\ \mu m$.

**37.** The process according to any one of claims 1 to 29, wherein $d_{90}^{Q}$ for each of the sources used is $\leq 0.2\ \mu m$.

**38.** The process according to any one of claims 1 to 29, wherein each of the sources used, in the course of preparation of the aqueous mixture M, passes through the state of a colloidal solution or of a true solution.

**39.** The process according to any one of claims 1 to 29, wherein the component T comprises the element Si and each of the sources of the elements other than silicon used, in the course of preparation of the aqueous mixture M, passes through the state of a true solution and the source of the element Si used is a silica sol.

**40.** The process according to any one of claims 1 to 39, wherein the finely divided starting material A2 is obtained by spray-drying the aqueous mixture M.

**41.** The process according to any one of claims 1 to 40, wherein the aqueous mixture M comprises at least one auxiliary substance from the group consisting of $NH_4OH$, $(NH_4)_2CO_3$, $NH_4HCO_3$, $NH_4NO_3$, urea, $NH_4CHO_2$, $H_2CO_3$, $HNO_3$, $H_2SO_4$, $NH_4CH_3CO_2$, $NH_4Cl$, $HCl$, $NH_4HSO_4$ $(NH_4)_2SO_4$, ammonium oxalate and the hydrates of the aforementioned compounds.

**42.** The process according to any one of claims 1 to 41, wherein $Z^1$ = Co.

**43.** The process according to any one of claims 1 to 42, wherein $Z^2$ = K, Cs and/or Sr.

**44.** The process according to any one of claims 1 to 43, wherein $Z^4$ = Si.

**45.** The process according to any one of claims 1 to 44, wherein b is from 0.5 to 3.

**46.** The process according to any one of claims 1 to 44, wherein b is from 1 to 2.5.

**47.** The process according to any one of claims 1 to 46, wherein c is from 3 to 8.

**48.** The process according to any one of claims 1 to 46, wherein c is from 4 to 7.

**49.** The process according to any one of claims 1 to 48, wherein d is from 0.02 to 2.

**50.** The process according to any one of claims 1 to 48, wherein d is from 0.03 to 1.

**51.** The process according to any one of claims 1 to 48, wherein d is from 0.05 to 0.5.

**52.** The process according to any one of claims 1 to 51, wherein e is from 0.1 to 4.5.

**53.** The process according to any one of claims 1 to 51, wherein e is from 1 to 4.

**54.** The process according to any one of claims 1 to 53, wherein g is from 0.1 to 8.

**55.** The process according to any one of claims 1 to 53, wherein g is from 0.5 to 3.

**56.** The process according to any one of claims 1 to 55, wherein the finely divided mixed oxide $Bi_1W_bO_x$ is the mixed oxide $Bi_1W_2O_{7.5}$.

**57.** The process according to any one of claims 1 to 56, wherein finely divided starting material A1, finely divided starting material A2 and shaping assistant comprising finely divided hydrophobized silica are mixed with one another to give the finely divided starting material A3.

**58.** The process according to any one of claims 1 to 57, wherein finely divided starting material A1, finely divided starting material A2 and shaping assistant comprising finely divided graphite are mixed with one another to give the finely divided starting material A3.

**59.** The process according to any one of claims 1 to 58, wherein geometric shaped bodies V are formed with finely divided starting material A3 by compacting the finely divided starting material A3.

**60.** The process according to claim 59, wherein the compaction is effected by extrusion or tableting.

**61.** The process according to any one of claims 1 to 60, wherein the geometric shaped body V is a ring.

**62.** The process according to claim 61, wherein the side crushing strength SCS of the annular shaped body V satisfies the condition 12 N $\leq$ SCS $\leq$ 25 N.

**63.** The process according to any one of claims 1 to 60, wherein the geometric shaped body V is a sphere.

**64.** The process according to any one of claims 1 to 60, wherein the geometric shaped body V is a solid cylinder.

**65.** The process according to claim 61 or 62, wherein the external diameter = from 2 to 10 mm, the height = from 2 to 10 mm and the wall thickness of the ring is from 1 to 3 mm.

**66.** The process according to claim 63, wherein the sphere diameter is from 2 to 10 mm.

**67.** The process according to claim 64, wherein the external diameter = from 1 to 10 mm and the height of the solid cylinder is from 2 to 10 mm.

**68.** The process according to any one of claims 1 to 58, wherein geometric shaped bodies V are formed with finely divided starting material A3 by applying the finely divided starting material A3 to the surface of a geometric shaped support body with the aid of a liquid binder.

**69.** The process according to any one of claims 1 to 68, wherein the temperature of 350°C is exceeded and the temperature of 600°C is not exceeded in the course of thermal treatment of the shaped bodies V.

**70.** The process according to any one of claims 1 to 68, wherein the temperature of 420°C is exceeded and the temperature of 500°C is not exceeded in the course of thermal treatment of the shaped bodies V.

**71.** The process according to any one of claims 1 to 70, wherein the thermal treatment is effected in the presence of air.

**72.** The process according to any one of claims 1 to 71, wherein the particle diameter $d_{50}^{A2}$ of the finely divided starting material A2 satisfies the condition $10~\mu\text{m} \leq d_{50}^{A2} \leq 50~\mu\text{m}$.

**73.** The process according to any one of claims 1 to 71, wherein the particle diameter $d_{50}^{A2}$ of the finely divided starting material A2 satisfies the condition $20~\mu\text{m} \leq d_{50}^{A2} \leq 40~\mu\text{m}$.

**74.** The process according to any one of claims 1 to 73, wherein the value F* of the product

$$(d_{50}^{A1})^{0.7} \bullet (d_{50}^{A2})^{0.7} \bullet (\text{a}^{-1})$$

satisfies the condition F* ≥ 15, where the two particle diameters $d_{50}$ are reported in the length unit $\mu$m.

**75.** The process according to any one of claims 1 to 74, wherein the ratio of the particle diameter $d_{90}^{A2}$ of the finely divided starting material A2 to the particle diameter $d_{10}^{A2}$ of the finely divided starting material A2 is in the range from 5 to 20.

**76.** A shaped catalyst body obtainable by a process according to any one of claims 1 to 75.

**77.** A process for heterogeneously catalyzed partial gas phase oxidation of an alkane, alkanol, alkanal, alkene and/or alkenal which comprises from 3 to 6 carbon atoms over a catalyst bed, wherein said catalyst bed comprises a shaped catalyst body according to claim 76.

**78.** The process according to claim 77, which is a process for heterogeneously catalyzed partial gas phase oxidation of propene to acrolein.

**79.** The process according to claim 77, which is a process for heterogeneously catalyzed partial gas phase oxidation of isobutene to methacrolein.

**80.** The process according to claim 77, which is a process for ammoxidation of propene to acrylonitrile or a process for ammoxidation of isobutene to methacrylonitrile.

**Revendications**

1. Procédé de fabrication de corps moulés catalytiques géométriques K, qui contiennent en tant que masse active un oxyde de plusieurs éléments I de stoechiométrie générale I

$$[Bi_1W_bO_x]_a[Mo_{12}Z^1_cZ^2_dFe_eZ^3_fZ^4_gZ^5_hO_y]_1 \qquad (I)$$

avec

Z$^1$ = un élément ou plus d'un élément du groupe constitué par le nickel et le cobalt,

Z$^2$ = un élément ou plus d'un élément du groupe constitué par les métaux alcalins, les métaux alcalino-terreux et le thallium,

Z$^3$ = un élément ou plus d'un élément du groupe constitué par le zinc, le phosphore, l'arsenic, le bore, l'antimoine, l'étain, le cérium, le vanadium, le chrome et le bismuth,

Z$^4$ = un élément ou plus d'un élément du groupe constitué par le silicium, l'aluminium, le titane, le tungstène et le zirconium,

Z$^5$ = un élément ou plus d'un élément du groupe constitué par le cuivre, l'argent, l'or, l'yttrium, le lanthane et les lanthanides,

a = 0, 1 à 3,

b = 0, 1 à 10,

c = 1 à 10,

d = 0,01 à 2,

e = 0,01 à 5,

f = 0 à 5,

g = 0 à 10,

h = 0 à 1, et

x, y = nombres déterminés par la valence et la fréquence des éléments différents de l'oxygène dans I,

selon lequel

- un oxyde mixte finement divisé $Bi_1W_bO_x$ présentant un diamètre de particule $d^{A1}_{50}$ donné en l'unité de longueur μm est préformé en tant que matière première A1, à condition que $1 \ \mu m \ \leq \ d^{A1}_{50} \ \leq \ 10 \ \mu m$ ;

- un mélange aqueux intime M est formé en utilisant des sources des éléments différents de l'oxygène de la fraction T = $[Mo_{12}Z^1_cZ^2_dFe_eZ^3_fZ^4_gZ^5_hO_y]_1$ de l'oxyde de plusieurs éléments I dans un milieu aqueux, à condition que

- chacune des sources utilisées passe au cours de la fabrication du mélange aqueux M par un degré de décomposition Q qui est **caractérisé en ce que** son diamètre $d^Q_{90} \ \leq \ 5 \ \mu m$, et

- que le mélange aqueux M contienne les éléments Mo, Z$^1$, Z$^2$, Fe, Z$^3$, Z$^4$ et Z$^5$ en la stoechiométrie I*

$$Mo_{12}Z^1_cZ^2_dFe_eZ^3_fZ^4_gZ^5_h \qquad (I^*) ;$$

- à partir du mélange aqueux M, une matière première finement divisée A2 présentant un diamètre de particule $d^{A2}_{90}$ donné en l'unité de longueur μm est formée par séchage et ajustement du degré de décomposition $d^{A2}_{90}$, à condition que $200 \ \mu m \ \geq \ d^{A2}_{90} \ \geq \ 20 \ \mu m$ ;

- la matière première A1 et la matière première A2, ou la matière première A1, la matière première A2 et des adjuvants de façonnage finement divisés sont mélangés les uns avec les autres pour former une matière première finement divisée A3, à condition que la matière première A3 contienne les éléments de l'oxyde de plusieurs éléments I, différents de l'oxygène, introduits dans la matière première A3 par les matières premières A1 et A2, en la stoechiométrie I**

$$[Bi_1W_b]_a \ [Mo_{12}Z^1_cZ^2_dFe_eZ^3_fZ^4_gZ^5_h]_1 \qquad (I^{**}),$$

- des corps moulés géométriques V sont formés avec la matière première finement divisée A3, et
- les corps moulés V sont traités thermiquement à température élevée pour obtenir les corps moulés catalytiques géométriques K,

**caractérisé en ce que** la valeur F du produit

$$( d_{50}^{A1} )^{0,7} \cdot ( d_{90}^{A2} )^{1,5} \cdot ( a^{-1} )$$

est $\geq 820$,

les diamètres de particule $d_{50}^{A1}$, $d_{90}^{Q}$ et $d_{90}^{A2}$ étant calculés à partir des diamètres de particule en volume déterminés par diffraction laser selon ISO 13320 à une pression de dispersion de 2 bar absolu.

2. Procédé selon la revendication 1, **caractérisé en ce que** F $\geq$ 830.

3. Procédé selon la revendication 1, **caractérisé en ce que** F $\geq$ 840.

4. Procédé selon la revendication 1, **caractérisé en ce que** F $\geq$ 850.

5. Procédé selon la revendication 1, **caractérisé en ce que** F $\geq$ 870.

6. Procédé selon la revendication 1, **caractérisé en ce que** F $\geq$ 900.

7. Procédé selon la revendication 1, **caractérisé en ce que** F $\geq$ 950.

8. Procédé selon la revendication 1, **caractérisé en ce que** F $\geq$ 1 000.

9. Procédé selon la revendication 1, **caractérisé en ce que** F $\geq$ 1 050.

10. Procédé selon la revendication 1, **caractérisé en ce que** F $\geq$ 1 100.

11. Procédé selon la revendication 1, **caractérisé en ce que** F $\geq$ 1 150.

12. Procédé selon l'une quelconque des revendications 1 à 11, **caractérisé en ce que** F $\leq$ 2 500.

13. Procédé selon l'une quelconque des revendications 1 à 11, **caractérisé en ce que** F $\leq$ 2 400.

14. Procédé selon l'une quelconque des revendications 1 à 11, **caractérisé en ce que** F $\leq$ 2 200.

15. Procédé selon l'une quelconque des revendications 1 à 11, **caractérisé en ce que** F $\leq$ 2 000.

16. Procédé selon l'une quelconque des revendications 1 à 11, **caractérisé en ce que** F $\leq$ 1 900.

17. Procédé selon l'une quelconque des revendications 1 à 11, **caractérisé en ce que** F $\leq$ 1 800.

18. Procédé selon l'une quelconque des revendications 1 à 11, **caractérisé en ce que** F $\leq$ 1 700.

19. Procédé selon l'une quelconque des revendications 1 à 11, **caractérisé en ce que** F $\leq$ 1 500.

20. Procédé selon l'une quelconque des revendications 1 à 19, **caractérisé en ce que** le coefficient stoechiométrique a est de 0,2 à 2.

21. Procédé selon l'une quelconque des revendications 1 à 19, **caractérisé en ce que** le coefficient stoechiométrique a est de 0,4 à 1,5.

**22.** Procédé selon l'une quelconque des revendications 1 à 19, **caractérisé en ce que** le coefficient stoechiométrique a est de 0,5 à 1.

**23.** Procédé selon l'une quelconque des revendications 1 à 22, **caractérisé en ce que**

$$1,2 \ \mu m \ \leq \ d_{50}^{A1} \ \leq \ 8 \ \mu m.$$

**24.** Procédé selon l'une quelconque des revendications 1 à 22, **caractérisé en ce que**

$$1,5 \ \mu m \ \leq \ d_{50}^{A1} \ \leq \ 6 \ \mu m.$$

**25.** Procédé selon l'une quelconque des revendications 1 à 22, **caractérisé en ce que**

$$1,5 \ \mu m \ \leq \ d_{50}^{A1} \ \leq \ 4 \ \mu m.$$

**26.** Procédé selon l'une quelconque des revendications 1 à 22, **caractérisé en ce que**

$$2 \ \mu m \ \leq \ d_{50}^{A1} \ \leq \ 3 \ \mu m.$$

**27.** Procédé selon l'une quelconque des revendications 1 à 26, **caractérisé en ce que**

$$170 \ \mu m \ \geq \ d_{90}^{A2} \ \geq \ 30 \ \mu m.$$

**28.** Procédé selon l'une quelconque des revendications 1 à 26, **caractérisé en ce que**

$$150 \ \mu m \ \geq \ d_{90}^{A2} \ \geq \ 40 \ \mu m.$$

**29.** Procédé selon l'une quelconque des revendications 1 à 26, **caractérisé en ce que** 130 $\mu$m $\geq$

$$130 \ \mu m \ \geq \ d_{90}^{A2} \ \geq \ 50 \ \mu m.$$

**30.** Procédé selon l'une quelconque des revendications 1 à 29, **caractérisé en ce que** $d_{90}^{Q}$ pour chacune des sources utilisées est $\leq$ 4 $\mu$m.

**31.** Procédé selon l'une quelconque des revendications 1 à 29, **caractérisé en ce que** $d_{90}^{Q}$ pour chacune des sources utilisées est $\leq$ 3 $\mu$m.

**32.** Procédé selon l'une quelconque des revendications 1 à 29, **caractérisé en ce que** $d_{90}^{Q}$ pour chacune des sources utilisées est $\leq$ 2 $\mu$m.

**33.** Procédé selon l'une quelconque des revendications 1 à 29, **caractérisé en ce que** $d_{90}^{Q}$ pour chacune des sources utilisées est $\leq$ 1 $\mu$m.

**34.** Procédé selon l'une quelconque des revendications 1 à 29, **caractérisé en ce que** $d_{90}^{Q}$ pour chacune des sources utilisées est $\leq$ 0,8 $\mu$m.

**35.** Procédé selon l'une quelconque des revendications 1 à 29, **caractérisé en ce que** $d_{90}^{Q}$ pour chacune des sources utilisées est $\leq$ 0,5 $\mu$m.

**36.** Procédé selon l'une quelconque des revendications 1 à 29, **caractérisé en ce que** $d_{90}^{Q}$ pour chacune des sources utilisées est $\leq$ 0,3 $\mu$m.

**37.** Procédé selon l'une quelconque des revendications 1 à 29, **caractérisé en ce que** $d_{90}^{Q}$ pour chacune des sources

utilisées est $\leq$ 0,2 $\mu$m.

**38.** Procédé selon l'une quelconque des revendications 1 à 29, **caractérisé en ce que** chacune des sources utilisées passe au cours de la fabrication du mélange aqueux M par l'état d'une solution colloïdale ou véritable.

**39.** Procédé selon l'une quelconque des revendications 1 à 29, **caractérisé en ce que** la fraction T contient l'élément Si et chacune des sources utilisées des éléments différents du silicium passe au cours de la fabrication du mélange aqueux M par l'état d'une solution véritable et un sol de silice est utilisé en tant que source de l'élément Si.

**40.** Procédé selon l'une quelconque des revendications 1 à 39, **caractérisé en ce que** la matière première finement divisée A2 est formée par séchage par pulvérisation du mélange aqueux M.

**41.** Procédé selon l'une quelconque des revendications 1 à 40, **caractérisé en ce que** le mélange aqueux M contient au moins une substance auxiliaire du groupe constitué par $NH_4OH$, $(NH_4)_2CO_3$, $NH_4HCO_3$, $NH_4NO_3$, urée, $NH_4CHO_2$, $H_2CO_3$, $HNO_3$, $H_2SO_4$, $NH_4CH_3CO_2$, $NH_4Cl$, $HCl$, $NH_4HSO_4$, $(NH_4)_2SO_4$, oxalate d'ammonium et les hydrates des composés susmentionnés.

**42.** Procédé selon l'une quelconque des revendications 1 à 41, **caractérisé en ce que** $Z^1$ = Co.

**43.** Procédé selon l'une quelconque des revendications 1 à 42, **caractérisé en ce que** $Z^2$ = K, Cs et/ou Sr.

**44.** Procédé selon l'une quelconque des revendications 1 à 43, **caractérisé en ce que** $Z^4$ = Si.

**45.** Procédé selon l'une quelconque des revendications 1 à 44, **caractérisé en ce que** b = 0,5 à 3.

**46.** Procédé selon l'une quelconque des revendications 1 à 44, **caractérisé en ce que** b = 1 à 2, 5.

**47.** Procédé selon l'une quelconque des revendications 1 à 46, **caractérisé en ce que** c = 3 à 8.

**48.** Procédé selon l'une quelconque des revendications 1 à 46, **caractérisé en ce que** c = 4 à 7.

**49.** Procédé selon l'une quelconque des revendications 1 à 48, **caractérisé en ce que** d = 0,02 à 2.

**50.** Procédé selon l'une quelconque des revendications 1 à 48, **caractérisé en ce que** d = 0,03 à 1.

**51.** Procédé selon l'une quelconque des revendications 1 à 48, **caractérisé en ce que** d = 0,05 à 0,5.

**52.** Procédé selon l'une quelconque des revendications 1 à 51, **caractérisé en ce que** e = 0,1 à 4, 5.

**53.** Procédé selon l'une quelconque des revendications 1 à 51, **caractérisé en ce que** e = 1 à 4.

**54.** Procédé selon l'une quelconque des revendications 1 à 53, **caractérisé en ce que** g = 0,1 à 8.

**55.** Procédé selon l'une quelconque des revendications 1 à 53, **caractérisé en ce que** g = 0,5 à 3.

**56.** Procédé selon l'une quelconque des revendications 1 à 55, **caractérisé en ce que** l'oxyde mixte finement divisé $Bi_1W_bO_x$ est l'oxyde mixte $Bi_1W_2O_{7,5}$.

**57.** Procédé selon l'une quelconque des revendications 1 à 56, **caractérisé en ce que** la matière première finement divisée A1, la matière première finement divisée A2 et un adjuvant de façonnage comprenant de la silice hydrophobée finement divisée sont mélangés les uns avec les autres pour former la matière première finement divisée A3.

**58.** Procédé selon l'une quelconque des revendications 1 à 57, **caractérisé en ce que** la matière première finement divisée A1, la matière première finement divisée A2 et un adjuvant de façonnage comprenant du graphite finement divisé sont mélangés les uns avec les autres pour former la matière première finement divisée A3.

**59.** Procédé selon l'une quelconque des revendications 1 à 58, **caractérisé en ce que** le façonnage de corps moulés géométriques V avec la matière première finement divisée A3 a lieu par compression de la matière première finement divisée A3.

**60.** Procédé selon la revendication 59, **caractérisé en ce que** la compression a lieu par filage à la presse, extrusion ou pastillage.

**61.** Procédé selon l'une quelconque des revendications 1 à 60, **caractérisé en ce que** le corps moulé géométrique V est un anneau.

**62.** Procédé selon la revendication 61, **caractérisé en ce que** la résistance à la compression latérale SD du corps moulé annulaire V satisfait la condition 12 N $\leq$ SD $\leq$ 25 N.

**63.** Procédé selon l'une quelconque des revendications 1 à 60, **caractérisé en ce que** le corps moulé géométrique V est une bille.

**64.** Procédé selon l'une quelconque des revendications 1 à 60, **caractérisé en ce que** le corps moulé géométrique V est un cylindre plein.

**65.** Procédé selon la revendication 61 ou 62, **caractérisé en ce que** le diamètre extérieur = 2 à 10 mm, la hauteur = 2 à 10 mm et l'épaisseur de paroi de l'anneau est de 1 à 3 mm.

**66.** Procédé selon la revendication 63, **caractérisé en ce que** le diamètre de la bille est de 2 à 10 mm.

**67.** Procédé selon la revendication 64, **caractérisé en ce que** le diamètre extérieur = 1 à 10 mm et la hauteur du cylindre plein est de 2 à 10 mm.

**68.** Procédé selon l'une quelconque des revendications 1 à 58, **caractérisé en ce que** le façonnage de corps moulés géométriques V avec la matière première finement divisée A3 a lieu par application de la matière première finement divisée A3 à l'aide d'un liant liquide sur la surface d'un corps moulé support géométrique.

**69.** Procédé selon l'une quelconque des revendications 1 à 68, **caractérisé en ce que**, dans le cadre du traitement thermique des corps moulés V, la température de 350 °C est dépassée et la température de 600 °C n'est pas dépassée.

**70.** Procédé selon l'une quelconque des revendications 1 à 68, **caractérisé en ce que**, dans le cadre du traitement thermique des corps moulés V, la température de 420 °C est dépassée et la température de 500 °C n'est pas dépassée.

**71.** Procédé selon l'une quelconque des revendications 1 à 70, **caractérisé en ce que** le traitement thermique a lieu en présence d'air.

**72.** Procédé selon l'une quelconque des revendications 1 à 71, **caractérisé en ce que** le diamètre de particule $d_{50}^{A2}$ de la matière première finement divisée A2 satisfait la condition $10 \ \mu m \leq d_{50}^{A2} \leq 50 \ \mu m$.

**73.** Procédé selon l'une quelconque des revendications 1 à 71, **caractérisé en ce que** le diamètre de particule $d_{50}^{A2}$ de la matière première finement divisée A2 satisfait la condition $20 \ \mu m \leq d_{50}^{A2} \leq 40 \ \mu m$.

**74.** Procédé selon l'une quelconque des revendications 1 à 73, **caractérisé en ce que** la valeur F* du produit

$$(d_{50}^{A1})^{0,7} \cdot (d_{90}^{A2})^{0,7} \cdot (a^{-1})$$

satisfait la condition F* $\geq$ 15, les deux diamètres de particule $d_{50}$ étant donnés en l'unité de longueur $\mu m$.

**75.** Procédé selon l'une quelconque des revendications 1 à 74, **caractérisé en ce que** le rapport entre le diamètre de particule $d_{90}^{A2}$ de la matière première finement divisée A2 et le diamètre de particule $d_{10}^{A2}$ de la matière première finement divisée A2 se situe dans la plage allant de 5 à 20.

**76.** Corps moulé catalytique pouvant être obtenu par un procédé selon l'une quelconque des revendications 1 à 75.

**77.** Procédé d'oxydation partielle en phase gazeuse sous catalyse hétérogène d'un alcane, alcanol, alcanal, alcène et/ou alcénal contenant 3 à 6 atomes C sur un lit catalytique, **caractérisé en ce que** lit catalytique comprend un corps moulé catalytique selon la revendication 76.

**78.** Procédé selon la revendication 77, **caractérisé en ce qu'**il s'agit d'un procédé d'oxydation partielle en phase gazeuse sous catalyse hétérogène de propène en acroléine.

**79.** Procédé selon la revendication 77, **caractérisé en ce qu'**il s'agit d'un procédé d'oxydation partielle en phase gazeuse sous catalyse hétérogène d'iso-butène en méthacroléine.

**80.** Procédé selon la revendication 77, **caractérisé en ce qu'**il s'agit d'un procédé d'ammoxydation de propène en acrylonitrile ou d'un procédé d'ammoxydation d'isobutène en méthacrylonitrile.

Fig. 1

Fig. 2

Fig. 3

Fig. 4

Fig. 5

Fig. 6

Fig. 7

Fig. 8

## IN DER BESCHREIBUNG AUFGEFÜHRTE DOKUMENTE

**In der Beschreibung aufgeführte Patentdokumente**

- DE 102007003778
- EP 575897 A
- WO 2007017431 A
- WO 0224620 A
- WO 200542459 A
- WO 200547224 A
- WO 200549200 A
- WO 2005113127 A
- DE 102008040093
- DE 102008040094
- DE 102007005606 A
- EP 1734030 A
- WO 200782827 A
- WO 20049525 A
- DE 102006000996 A
- WO 200777145 A
- DE 19835247 A
- DE 10051419 A
- DE 10046672 A
- DE 10122027 A
- DE 3338380 A
- EP 835 A
- WO 2005030393 A
- DE 10325487 A
- DE 102007004961 A
- US 20050131253 A
- WO 02062737 A
- EP 184790 A
- DE 10046957 A
- WO 0249757 A
- WO 2007082827 A
- WO 2005047224 A
- WO 2005042459 A
- EP 990636 A
- EP 1106598 A
- DE 10246119 A
- DE 10245585 A
- DE 4407020 A
- DE 10232748 A
- DE 4431957 A
- EP 700714 A
- EP 700893 A
- EP 468290 B
- DE 19910506 A
- DE 10313213 A
- DE 10313208 A
- DE 19948523 A
- DE 19948248 A
- DE 10313209 A
- WO 0053557 A
- WO 0053558 A
- WO 0136364 A
- DE 10337788 A
- DE 10361456 A

**In der Beschreibung aufgeführte Nicht-Patentliteratur**

- **H. BRUNNER ; D. SCHUTTE.** *Chem. Ing. Techn.,* 1965, vol. 89, 437
- Die Tablette. **W.A. RITSCHEL ; A. BAUER-BRANDL.** Handbuch der Entwicklung, Herstellung und Qualitätssicherung. Verlag Aulendorf, 2002
- Ullmann's Encyclopedia of Industrial Chemistry. VCH, 1992, vol. B4, 221
- **O. V. UDALOVA ; D. P. SHASHKIN ; M. D. SHIBANOVA ; O. V. KRYLOV.** *Kinetics and Catalysis,* 2005, vol. 46, 535-544
- **D. P. SHASHKIN ; O. V. UDALOVA ; M. D. SHIBANOVA ; O. V. KRYLOV.** *Kinetics and Catalysis,* 2005, vol. 46, 545-549
- **Y. MORO-OKA ; W. UEDA.** *Adv. Catal.,* 1994, vol. 40, 233-273
- **M. W. J. WOLFS ; P. A. BATIST.** *J. Catal.,* 1974, vol. 32, 25-36
- **D.-H. HE ; W. UEDA ; Y. MORO-OKA.** *Catal. Lett.,* 1992, vol. 12, 35-44
- **Y. HAYKAWA ; T. TSUNODA ; H. ORITA ; T. KAMEYAMA ; H. TAKAHASHI ; K. FUKUDA ; K. TAKEHIRA.** *J. Chem. Soc. Chem. Commun,* 1987, 780-782
- **M. T. LE ; W. J. M. V. WELL ; P. STOLTZE ; I. V. DRIESSCHE ; S. HASTE.** *Appl. Catal. A. Gen.,* 2005, vol. 282, 189-194
- **W. J. M. V. WELL ; M. T. LE ; N.C. SCHIEDT ; S. HOSTE ; P. STOLZTE.** *J. Mol. Catal. A. Chemical,* 2006, vol. 256, 1-8

- **J. M. M. MILLET ; G. COUDURIER ; J. M. HERRMANN ; J. C. VÉDRINE.** *J. Catal.,* 1993, vol. 142, 381-391
- Moderne Röntgenbeugung, Spieß-Schwarzer-Behnken-Teichert. Vieweg + Teubner, 2005
- **R. A. YOUNG.** The Rietveld Methode, IUCr. Oxford University Press, 1995
- **H. KRISCHNER.** Röntgenstrukturanalyse und Rietveldmethode. Vieweg Lehrbuch, 1994
- **HILL ; HOWARD.** *J. Appl. Cryst.,* 1987, vol. 20, 467-474